(19) Europäisches Patentamt
European Patent Office
Office européen des brevets

(11) Veröffentlichungsnummer: **0 175 249**
**A2**

(12) # EUROPÄISCHE PATENTANMELDUNG

(21) Anmeldenummer: **85111351.4**

(22) Anmeldetag: **09.09.85**

(51) Int. Cl.⁴: **C 07 C 121/75**, C 07 D 295/18, A 61 K 31/275

(30) Priorität: **12.09.84 DE 3433383**

(43) Veröffentlichungstag der Anmeldung: **26.03.86 Patentblatt 86/13**

(84) Benannte Vertragsstaaten: **AT BE CH DE FR GB IT LI LU NL SE**

(71) Anmelder: **Boehringer Mannheim GmbH, Sandhofer Strasse 116, D-6800 Mannheim 31 (DE)**

(72) Erfinder: **Leinert, Herbert, Dr. Ing., Essigkamm 11, D-6148 Heppenheim (DE)**
Erfinder: **Kampe, Wolfgang, Dr.rer.nat., Zedernstrasse 46, D-6805 Heddesheim (DE)**
Erfinder: **Strein, Klaus, Prof., Eichenstrasse 45, D-6944 Hemsbach (DE)**
Erfinder: **Müller-Beckmann, Bernd, Dr., Hochgewanne 46, D-6718 Grünstadt (DE)**
Erfinder: **Bartsch, Wolfgang, Dr.med.vet, Franconviller-Strasse 5, D-8606 Vierheim (DE)**

(54) **Neue Phenyl-acetonitril-Derivate.**

(57) Die vorliegende Erfindung betrifft neue Phenyl-acetonitril-Derivate der allgemeinen Formel I, ihre Herstellung, Arzneimittel, die diese enthalten und deren Verwendung zur Behandlung von Herz- und Kreislauferkrankungen.

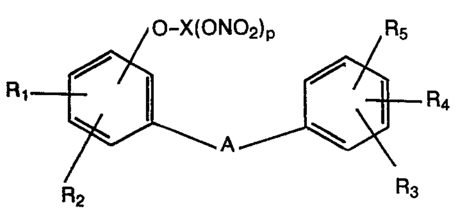

I

worin

$R_1$, $R_2$, $R_3$, $R_4$, $R_5$ gleich oder verschieden sind und Wasserstoffatome, Halogenatome, Alkylgruppen, Alkoxygruppen, Nitrogruppen, Aminogruppen und Acylaminogruppen bedeuten, wobei auch jeweils zwei Reste in Nachbarstellung zusammen einen Methylendioxy- oder Ethylendioxy-Ring bilden können,
A folgende Bedeutungen besitzt,

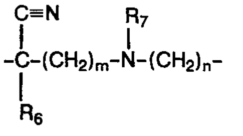

oder

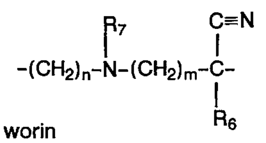

worin

$R_6$ einen geradkettigen, cyclischen oder verzweigten, gesättigten oder ungesättigten Alkylrest mit 2–12 Kohlenstoffatomen und

$R_7$ ein Wasserstoffatom oder einen geradkettigen oder verzweigten, gesättigten Alkylrest mit 1–6 Kohlenstoffatomen darstellen,

m und

n gleich oder verschieden sind und die Zahlen 2 und 3 bedeuten und

p die Zahlen 1 und 2 bedeutet,

X einen geradkettigen, cyclischen oder verzweigten Alkylrest mit 2–10 Kohlenstoffatomen, der gegebenenfalls noch durch eine Aminogruppe substituiert sein kann oder eine Gruppierung der allgemeinen Formeln II oder II′

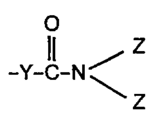

II

EP 0 175 249 A2

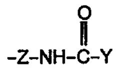 II'

worin Y und Z gleich oder verschieden sind und einen geradkettigen oder verzweigten Alkylrest mit 1–8 Kohlenstoffatomen oder einen Cycloalkylrest, Alkylcycloalkyl oder Cycloalkylalkyl darstellen, wobei diese Reste ggf. noch durch ein Sauerstoff- oder Schwefelatom unterbrochen sein können, und eine der Gruppen Z in Formel II auch Wasserstoff sein kann oder die beiden Gruppen Z zu einem Ring mit 4–6 C-Atomen geschlossen sind, der ggf. noch durch ein weiteres Stickstoffatom unterbrochen ist, welches durch Alkyl oder Alkanoyl substituiert sein kann, wobei die O–$NO_2$-Gruppen sowohl Substituenten von Y als auch Z sein können, sowie deren Salze mit physiologisch verträglichen Säuren.

BOEHRINGER MANNHEIM GMBH

2668 0175249

## Neue Phenyl-acetonitril-Derivate

Die vorliegende Erfindung betrifft neue Phenyl-acetonitril-Derivate, deren pharmakologisch vertraegliche Salze, ihre Herstellung sowie diese enthaltende Arzneimittel.

Gegenstand der Erfindung sind neue Phenyl-acetonitril-Derivate der allgemeinen Formel I,

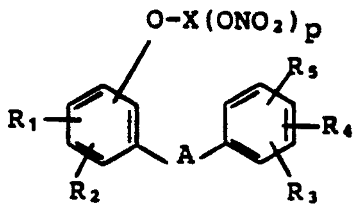

I

worin

$R_1$, $R_2$, $R_3$, $R_4$, $R_5$ gleich oder verschieden sind und Wasserstoffatome, Halogenatome, Alkylgruppen, Alkoxygruppen, Nitrogruppen, Aminogruppen und Acylaminogruppen bedeuten, wobei auch jeweils zwei Reste in Nachbarstellung zusammen einen Methylendioxy- oder Ethylendioxy-Ring bilden können,

A folgende Bedeutungen besitzt,

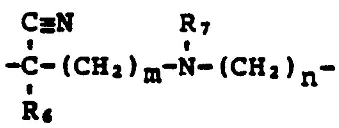

oder

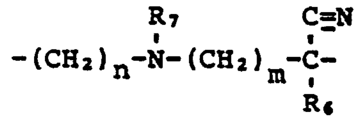

worin

$R_6$ einen geradkettigen, cyclischen oder verzweigten, gesättigten oder ungesättigten Alkylrest mit 2-12 Kohlenstoffatomen und

$R_7$ ein Wasserstoffatom oder einen geradkettigen oder verzweigten, gesättigten Alkylrest mit 1-6 Kohlenstoffatomen darstellen,

m und

n gleich oder verschieden sind und die Zahlen 2 u. 3 bedeuten und

p die Zahlen 1 und 2 bedeutet,

X einen geradkettigen, cyclischen oder verzweigten Alkylrest mit 2-10 Kohlenstoffatomen, der gegebenenfalls noch durch eine Aminogruppe substituiert sein kann oder eine Gruppierung der allgemeinen Formeln II oder II'

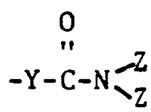 II

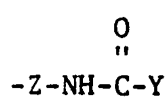 II'

worin Y und Z gleich oder verschieden sind und einen geradkettigen oder verzweigten Alkylrest mit 1-8 Kohlenstoffatomen oder einen Cycloalkylrest, Alkylcycloalkyl oder Cycloalkylalkyl darstellen, wobei diese Reste ggf. noch durch ein Sauerstoff- oder Schwefelatom unterbrochen sein können, und eine der Gruppen Z in Formel II auch Wasserstoff sein kann oder die beiden Gruppen Z zu einem Ring mit 4-6 C-Atomen geschlossen sind, der ggf. noch durch ein weiteres Stickstoffatom unterbrochen ist, welches durch Alkyl oder Alkanoyl substituiert sein kann, wobei die $O-NO_2$-Gruppen sowohl Substituenten von Y als auch Z sein können,
sowie deren Salze mit physiologisch verträglichen Säuren.

Als physiologisch vertraegliche Saeuren kommen z.B. in Frage: Salzsaeure, Bromwasserstoffsaeure, Schwefelsaeure, Phosphorsaeure, Essigsaeure, Malonsaeure, Bernsteinsaeure, Fumarsaeure, Maleinsaeure, Zitronensaeure, Weinsaeure, Milchsaeure, Amidosulfonsaeure, Benzoesaeure, Oxalsaeure, Adipinsaeure, Salicylsaeure, Naphthoesaeure und o-Acetoxybenzoesaeure.

Die erfindungsgemaeßen neuen Verbindungen besitzen mindestens ein asymmetrisches Kohlenstoffatom. Gegenstand der Erfindung sind daher saemtliche moeglichen Diastereomerengemische, Racemate und saemtliche optisch aktive Formen der erfindungsgemaeßen Verbindungen.

Es sind bereits eine Reihe aehnlicher Verbindungen bekannt (vgl. DE-PS 11 54810). Die erfindungsgemaeßen Verbindungen der allgemeinen Formel I unterscheiden sich jedoch von den dort beschriebenen Verbindungen, daß sie in mindestens einem der beiden Phenylkerne die Gruppe

$$-O-X-(ONO_2)_p$$

besitzen.

Die Verbindungen der allgemeinen Formel I besitzen gegenueber den in der DE-PS 11 54810 beschriebenen Verbindungen ueberlegene pharmakologische Eigenschaften.

Untersuchungen an wachen Hunden mit Substanzen der DE-PS 11 54810 (z.B. Verapamil) zeigen einen dosisabhaengigen Abfall des arteriellen Blutdruckes und einen dosisabhaengigen Anstieg des rechten Vorhofdruckes, d.h. eine Nachlastsenkung aber einer Anhebung der Vorlast. Im Gegensatz dazu zeigen die beanspruchten Substanzen der allg. Formel I eine dosisabhaengige Senkung des arteriellen Blutdruckes und auch des Vorhofdruckes und damit der Vorlast. Dies ist als ein wichtiger therapeutischer Vorteil anzusehen, da eine Vorlastsenkung ein anerkannt wirksames Therapieprinzip bei verschiedenen Erkrankungen des Herz-Kreislaufsystemes ist, z.B. bei Angina pectoris oder Herzinsuffiziens usw..

Weiterhin zeigen in vitro Untersuchungen an Blutgefaeßen, daß die Verbindungen der allg. Formel I im Gegensatz zu denen der Anmeldung DE-PS 1154810 den Gehalt an cyclischem Guanosinmonophosphat (c-GMP) erhoehen. Erhoehung des c-GMP-Gehaltes fuehrt nach heutiger Ansicht zur Gefaeßrelaxation, einem wichtigen Therapieprinzip bei Herzkreislauferkrankungen.

Die erfindungsgemaeßen Verbindungen der allgemeinen Formel I, in der $R_1$, $R_2$, $R_3$, $R_4$, $R_5$, A, X und p die oben genannten Bedeutungen besitzen, können dargestellt werden, indem man

a) eine Verbindung der allgemeinen Formel III,

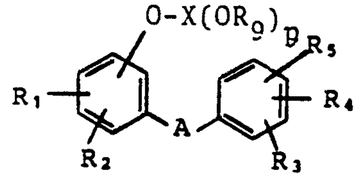

III

in der $R_1$, $R_2$, $R_3$, $R_4$, $R_5$, A, X und p die oben angegebenen Bedeutungen besitzen und $R_9$ Wasserstoff oder eine leicht abspaltbare Gruppe darstellt einer Nitratester-Bildungsreaktion unterwirft, oder

b) indem man eine Verbindung der allgemeinen Formel IV,

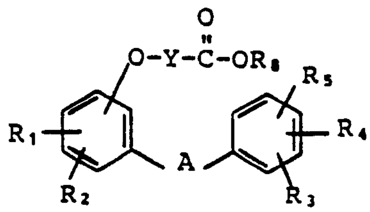

IV

in der $R_1$, $R_2$, $R_3$, $R_4$, A und Y die oben angegebenen Bedeutungen besitzen und $R_8$ Wasserstoff oder eine niedere Alkylgruppe bedeutet, einer Amidbildungsreaktion mit einer Verbindung der allgemeinen Formel V,

$$H_2N-Z-(ONO_2)_p \qquad V$$

wobei Z und p die angegebenen Bedeutungen besitzen, unterwirft,

oder

c) indem man eine Verbindung der allgemeinen Formel VI,

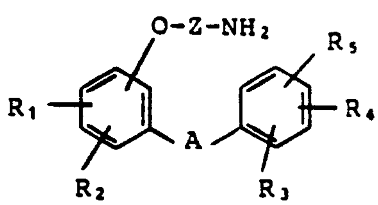

VI

in der $R_1$, $R_2$, $R_3$, $R_4$, $R_5$, A und Z die oben angegebenen Bedeutungen besitzen, einer Amidbildungsreaktion mit einer Verbindung der allgemeinen Formel VII,

$$R_8O-\overset{O}{\overset{\|}{C}}-Y-(ONO_2)_p \qquad VII$$

wobei $R_8$, Y und p die angegebenen Bedeutungen besitzen, unterwirft und
die erhaltenen Verbindungen ggf. mit Säuren oder Basen in die entsprechenden Salze überführt oder daraus freisetzt.

Verbindungen der allgemeinen Formel III werden erhalten, indem man eine Verbindung der allgemeinen Formel IV mit einer Verbindung der allgemeinen Formel V',

$$H_2N\text{-}Z\text{-}(OR_9)_p \qquad V'$$

wobei Z und p die oben genannten Bedeutungen besitzen und $R_9$ Wasserstoff oder eine leicht abspaltbare Schutzgruppe bedeutet, umsetzt,

oder

indem man eine Verbindung der allgemeinen Formel VI mit einer Verbindung der allgemeinen Formel VII',

$$R_8O\text{-}\overset{\overset{\displaystyle O}{\|}}{C}\text{-}Y\text{-}(OR_9)_p \qquad VII'$$

worin $R_8$, $R_9$, Y und p die oben genannten Bedeutungen besitzen, umsetzt.

Die Nitratester-Bildungsreaktion der Verbindungen der allgemeinen Formel III, worin $R_9$ Wasserstoff oder eine leicht abspaltbare Schutzgruppe bedeutet, kann durchgeführt werden, indem man mit einem nitratesterbildenden Reagenz, wie rauchende Salpetersäure und Acetanhydrid oder einer Mischung aus rauchender Salpetersäure und konz. Schwefelsäure bei niedrigen Temperaturen in Gegenwart oder Abwesenheit eines inerten Lösungsmittels umsetzt. Die Reaktionstemperaturen liegen zwischen Raumtemperatur und -60°C, bevorzugt zwischen -10°C und -30°C. Das Molverhältnis liegt zwischen 1 und 10.

Als Schutzgruppen von $R_9$ kommen vorzugsweise Estergruppen in Frage, die unter den sauren Bedingungen der Nitratierungsreaktion leicht abgespalten bzw. gegen die Nitratgruppe ausgetauscht werden. Bevorzugt sind die Benzoyloxy-, Alkoxycarbonyl-, Alkanoyl- und Sulfonylgruppen. Basisch abspaltbare Gruppen müssen ggf. in einer Vorreaktion entfernt werden.

Alternativ kann die Nitratester-Bildungsreaktion auch durchgeführt werden, indem man in einer Verbindung der allgemeinen Formel III, worin $R_9$ Wasserstoff oder einen aktiven Ester, bspw. eine Sulfonylgruppe, bedeutet, die Gruppe $OR_9$ halogeniert und anschließend das Reaktionsprodukt mit Silbernitrat in Gegenwart oder Abwesenheit eines Lösungsmittels bei Temperaturen zwischen Raumtemperatur und 100 C umsetzt.

Die Halogenierungsreaktion kann nach Literatur bekannten Verfahren durchgeführt werden, indem man eine Verbindung der allgemeinen Formel III mit Mesylchlorid oder Tosylchlorid in Gegenwart eines säurebindenden Mittels umsetzt und anschließend das Reaktionsprodukt mit einen Alkalihalogenid in einem organischen Lösungsmittel, z.B. Dimethylformamid, zu Reaktion bringt. Das Molverhältnis der Mischungsreaktion zwischen der Halogenverbindung und Silbernitrat ist nicht kritisch. Es kann zwischen 1 und 10 betragen. Auch diese Reaktion wird üblicherweise zwischen 20°C und 100°C durchgeführt.

Die Amidbildungsreaktion einer Verbindung der allgemeinen Formel IV mit einer Verbindung der allgemeinen Formel V kann durchgefuehrt werden, indem man die beiden Verbindungen in einem inerten Loesungsmittel wie z.B. Tetrahydrofuran, Dioxan, Dimethylformamid oder Methylenchlorid zur Reaktion bringt fuer den Fall, daß $R_8$ in der allgemeinen Formel IV einen niederen Alkylrest darstellt. Die Reaktion kann zwischen Raumtemperatur und 100°C durchgefuehrt werden. Die Reaktionsdauer kann zwischen einer und 10 Stunden betragen. Das Molverhaeltnis der Reaktanden kann zwischen 1 und 3 liegen.

Fuer den Fall, daß $R_8$ in der allgemeinen Formel IV Wasserstoff darstellt, kann die Amidbildungsreaktion mit einer Verbindung der allgemeinen Formel V mit Hilfe eines Kupplungsreagenzes wie Dicyclohexylcarbodiimid oder Carbonyldiimidazol oder durch Ueberfuehrung der Carboxylgruppe in einer Verbindung der allgemeinen Formel IV in ein aktiviertes Carbonsaeurederivat wie z.B. ein Carbonsaeurehalogenid oder gemischtes Anhydrid nach an sich bekannten Verfahren durchgefuehrt werden. Als Loesungsmittel kommen inerte organische Loesungsmittel zur Anwendung wie z.B. Tetrahydrofuran, Dioxan, Dimethylformamid oder Methylenchlorid. Die Reaktionstemperaturen liegen zwischen -30°C und +50°C. Die Reaktionszeiten betragen zwischen 30 min und 10 Stunden. Das Molverhaeltnis der Reaktanden kann zwischen 1 und 5 liegen.

Die Amidbildungsreaktion einer Verbindung der allgemeinen Formel IV mit einer Verbindung der allgemeinen Formel V' kann durchgefuehrt werden, indem man die beiden Verbindungen direkt oder in einem inerten Loesungsmittel wie z.B. Tetrahydrofuran, Dioxan, Dimethylformamid oder Methylenchlorid zur Reaktion bringt fuer den Fall, daß $R_8$ in der allgemeinen Formel IV einen niederen Alkylrest darstellt und $R_9$ in der allgemeinen Formel V' Wasserstoff oder eine Schutzgruppe bedeutet.

Die Reaktion kann zwischen Raumtemperatur und 100°C durchgefuehrt werden. Die Reaktionsdauer kann zwischen einer und 10 Stunden betragen. Das Molverhaeltnis der Reaktanden kann zwischen 1 und 3 liegen.

Für den Fall, daß $R_8$ in der allgemeinen Formel IV Wasserstoff darstellt, kann die Amidbildungsreaktion mit einer Verbindung der allgemeinen Formel V' mit Hilfe eines Kupplungsreagenzes wie Dicyclohexylcarbodiimid oder Carbonyldiimidazol oder durch Überführung der Carboxylgruppe in einer Verbindung der allgemeinen Formel IV in ein aktiviertes Carbonsäurederivat, wie z.B. ein Carbonsäurehalogenid oder gemischtes Anhydrid, z.B. mit Halogenameisensäureester, nach an sich bekannten Verfahren durchgeführt werden. Als Lösungsmittel kommen inerte organische Lösungsmittel zur Anwendung, wie z.B. Tetrahydrofuran, Dioxan, Dimethylformamid oder Methylenchlorid. Die Reaktionstemperaturen liegen zwischen -30°C und +50°C. Die Reaktionszeiten betragen zwischen 30 min und 10 Stunden. Das Molverhältnis der Reaktanden kann zwischen 1 und 5 liegen.

Die Amidbildungsreaktionen einer Verbindung der allgemeinen Formeln VII oder VII' mit einer Verbindung der allgemeinen Formel VI können grundsätzlich nach den gleichen Verfahren, wie bei den Amidbildungsreaktionen aus einer Verbindung der allgemeinen Formel IV mit einer Verbindung der allgemeinen Formeln V oder V' beschrieben, durchgeführt werden.

Die Verbindungen der allgemeinen Formeln III, IV u. VI koennen dargestellt werden, indem man eine Verbindung der allgemeinen Formel VIII,

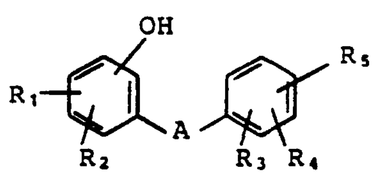

VIII

worin $R_1$, $R_2$, $R_3$, $R_4$, $R_5$ und A die oben angegebenen Bedeutungen besitzen, nach an sich bekannten Verfahren mit einer Verbindung der allgemeinen Formel IX,

Hal-X'-W IX

worin Hal ein Halogenatom wie Chlor oder Brom bedeutet und

X' einen geradkettigen oder verzweigten Alkylrest mit 1-8 Kohlenstoffatomen, der gegebenenfalls noch durch eine Aminogruppe substituiert sein kann, darstellt, uns

W eine Hydroxylgruppe, Aminogruppe, Carboxylgruppe oder Carbalkoxygruppe oder eine in diese nachtraeglich umwandelbare Atomgruppierung darstellt oder

ein Kohlenstoffatom von X' und W zusammen einen Epoxidring bilden koennen.

Die Verbindungen der allgemeinen Formel VIII koennen z.B. dargestellt werden, indem man eine Verbindung der allgemeinen Formel X,

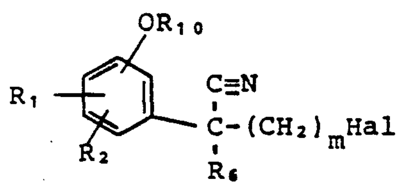

X

in der $R_1$, $R_2$, $R_6$, Hal und m die oben genannten Bedeutungen besitzen und

$R_{10}$ eine Benzylgruppe bedeutet, mit einer Verbindung der allgemeinen Formel XI,

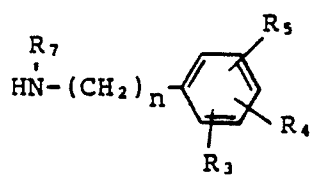

XI

worin $R_3$, $R_4$, $R_5$, $R_7$ und n die oben genannten Bedeutungen besitzen, nach an sich bekannten Verfahren zur Reaktion bringt und anschließend die Benzylgruppe hydrogenolytisch abspaltet.

Die Verbindungen der allgemeinen Formel VIII koennen auch dargestellt werden, indem man eine Verbindung der allgemeinen Formel XII,

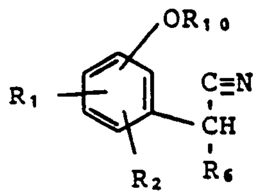

XII

worin $R_1$, $R_2$, $R_6$ und $R_{10}$ die oben genannten Bedeutungen besitzen, mit einer Verbindung der allgemeinen Formel XIII,

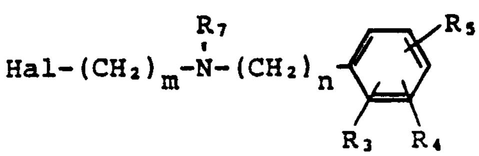

XIII

worin $R_3$, $R_4$, $R_5$, $R_7$, Hal, m und n die oben genannten Bedeutungen besitzen, umsetzt und anschließend die Benzylgruppe hydrogenolytisch abspaltet.

Die Verbindungen der Formeln III, IV, VI u. VIII sind neu und zum Teil ebenfalls pharmakologisch wirksam.

Die Herstellung der Verbindungen der allgemeinen Formel X erfolgt analog zu allgemein bekannten Verfahren, z.B. durch Umsetzung einer Verbindung der allgemeinen Formel XII mit einer Verbindung der allgemeinen Formel XIV,

$$Hal(CH_2)_mHal \qquad XIV$$

worin Hal ein Chlor- oder Bromatom bedeutet, mit Hilfe einer phasentransferkatalysierten Reaktion. Als Katalysatoren kommen quartaere Ammonium- und Phosphoniumsalze oder Kronenether zur Anwendung.

**Die Herstellung der** Verbindungen der allgemeinen Formel XII erfolgt ebenfalls analog zu bekannten Verfahren z.B. durch Umsetzung einer Verbindung der allgemeinen Formel XV

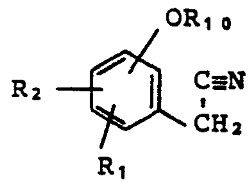

XV

worin $R_1$, $R_2$ u. $R_{10}$ die oben genannten Bedeutungen besitzen, mit einer Verbindung der allgemeinen Formel XVI

$R_6$-Hal XVI

wobei $R_6$ u. Hal die oben genannten Bedeutungen besitzen, mit Hilfe einer phasentransferkatalysierten Reaktion. Als Katalysatoren kommen quartaereAmmonium- und Phosphoniumsalze oder Kronenether zur Anwendung.

Halogen im Sinne dieser Anmeldung bedeutet Fluor, Chlor, Brom und Jod, wobei Fluor und Chlor bevorzugt sind. Alkyl auch in Alkoxy bedeutet, soweit nicht anders definiert, eine Gruppe mit 1-6 C-Atomen, insbesondere die Methyl, Ethyl, Propyl, i. Propyl und Butylgruppe.

Unter cyclischen Alkylgruppen sind Mono- u. Bicyclen, insbesondere c. Propyl, c. Pentyl u. c. Hexyl zu verstehen.

Besonders bevorzugt sind Verbindungen, in denen A in der Formel I die Gruppe

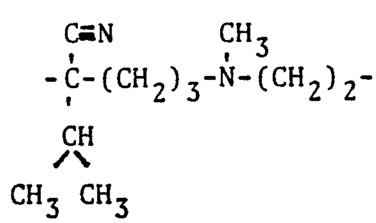

darstellt.

Unter Acylaminogruppe sind Alkanoylaminogruppen mit 1-6 C-Atomen, insbesondere die Formyl- und Acetylaminogruppe zu verstehen.

Die erfindungsgemaeßen neuen Substanzen I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt. Derartige Zusaetze sind z.B. Tartrat- und Citratpuffer, Ethanol, Komplexbildner (wie Ethylendiamintetraessigsaeure und deren nicht-toxische Salze), hochmolekulare Polymere (wie fluessiges Polyethylenoxyd) zur Viskositaetsregulierung. Feste Traegerstoffe sind z.B. Staerke, Lactose, Mannit, Methylcellulose, Talkum, hochdisperse Kieselsaeuren, hoehermolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole), fuer orale Applikation geeignete Zubereitungen koennen gewuenschtenfalls Geschmacks- und Sueßstoffe enthalten.

Die erfindungsgemäßen Verbindungen werden üblicherweise in Mengen von 50-500 mg pro Tag bezogen auf 75 kg Körpergewicht appliziert. Bevorzugt ist es, 2-3 mal pro Tag 1-2 Tabletten mit einem Wirkstoffgehalt von 20-200 mg zu verabreichen. Die Tabletten können auch retardiert sein, wodurch nur noch 1 mal pro Tag 1-2 Tabletten mit 50-500 mg Wirkstoff gegeben werden muß. Der Wirkstoff kann auch durch Injektion 1-8 mal pro Tag bzw. durch Dauerinfusion gegeben werden, wobei Mengen von 10-200 mg/Tag normalerweise ausreichen.

Bevorzugte Verbindungen sind:

2-Methoxy-4-<1-cyano-1-(methylethyl)-4-[N-methyl-N-[2-(3,4-dimethoxy-phenyl)-ethyl]-amino-butyl]>-phenoxy-N-(3-nitro-oxy-propyl-1)-acetamid

2-Methoxy-4-<1-cyano-1-(methylethyl)-4-[N-methyl-N-[2-(3,4-dimethoxy-phenyl)-ethyl]-amino-butyl]>- phenoxyessigsaeure-4-(nitro-oxy-methyl)-piperidylamid

2-Methoxy-4-<1-cyano-1-(methylethyl)-4-[N-methyl-N-[2-(3,4-dimethoxy-phenyl)-ethyl]-amino-butyl]>-phenoxyessigsaeure-4(α-nitro-oxy-ethyl)-piperidylamid

2-Methoxy-4-<1-cyano-1-(methylethyl)-4-[N-methyl-N-[2-(3,4-dimethoxy-phenyl)-ethyl]-amino-butyl]>-phenoxy-N-(4-methyl-3-nitro-oxy-pentyl-2)-acetamid

2-Methoxy-4-<1-cyano-1-(methylethyl)-4-[N-methyl-N-[2-(3,4-dimethoxy-phenyl)-ethyl]-amino-butyl]>-phenoxy-N-(4,4-dimethyl-3-nitro-oxy-pentyl-2)-acetamid

2-Methoxy-4-<1-cyano-1-(methylethyl)-4-[N-methyl-N-[2-(3,4-dimethoxy-phenyl)-ethyl]-amino-butyl]>-phenoxy-N-(2,4-dimethyl-3-nitro-oxy-pentyl-2)-acetamid

2-Methoxy-4-<1-cyano-1-(methylethyl)-4-[N-methyl-N-[2-(3,4-dimethoxy-phenyl)-ethyl]-amino-butyl]>-phenoxy-N-(2,4,4-trimethyl-3-nitro-oxy-pentyl-2)-acetamid

2-Methoxy-4-<1-cyano-1-(methylethyl)-4-[N-methyl-N-[2-(3,4-dimethoxy-phenyl)-ethyl]-amino-butyl]>-phenoxy-N-(2,2-dimethyl-3-nitro-oxy-propyl-1)-acetamid

2-Chlor-3-ethyl-4-<1-cyano-1-(methylethyl)-3-[N-methyl-N-[2-(3,4-dimethyl-phenyl)ethyl]-amino-propyl ]>-phenoxy-N-(3-nitro-oxy-propyl-1)-acetamid

2-Methoxy-5-<1-cyano-1-(methylethyl)-4-[N-methyl-N-[2-(3,4-dimethoxy-phenyl)-ethyl]-amino-butyl]>-phenoxy-N-(3-nitro-oxy-propyl-1)-acetamid

2-Methoxy-5-<1-cyano-1-(methylethyl)-4-[N-methyl-N-[2-(3,4-dimethoxy-phenyl)-ethyl]-amino-butyl]>-phenoxyessigsaeure-4-(nitro-oxy-methyl)-piperidylamid

2-Methoxy-5-<1-cyano-1-(methylethyl)-4-[N-methyl-N-[2-(3,4-dimethoxy-phenyl)-ethyl]-amino-butyl]>-phenoxyessigsaeure-4-(α-nitro-oxy-ethyl)-piperidylamid

2-Methoxy-5-<1-cyano-1-(methylethyl)-4-[N-methyl-N-[2-(3,4-dimethoxy-phenyl)-ethyl]-amino-butyl]>-phenoxy-N-(4-methyl-3-nitro-oxy-pentyl-2)-acetamid

2-Methoxy-5-<1-cyano-1-(methylethyl)-4-[N-methyl-N-[2-(3,4-dimethoxy-phenyl)-ethyl]-amino-butyl]>-phenoxy-N-(4,4-dimethyl-3-nitro-oxy-pentyl-2)-acetamid

2-Methoxy-5-<1-cyano-1-(methylethyl)-4-[N-methyl-N-[2-(3,4-dimethoxy-phenyl)-ethyl]-amino-butyl]>-phenoxy-N-(2,4-dimethyl-3-nitro-oxy-pentyl-2)-acetamid

2-Methoxy-5-<1-cyano-1-(methylethyl)-4-[N-methyl-N-[2-(3,4-dimethoxy-phenyl)-ethyl]-amino-butyl]>-phenoxy-N-(2,4,4-trimethyl-3-nitro-oxy-pentyl-2)-acetamid

2-Methoxy-5-<1-cyano-1-(methylethyl)-4-[N-methyl-N-[2-(3,4-dimethoxy-phenyl)-ethyl]-amino-butyl]>-phenoxy-N-(2,2-dimethyl-3-nitro-oxy-propyl-1)-acetamid

2-Methoxy-5-<1-cyano-1-(c.hexyl)-4-[N-ethyl-N-[2-(3,4-methylendioxy-phenyl)-ethyl]-amino-butyl]>-phenoxy-N-(3-nitro-oxy-propyl-1)-acetamid

2-Methoxy-4-<2-[N-methyl-N-[4-cyano-4-(methylethyl)-4-(3,4-dimethoxy-phenyl)-butyl]-amino-ethyl]>-phenoxy-N-(3-nitro-oxy-propyl-1)-acetamid

2-Methoxy-4-<2-[N-methyl-N-[4-cyano-4-(methylethyl)-4-(3,4-dimethoxy-phenyl)-butyl]-amino-ethyl]>-phenoxyessigsaeure-4-(nitro-oxy-methyl)-piperidylamid

2-Methoxy-4-<2-[N-methyl-N-[4-cyano-4-(methylethyl)-4-(3,4-dimethoxy-phenyl)-butyl]-amino-ethyl]>-phenoxyessigsaeure-4-(α-nitro-oxy-ethyl)-piperidylamid

2-Methoxy-4-<2-[N-methyl-N-[4-cyano-4-(methylethyl)-4-(3,4-dimethoxy-phenyl)-butyl]-amino-ethyl]>-phenoxy-N-(4-methyl-3-nitro-oxy-pentyl-2)-acetamid

2-Methoxy-4-<2-[N-methyl-N-[4-cyano-4-(methylethyl)-4-(3,4-dimethoxy-phenyl)-butyl]-amino-ethyl]>- phenoxy-N-(4,4-dimethyl-3-nitro-oxy-pentyl-2)-acetamid

2-Methoxy-4-<2-(N-methyl-N-[4-cyano-4-(methylethyl)-4-(3,4-dimethoxy-phenyl)-butyl]-amino-ethyl]>-phenoxy-N-(2,4-dimethyl-3-nitro-oxy-pentyl-2)-acetamid

2-Methoxy-4-<2-(N-methyl-N-[4-cyano-4-(methylethyl)-4-(3,4-dimethoxy-phenyl)-butyl]-amino-ethyl]>-phenoxy-N-(2,4,4-trimethyl-3-nitro-oxy-pentyl-2)-acetamid

2-Methoxy-4-<2-(N-methyl-N-[4-cyano-4-(methylethyl)-4-(3,4-dimethoxy-phenyl)-butyl]-amino-ethyl]>-phenoxy-N-(2,2-dimethyl-3-nitro-oxy-propyl-1)-acetamid

2-Propoxy-4-<2-N-[4-cyano-4-(methylethyl)-4-(4-fluor-phenyl)-butyl]-aminoethyl]>-phenoxy-(2-nitro-oxy-ethyl-1)-acetamid

2-Methoxy-5-<2-[N-methyl-N-[4-cyano-4-(methylethyl)-4-(3,4-dimethoxy-phenyl)-butyl]-amino-ethyl]>-phenoxy-N-(3-nitro-oxy-propyl-1)-acetamid

2-Methoxy-5-<2-[N-methyl-N-[4-cyano-4-(methylethyl)-4-(3,4-dimethoxy-phenyl)-butyl]-amino-ethyl]>-phenoxyessigsaeure-4-(nitro-oxy-methyl)-piperidylamid

2-Methoxy-5-<2-[N-methyl-N-[4-cyano-4-(methylethyl)-4-(3,4-dimethoxy-phenyl)-butyl]-amino-ethyl]>-phenoxyessigsaeure-4-(α-nitro-oxy-ethyl)-piperidylamid

2-Methoxy-5-<2-[N-methyl-N-[4-cyano-4-(methylethyl)-4-(3,4-dimethoxy-phenyl)-butyl]-amino-ethyl]>-phenoxy-N-(4-methyl-3-nitro-oxy-pentyl-2)-acetamid

2-Methoxy-5-<2-[N-methyl-N-[4-cyano-4-(methylethyl)-4-(3,4-dimethoxy-phenyl)-butyl]-amino-ethyl]>-phenoxy-N-(4,4-dimethyl-3-nitro-oxy-pentyl-2)-acetamid

2-Methoxy-5-<2-(N-methyl-N-[4-cyano-4-(methylethyl)-4-(3,4-dimethoxy-phenyl)-butyl]-amino-ethyl]>phenoxy-N-(2,4-dimethyl-3-nitro-oxy-pentyl-2)-acetamid

2-Methoxy-5-<2-(N-methyl-N-[4-cyano-4-(methylethyl)-4-(3,4-dimethoxy-phenyl)-butyl]-amino-ethyl]>-phenoxy-N-(2,4,4-trimethyl-3-nitro-oxy-pentyl-2)-acetamid

2-Methoxy-5-<2-(N-methyl-N-[4-cyano-4-(methylethyl)-4-(3,4-dimethoxy-phenyl)-butyl]-amino-ethyl]>-phenoxy-N-(2,2-dimethyl-3-nitro-oxy-propyl-1)-acetamid

2-Methoxy-4-<2-(N-methyl-N-[4-cyano-4-(methylethyl)-4-(3-nitro-4-methoxy-phenyl)-butyl]-amino-ethyl]>-phenoxy-N-(2-nitro-oxy-propyl-1)-acetamid

2-Methoxy-4-<2-(N-methyl-N-[4-cyano-4-(methylethyl)-4-(3-amino-4-methoxy-phenyl)-butyl]-amino-ethyl]>-phenoxy-N-(2-nitro-oxy-propyl-1)-acetamid

2-Methoxy-4-<2-(N-methyl-N-[4-cyano-4-(methylethyl)-4-(3-acetamid-4-methoxy-phenyl)-butyl]-amino-ethyl]>-phenoxy-N-(2-nitro-oxy-propyl-1)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(nitro-oxy-ethyl)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(2-nitro-oxy-propyl)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(3-nitro-oxy-butyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(2-methyl-3-nitro-oxy-propyl-2)-acetamid

2-Methoxy-5-<2-(N-methyl-N-[4-cyano-4-(methylethyl)-4-(3,4,5-trimethoxy-phenyl)-butyl]-amino-ethyl]>-phenoxy-N-(1,3-bis-nitro-oxy-cyclohexyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(3-nitro-oxy-propyl-1)-acetamid

2-Methoxy-5-<2-[N-methyl-N-[4-cyano-4-(methylethyl)-4-(3,4,5-trimethoxy-phenyl)-butyl]-amino-ethyl]>-phenoxyessigsaeure-4-(nitro-oxy-methyl)-piperidylamid

2-Methoxy-5-<2-[N-methyl-N-[4-cyano-4-(methylethyl)-4-(3,4,5-trimethoxy-phenyl)-butyl]-amino-ethyl]>-phenoxyessigsaeure-4-(α-nitro-oxy-ethyl)-piperidylamid

2-Methoxy-5-<2-[N-methyl-N-[4-cyano-4-(methylethyl)-4-(3,4,5-trimethoxy-phenyl)-butyl]-amino-ethyl]>-phenoxy-N-(4-methyl-3-nitro-oxy-pentyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(4,4-dimethyl-3-nitro-oxy-pentyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(2,4-dimethyl-3-nitro-oxy-pentyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(2,4,4-trimethyl-3-nitro-oxy-pentyl-2)-acetamid

2-Methoxy-5-<2-[N-methyl-N-[4-cyano-4-(methylethyl)-4-(3,4,5-trimethoxy-phenyl)-butyl]-amino-ethyl]>-phenoxy-N-(2,2-dimethyl-3-nitro-oxy-propyl-1)-acetamid

2-Methoxy-4-<2-[N-methyl-N-[4-cyano-4-(methylethyl)-4-(3,4,5-trimethoxy-phenyl)-butyl]-amino-ethyl]>-phenoxy-N-(3-nitro-oxy-propyl-1)-acetamid

2-Methoxy-4-<2-[N-methyl-N-[4-cyano-4-(methylethyl)-4-(3,4,5-trimethoxy-phenyl)-butyl]-amino-ethyl]>-phenoxyessigsaeure-4-(nitro-oxy-methyl)-piperidylamid

2-Methoxy-4-<2-[N-methyl-N-[4-cyano-4-(methylethyl)-4-(3,4,5-trimethoxy-phenyl)-butyl]-amino-ethyl]>-phenoxyessigsaeure-4-(α-nitro-oxy-ethyl)-piperidylamid

2-Methoxy-4-<2-[N-methyl-N-[4-cyano-4-(methylethyl)-4-(3,4,5-trimethoxy-phenyl)-butyl]-amino-ethyl]>-phenoxy-N-(4-methyl-3-nitro-oxy-pentyl-2)-acetamid

2-Methoxy-4-<2-[N-methyl-N-[4-cyano-4-(methylethyl)-4-(3,4,5-trimethoxy-phenyl)-butyl]-amino-ethyl]>-phenoxy-N-(4,4-dimethyl-3-nitro-oxy-pentyl-2)-acetamid

2-Methoxy-4-<2-[N-methyl-N-[4-cyano-4-(methylethyl)-4-(3,4,5-trimethoxy-phenyl)-butyl]-amino-ethyl]>-phenoxy-N-(2,4-dimethyl-3-nitro-oxy-pentyl-2)-acetamid

2-Methoxy-4-<2-[N-methyl-N-[4-cyano-4-(methylethyl)-4-(3,4,5-trimethoxy-phenyl)-butyl]-amino-ethyl]>-phenoxy-N-(2,4,4-trimethyl-3-nitro-oxy-pentyl-2)-acetamid

2-Methoxy-4-<2-[N-methyl-N-[4-cyano-4-(methylethyl)-4-(3,4,5-trimethoxy-phenyl)-butyl]-amino-ethyl]>-phenoxy-N-(2,2-dimethyl-3-nitro-oxy-propyl-1)-acetamid

3-<2-[N-methyl-N-[4-cyano-4-dodecyl-4-(3,4,5-trimethoxy-phenyl)-butyl]-amino-ethyl]>-phenoxy-N-(2-nitro-oxy-proypl)-acetamid

3-<2-[N-methyl-N-[4-cyano-4-dodecyl-4-(3,4,5-trimethoxy-phenyl)-butyl]-amino-ethyl]>-phenoxy-N-(3-nitro-oxy-butyl-2)-acetamid

3-<2-[N-methyl-N-[4-cyano-4-dodecyl-4-(3,4,5-trimethoxy-phenyl)-butyl]-amino-ethyl]>-phenoxy-N-(3-nitro-oxy-propyl-1)-acetamid

3-<2-[N-methyl-N-[4-cyano-4-dodecyl-4-(3,4,5-trimethoxy-phenyl)-butyl]-amino-ethyl]>-phenoxyessigsaeure-4-(nitro-oxy-methyl)-piperidylamid

3-<2-[N-methyl-N-[4-cyano-4-dodecyl-4-(3,4,5-trimethoxy-phenyl)-butyl]-amino-ethyl]>-phenoxyessigsaeure-4-(α-nitro-oxy-ethyl)-piperidylamid

3-<2-[N-methyl-N-[4-cyano-4-dodecyl-4-(3,4,5-trimethoxy-phenyl)-butyl ]-amino-ethyl]>-phenoxy-N-(4-methyl-3-nitro-oxy-pentyl-2)-acetamid

3-<2-[N-methyl-N-[4-cyano-4-dodecyl-4-(3,4,5-trimethoxy-phenyl)-butyl]-amino-ethyl]>-phenoxy-N-(4,4-dimethyl-3-nitro-oxy-pentyl-2)-acetamid

3-<2-[N-methyl-N-[4-cyano-4-dodecyl-4-(3,4,5-trimethoxy-phenyl)-butyl]-amino-ethyl]>-phenoxy-N-(2,4-dimethyl-3-nitro-oxy-pentyl-2)-acetamid

3-<2-[N-methyl-N-[4-cyano-4-dodecyl-4-(3,4,5-trimethoxy-phenyl)-butyl]-amino-ethyl]>-phenoxy-N-(2,4,4-trimethyl-3-nitro-oxy-pentyl-2)-acetamid

3-<2-[N-methyl-N-[4-cyano-4-dodecyl-4-(3,4,5-trimethoxy-phenyl)-butyl]-amino-ethyl]>-phenoxy-N-(2,2-dimethyl-3-nitro-oxy-propyl-1)-acetamid

4-<2-[N-methyl-N-[4-cyano-4-dodecyl-4-(3,4,5-trimethoxy-phenyl)-butyl]-amino-ethyl]>-phenoxy-N-(nitro-oxy-ethyl)-acetamid

4-<2-[N-methyl-N-[4-cyano-4-dodecyl-4-(3,4,5-trimethoxy-phenyl)-butyl]-amino-ethyl]>-phenoxy-N-(2-nitro-oxy-propyl)-acetamid

4-<2-[N-methyl-N-[4-cyano-4-dodecyl-4-(3,4,5-trimethoxy-phenyl)-butyl]-amino-ethyl]>-phenoxy-N-(3-nitro-oxy-butyl-2)-acetamid

4-<2-[N-methyl-N-[4-cyano-4-dodecyl-4-(3,4,5-trimethoxy-phenyl)-butyl]-amino-ethyl]>-phenoxy-N-(3-nitro-oxy-propyl-1)-acetamid

4-<2-[N-methyl-N-[4-cyano-4-dodecyl-4-(3,4,5-trimethoxy-phenyl)-butyl]-amino-ethyl]>-phenoxyessigsaeure-4-(nitro-oxy-methyl)-piperidylamid

4-<2-[N-methyl-N-[4-cyano-4-dodecyl-4-(3,4,5-trimethoxy-phenyl)-butyl]-amino-ethyl]>-phenoxyessigsaeure-4-(α-nitro-oxy-ethyl)-piperidylamid

4-<2-[N-methyl-N-[4-cyano-4-dodecyl-4-(3,4,5-trimethoxy-phenyl)-butyl]-amino-ethyl]>-phenoxy-N-(4-methyl-3-nitro-oxy-pentyl-2)-acetamid

4-<2-[N-methyl-N-[4-cyano-4-dodecyl-4-(3,4,5-trimethoxy-phenyl)-butyl]-amino-ethyl]>-phenoxy-N-(4,4-dimethyl-3-nitro-oxy-pentyl-2)-acetamid

4-<2-[N-methyl-N-[4-cyano-4-dodecyl-4-(3,4,5-trimethoxy-phenyl)-butyl]-amino-ethyl]>-phenoxy-N-(2,4-dimethyl-3-nitro-oxy-pentyl-2)-acetamid

4-<2-[N-methyl-N-[4-cyano-4-dodecyl-4-(3,4,5-trimethoxy-phenyl)-butyl]-amino-ethyl]>-phenoxy-N-(2,4,4-trimethyl-3-nitro-oxy-pentyl-2)-acetamid

4-<2-[N-methyl-N-[4-cyano-4-dodecyl-4-(3,4,5-trimethoxy-phenyl)-butyl]-amino-ethyl]>-phenoxy-N-(2,2-dimethyl-3-nitro-oxy-propyl-1)-acetamid

3-<2-[N-methyl-N-(4-cyano-4-dodecyl-4-(3,4-dimethoxy-phenyl)-butyl]-amino-ethyl]>-phenoxy-N-(3-nitro-oxy-butyl-2)-acetamid

3-<2-[N-methyl-N-(4-cyano-4-dodecyl-4-(3,4-dimethoxy-phenyl)-butyl]-amino-ethyl]>-phenoxy-N-(3-nitro-oxy-propyl-1)-acetamid

3-<2-[N-methyl-N-(4-cyano-4-dodecyl-4-(3,4-dimethoxy-phenyl)-butyl]-amino-ethyl]>-phenoxyessigsaeure-4-(nitro-oxy-methyl)-piperidylamid

3-<2-[N-methyl-N-(4-cyano-4-dodecyl-4-(3,4-dimethoxy-phenyl)-butyl]-amino-ethyl]>-phenoxyessigsaeure-4-(α-nitro-oxy-ethyl)-piperidylamid

3-<2-[N-methyl-N-(4-cyano-4-dodecyl-4-(3,4-dimethoxy-phenyl)-butyl]-amino-ethyl]>-phenoxy-N-(4-methyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(4,4-dimethyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(2,4-dimethyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(2,4,4-trimethyl-3-nitro-oxy-pentyl-2)-acetamid

3-<3-[N-methyl-N-[4-cyano-4-dodecyl-4-(3,4-dimethoxy-phenyl)-butyl]-amino-ethyl]>-phenoxy-N-(2,2-dimethyl-3-nitro-oxy-propyl-1)-acetamid

4-<2-[N-methyl-N-[4-cyano-4-dodecyl-4-(3,4-dimethoxy-phenyl)-butyl]-amino-ethyl]>-phenoxy-N-(nitro-oxy-ethyl)-acetamid

4-<2-[N-methyl-N-[4-cyano-4-dodecyl-4-(3,4-dimethoxy-phenyl)-butyl]-amino-ethyl]>-phenoxy-N-(2-nitro-oxy-propyl)-acetamid

4-<2-[N-methyl-N-[4-cyano-4-dodecyl-4-(3,4-dimethoxy-phenyl)-butyl]-amino-ethyl]>-phenoxy-N-(3-nitro-oxy-butyl-2)-acetamid

4-<2-[N-methyl-N-[4-cyano-4-dodecyl-4-(3,4-dimethoxy-phenyl)-butyl]-amino-ethyl]>-phenoxy-N-(3-nitro-oxy-propyl-1)-acetamid

4-<2-[N-methyl-N-[4-cyano-4-dodecyl-4-(3,4-dimethoxy-phenyl)-butyl]-amino-ethyl]>-phenoxyessigsaeure-4-(nitro-oxy-methyl)-piperidylamid

4-<2-[N-methyl-N-[4-cyano-4-dodecyl-4-(3,4-dimethoxy-phenyl)-butyl]-amino-ethyl]>-phenoxyessigsaeure-4-(α-nitro-oxy-ethyl)-piperidylamid

4-<2-[N-methyl-N-[4-cyano-4-dodecyl-4-(3,4-dimethoxy-phenyl)-butyl]-amino-ethyl]>-phenoxy-N-(4-methyl-3-nitro-oxy-pentyl-2)-acetamid

4-<2-[N-methyl-N-[4-cyano-4-dodecyl-4-(3,4-dimethoxy-phenyl)-butyl]-amino-ethyl]>-phenoxy-N-(4,4-dimethyl-3-nitro-oxy-pentyl-2)-acetamid

4-<2-[N-methyl-N-[4-cyano-4-dodecyl-4-(3,4-dimethoxy-phenyl)-butyl]-amino-ethyl]>-phenoxy-N-(2,4-dimethyl-3-nitro-oxy-pentyl-2)-acetamid

4-<2-[N-methyl-N-[4-cyano-4-dodecyl-4-(3,4-dimethoxy-phenyl)-butyl]-amino-ethyl]>-phenoxy-N-(2,4,4,-trimethyl-3-nitro-oxy-pentyl-2)-acetamid

4-<2-[N-methyl-N-[4-cyano-4-dodecyl-4-(3,4-dimethoxy-phenyl)-butyl]-amino-ethyl]>-phenoxy-N-(2,2-dimethyl-3-nitro-oxy-propyl-1)-acetamid

4-<2-[N-methyl-N-[4-cyano-4-hexyl-4-(3,4,5-trimethoxy-phenyl)-butyl]-amino-ethyl]>-phenoxy-N-(nitro-oxy-ethyl)-acetamid

4-<2-[N-methyl-N-[4-cyano-4-hexyl-4-(3,4,5-trimethoxy-phenyl)-butyl]-amino-ethyl]>-phenoxy-N-(2-nitro-oxy-propyl)-acetamid

4-<2-[N-methyl-N-[4-cyano-4-hexyl-4-(3,4,5-trimethoxy-phenyl)-butyl]-amino-ethyl]>-phenoxy-N-(3-nitro-oxy-butyl-2)-acetamid

4-<2-[N-methyl-N-[4-cyano-4-hexyl-4-(3,4,5-trimethoxy-phenyl)-butyl]-amino-ethyl]>-phenoxy-N-(3-nitro-oxy-propyl-1)-acetamid

4-<2-[N-methyl-N-[4-cyano-4-hexyl-4-(3,4,5-trimethoxy-phenyl)-butyl]-amino-ethyl]>-phenoxyessigsaeure-4-(nitro-oxy-methyl)-piperidylamid

4-<2-[N-methyl-N-[4-cyano-4-hexyl-4-(3,4,5-trimethoxy-phenyl)-butyl]-amino-ethyl]>-phenoxyessigsaeure-4-(α-nitro-oxy-ethyl)-piperidylamid

4-<2-[N-methyl-N-[4-cyano-4-hexyl-4-(3,4,5-trimethoxy-phenyl)-butyl]-amino-ethyl]>-phenoxy-N-(4-methyl-3-nitro-oxy-pentyl-2)-acetamid

4-<2-[N-methyl-N-[4-cyano-4-hexyl-4-(3,4,5-trimethoxy-phenyl)-butyl]-amino-ethyl]>-phenoxy-N-(4,4-dimethyl-3-nitro-oxy-pentyl-2)-acetamid

4-<2-[N-methyl-N-[4-cyano-4-hexyl-4-(3,4,5-trimethoxy-phenyl)-butyl]-amino-ethyl]>-phenoxy-N-(2,4-dimethyl-3-nitro-oxy-pentyl-2)-acetamid

4-<2-[N-methyl-N-[4-cyano-4-hexyl-4-(3,4,5-trimethoxy-phenyl)-butyl]-amino-ethyl]>-phenoxy-N-(2,4,4-trimethyl-3-nitro-oxy-pentyl-2)-acetamid

4-<2-[N-methyl-N-[4-cyano-4-hexyl-4-(3,4,5-trimethoxy-phenyl)-butyl]-amino-ethyl]>-phenoxy-N-(2,2-dimethyl-3-nitro-oxy-propyl-1)-acetamid

4-<2-[N-methyl-N-[4-cyano-4-dodecyl-4-(3,4-dimethoxy-phenyl)-butyl]-amino-ethyl]>-phenoxy-N-(nitro-oxy-ethyl)-acetamid

4-<2-[N-methyl-N-[4-cyano-4-dodecyl-4-(3,4-dimethoxy-phenyl)-butyl]-amino-ethyl]>-phenoxy-N-(2-nitro-oxy-propyl)-acetamid

3-<2-[N-methyl-N-[4-cyano-4-hexyl-4-(3,4-dimethoxy-phenyl)-butyl]-amino-ethyl]>-phenoxy-N-(3-nitro-oxy-butyl-2)-acetamid

3-<2-[N-methyl-N-[4-cyano-4-hexyl-4-(3,4-dimethoxy-phenyl)-butyl]-amino-ethyl]>-phenoxy-N-(3-nitro-oxy-propyl-1)-acetamid

3-<2-[N-methyl-N-[4-cyano-4-hexyl-4-(3,4-dimethoxy-phenyl)-butyl]-amino-ethyl]>-phenoxyessigsaeure-4-(nitro-oxy-methyl)-piperidylamid

3-<2-[N-methyl-N-[4-cyano-4-hexyl-4-(3,4-dimethoxy-phenyl)-butyl]-amino-ethyl]>-phenoxyessigsaeure-4-(α-nitro-oxy-ethyl)-piperidylamid

3-<2-[N-methyl-N-[4-cyano-4-hexyl-4-(3,4-dimethoxy-phenyl)-butyl]-amino-ethyl]>-phenoxy-N-(4-methyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(4,4-dimethyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(2,4-dimethyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(2,4,4-trimethyl-3-nitro-oxy-pentyl-2)-acetamid

3-<2-[N-methyl-N-[4-cyano-4-dodecyl-4-(3,4-dimethoxy-phenyl)-butyl]-amino-ethyl]>-phenoxy-N-(2,2-dimethyl-3-nitro-oxy-propyl-1)-acetamid

4-<1-cyano-1-hexyl-4-[N-methyl-N-[2-(3-methoxy-phenyl)-ethyl]-amino-butyl]>-phenoxy-N-(nitro-oxy-ethyl)-acetamid

4-<1-cyano-1-hexyl-4-[N-methyl-N-[2-(3-methoxy-phenyl)-ethyl)-amino-butyl]>-phenoxy-N-(2-nitro-oxy-propyl)-acetamid

4-<1-cyano-1-hexyl-4-[N-methyl-N-[2-(3-methoxy-phenyl)-ethyl]-amino-butyl]>-phenoxy-N-(3-nitro-oxy-propyl-1)-acetamid

4-<1-cyano-1-hexyl-4-[N-methyl-N-[2-(3-methoxy-phenyl)-ethyl]-amino-butyl]>-phenoxyessigsaeure-4-(nitro-oxy-methyl)-piperidylamid

4-<1-cyano-1-hexyl-4-[N-methyl-N-[2-(3-methoxy-phenyl)-ethyl]-amino-butyl]>-phenoxyessigsaeure-4-(α-nitro-oxy-ethyl)-piperidylamid

4-<1-cyano-1-hexyl-4-[N-methyl-N-[2-(3-methoxy-phenyl)-ethyl]-amino-butyl]>-phenoxy-N-(4-methyl-3-nitro-oxy-pentyl-2)-acetamid

4-<1-cyano-1-hexyl-4-[N-methyl-N-[2-(3-methoxy-phenyl)-ethyl]-amino-butyl]>-phenoxy-N-(4,4-dimethyl-3-nitro-oxy-pentyl-2)-acetamid

4-<1-cyano-1-hexyl-4-[N-methyl-N-[2-(3-methoxy-phenyl)-ethyl]-amino-butyl]>-phenoxy-N-(2,4-dimethyl-3-nitro-oxy-pentyl-2)-acetamid

4-<1-cyano-1-hexyl-4-[N-methyl-N-[2-(3-methoxy-phenyl)-ethyl]-amino-butyl]>-phenoxy-N-(2,4,4-trimethyl-3-nitro-oxy-pentyl-2)-acetamid

4-<1-cyano-1-hexyl-4-[N-methyl-N-[2-(3-methoxy-phenyl)-ethyl]-amino-butyl]>-phenoxy-N-(2,2-dimethyl-3-nitro-oxy-propyl-1)-acetamid

4-<1-cyano-1-hexyl-4-[N-methyl-N-[2-(4-methoxy-phenyl)-ethyl]-amino-butyl]>-phenoxy-N-(nitro-oxy-ethyl)-acetamid

4-<1-cyano-1-hexyl-4-[N-methyl-N-[2-(4-methoxy-phenyl)-ethyl]-amino-butyl]>-phenoxy-N-(2-nitro-oxy-propyl)-acetamid

4-<1-cyano-1-hexyl-4-[N-methyl-N-[2-(4-methoxy-phenyl)-ethyl]-amino-butyl]>-phenoxy-N-(3-nitro-oxy-propyl-1)-acetamid

4-<1-cyano-1-hexyl-4-[N-methyl-N-[2-(4-methoxy-phenyl)-ethyl]-amino-butyl]>-phenoxyessigsaeure-4-(nitro-oxy-methyl)-piperidylamid

4-<1-cyano-1-hexyl-4-[N-methyl-N-[2-(4-methoxy-phenyl)-ethyl]-amino-butyl]>-phenoxyessigsaeure-4-(α-nitro-oxy-ethyl)-piperidylamid

4-<1-cyano-1-hexyl-4-[N-methyl-N-[2-(4-methoxy-phenyl)-ethyl]-amino-butyl]>-phenoxy-N-(4-methyl-3-nitro-oxy-pentyl-2)-acetamid

4-<1-cyano-1-hexyl-4-[N-methyl-N-[2-(4-methoxy-phenyl)-ethyl]-amino-butyl]>-phenoxy-N-(4,4-dimethyl-3-nitro-oxy-pentyl-2)-acetamid

4-<1-cyano-1-hexyl-4-[N-methyl-N-[2-(4-methoxy-phenyl)-ethyl]-amino-butyl]>-phenoxy-N-(2,4-dimethyl-3-nitro-oxy-pentyl-2)-acetamid

4-<1-cyano-1-hexyl-4-[N-methyl-N-[2-(4-methoxy-phenyl)-ethyl]-amino-butyl]>-phenoxy-N-(2,4,4-trimethyl-3-nitro-oxy-pentyl-2)-acetamid

4-<1-cyano-1-hexyl-4-[N-methyl-N-[2-(4-methoxy-phenyl)-ethyl]-amino-butyl]>-phenoxy-N-(2,2-dimethyl-3-nitro-oxy-propyl-1)-acetamid

3-<1-cyano-1-hexyl-4-[N-methyl-N-[2-(3-methoxy-phenyl)-ethyl]-amino-butyl]>-phenoxy-N-(nitro-oxy-ethyl)-acetamid

3-<1-cyano-1-hexyl-4-[N-methyl-N-[2-(3-methoxy-phenyl)-ethyl)-amino-butyl]>-phenoxy-N-(2-nitro-oxy-propyl)-acetamid

3-<1-cyano-1-hexyl-4-[N-methyl-N-[2-(3-methoxy-phenyl)-ethyl]-amino-butyl]>-phenoxy-N-(3-nitro-oxy-propyl-1)-acetamid

3-<1-cyano-1-hexyl-4-[N-methyl-N-[2-(3-methoxy-phenyl)-ethyl]-amino-butyl]>-phenoxyessigsaeure-4-(nitro-oxy-methyl)-piperidylamid

3-<1-cyano-1-hexyl-4-[N-methyl-N-[2-(3-methoxy-phenyl)-ethyl]-amino-butyl]>-phenoxyessigsaeure-4-(α-nitro-oxy-ethyl)-piperidylamid

3-<1-cyano-1-hexyl-4-[N-methyl-N-[2-(3-methoxy-phenyl)-ethyl]-amino-butyl]>-phenoxy-N-(4-methyl-3-nitro-oxy-pentyl-2)-acetamid

3-<1-cyano-1-hexyl-4-[N-methyl-N-[2-(3-methoxy-phenyl)-ethyl]-amino-butyl]>-phenoxy-N-(4,4-dimethyl-3-nitro-oxy-pentyl-2)-acetamid

3-<1-cyano-1-hexyl-4-[N-methyl-N-[2-(3-methoxy-phenyl)-ethyl]-amino-butyl]>-phenoxy-N-(2,4-dimethyl-3-nitro-oxy-pentyl-2)-acetamid

3-<1-cyano-1-hexyl-4-[N-methyl-N-[2-(3-methoxy-phenyl)-ethyl]-amino-butyl]>-phenoxy-N-(2,4,4-trimethyl-3-nitro-oxy-pentyl-2)-acetamid

3-<1-cyano-1-hexyl-4-[N-methyl-N-[2-(3-methoxy-phenyl)-ethyl]-amino-butyl]>-phenoxy-N-(2,2-dimethyl-3-nitro-oxy-propyl-1)-acetamid

3-<1-cyano-1-hexyl-4-[N-methyl-N-[2-(4-methoxy-phenyl)-ethyl]-amino-butyl]>-phenoxy-N-(nitro-oxy-ethyl)-acetamid

3-<1-cyano-1-hexyl-4-[N-methyl-N-[2-(4-methoxy-phenyl)-ethyl]-amino-butyl]>-phenoxy-N-(2-nitro-oxy-propyl)-acetamid

3-<1-cyano-1-hexyl-4-[N-methyl-N-[2-(4-methoxy-phenyl)-ethyl]-amino-butyl]>-phenoxy-N-(3-nitro-oxy-propyl-1)-acetamid

3-<1-cyano-1-hexyl-4-[N-methyl-N-[2-(4-methoxy-phenyl)-ethyl]-amino-butyl]>-phenoxyessigsaeure-4-(nitro-oxy-methyl)-piperidylamid

3-<1-cyano-1-hexyl-4-[N-methyl-N-[2-(4-methoxy-phenyl)-ethyl]-amino-butyl]>-phenoxyessigsaeure-4-(α-nitro-oxy-ethyl)-piperidylamid

3-<1-cyano-1-hexyl-4-[N-methyl-N-[2-(4-methoxy-phenyl)-ethyl]-amino-butyl]>-phenoxy-N-(4-methyl-3-nitro-oxy-pentyl-2)-acetamid

3-<1-cyano-1-hexyl-4-[N-methyl-N-[2-(4-methoxy-phenyl)-ethyl]-amino-butyl]>-phenoxy-N-(4,4-dimethyl-3-nitro-oxy-pentyl-2)-acetamid

3-<1-cyano-1-hexyl-4-[N-methyl-N-[2-(4-methoxy-phenyl)-ethyl]-amino-butyl]>-phenoxy-N-(2,4-dimethyl-3-nitro-oxy-pentyl-2)-acetamid

3-<1-cyano-1-hexyl-4-[N-methyl-N-[2-(4-methoxy-phenyl)-ethyl]-amino-butyl]>-phenoxy-N-(2,4,4-trimethyl-3-nitro-oxy-pentyl-2)-acetamid

3-<1-cyano-1-hexyl-4-[N-methyl-N-[2-(4-methoxy-phenyl)-ethyl]-amino-butyl]>-phenoxy-N-(2,2-dimethyl-3-nitro-oxy-propyl-1)-acetamid

α-Isopropyl-α-[(N-methyl-N-homoveratryl)-amino-γ-propyl]-3-methoxy-4-(2-nitro-oxy-ethoxy)-phenylacetonitril

α-Isopropyl-α[(N-methyl-N-homoveratryl)-amino-γ-propyl]-3-methoxy-4-(2-nitro-oxy-propoxy)-phenylacetonitril

α-Isopropyl-α-[(N-methyl-N-homoveratryl)-amino-γ-propyl]-3-methoxy-4-(3-amino-2-nitro-oxy-propoxy)-phenylacetonitril

α-Isopropyl-α-[(N-methyl-N-homoveratryl)-amino-γ-propyl]-3-methoxy-4-(3-dimethylamino-2-nitro-oxy-propoxy)-phenylacto-nitril

α-Isopropyl-α-[(N-methyl-N-homoveratryl)-amino-γ-propyl]-3-(2-nitro-oxy-ethoxy)-4-methoxy-phenylacetonitril

α-Isopropyl-α-[(N-methyl-N-homoveratryl)-amino-γ-propyl]-3-(2-nitro-oxy-propoxy)-4-methoxy-phenylacetonitril

α-Isopropyl-α-[(N-methyl-N-homoveratryl)-amino-γ-propyl]-3-(3-amino-2-nitro-oxy-propoxy)-4-methoxy-phenylacetonitril

α-Isopropyl-α-[(N-methyl-N-homoveratryl)-amino-γ-propyl]-3-(3-dimethylamino-2-nitro-oxy-propoxy)-4-methoxy-phenylacto-nitril

α-Isopropyl-α-<[N-methyl-N-[2-(3-methoxy-4-(2-nitro-oxy-ethyoxy)-phenyl)ethyl]]-amino-γ-propyl>-3,4-dimethoxy-phenylacetonitril

α-Isopropyl-α-<[N-methyl-N-[2-(3-methoxy-4-(2-nitro-oxy-propoxy)phenyl)-ethyl]]-amino-γ-propyl>-3,4-dimethoxy-phenyl-acetonitril

α-Isopropyl-α-<[N-methyl-N-[2-(3-methoxy-4-(3-amino-2-nitro-oxy-propoxy)phenyl)-ethyl]]-amino-γ-propyl>-3,4-dimethoxy-phenylacetonitril

α-Isopropyl-α-<[N-methyl-N-[2-(3-methoxy-4-(3-dimethylamino-2-nitro-oxy-propoxy)phenyl)-ethyl]]-amino-γ-propyl>-3,4-dimethoxy-phenylacetonitril

α-Isopropyl-α-<[N-methyl-N-[2-(4-methoxy-3-(2-nitro-oxy-ethoxy)-phenyl)-ethyl]]-amino-γ-propyl>-3,4-dimethoxy-phenylacetonitril

α-Isopropyl-α-<[N-methyl-N-[2-(4-methoxy-3-(2-nitro-oxy-propoxy)-phenyl)-ethyl]]-amino-γ-propyl-3,4-dimethoxy-phenylacetonitril

α-Isopropyl-α-<[N-methyl-N-[2-(4-methoxy-3-(3-amino-2-nitro-oxy-propoxy)-phenyl)-ethyl]]-amino-γ-propyl>-3,4-dimethoxy-phenylacetonitril

α-Isopropyl-α-<[N-methyl-N-[2-(4-methoxy-3-(3-dimethylamino-2-nitro-oxy-propoxy)-phenyl)-ethyl]]-amino-γ-propyl>-3,4-dimethoxy-phenylacetonitril

2-(Nitro-oxy)-N-<2-methoxy-4-<1-cyano-1-(methylethyl)-4-[N-methyl-N-[2-(3,4-dimethoxy-phenyl)ethyl]-amino-butyl]>-phenoxy-ethyl-2>-propionsäureamid

4-(Nitro-oxy)-N-<2-methoxy-4-<1-cyano-1-(methylethyl)-4-[N-methyl-N-[2-(3,4-dimethoxy-phenyl)-ethyl]-amino-butyl]>-phenoxy-ethyl-2>-buttersäureamid

3-(Nitro-oxy)-2,2-dimethyl-N-<2-methoxy-4-<1-cyano-1-(methylethyl)-4-[N-methyl-N-[2-(3,4-dimethoxy-phenyl)-ethyl]-amino-butyl]>-phenoxy-ethyl-2>-buttersäureamid

2-(Nitro-oxy)-N-<2-methoxy-4-<1-cyano-1-(methylethyl)-4-[N-methyl-N-[2-(3,4-dimethoxy-phenyl)-ethyl]-amino-butyl]>-phenoxy-ethyl-2>-cyclohexan-carbonsäureamid

4-(Nitro-oxy)-N-<2-methoxy-4-<1-cyano-1-(methylethyl)-
4-[N-methyl-N-[2-(3,4-dimethoxy-phenyl)-ethyl]-amino-butyl]>-
phenoxy-ethyl-2>-cyclohexancarbonsäureamid

2-(Nitro-oxy)-N-<2-methoxy-4-<2-<N-methyl-N-[4-cyano-
4-(methylethyl)-4-(3,4-dimethoxy-phenyl)butyl]-aminoethyl>>-
phenoxy-ethyl-2>-propionsäureamid

4-(Nitro-oxy)-N-<2-methoxy-4-<2-<N-methyl-N-[4-cyano-
4-(methylethyl)-4-(3,4-dimethoxy-phenyl)-butyl]-amino-ethyl>>-
phenoxy-ethyl-2>buttersäureamid

3-(Nitro-oxy)-2,2-dimethyl-N-<2-methoxy-4-<2-<N-methyl-
N-[4-cyano-4-(methylethyl)-4-(3,4-dimethoxy-phenyl)-butyl]-
amino-ethyl>>-phenoxy-ethyl-2>-buttersäureamid

2-(Nitro-oxy)-N-<2-methoxy-4-<2-<N-methyl-N-[4-cyano-
4-(methylethyl)-4-(3,4-dimethoxy-phenyl)-butyl]-amino-ethyl>>-
phenoxy-ethyl-2>-cyclohexancarbonsäureamid

4-(Nitro-oxy)-N-<2-methoxy-4-<2-<N-methyl-N-[4-cyano-
4-(methylethyl)-4-(3,4-dimethoxy-phenyl)-butyl]-amino-ethyl>>-
phenoxy-ethyl-2>-cyclohexancarbonsäureamid

Weiterhin bevorzugt sind die analogen Verbindungen, die an Stelle der "Nitro-oxy"-Gruppe eine Hydroxy-gruppe besitzen als Ausgangsstoffe, sowie ggf. als pharmakologisch wirksame Substanzen.

In den folgenden Tabellen sind weitere, im Sinne der Erfindung brauchbare Verbindungen der Übersichtlichkeit wegen tabellarisch wiedergegeben. Auf die Wiedergabe der entsprechenden Substanznamen, die der Fachmann ohne weiteres ergänzen kann, wurde verzichtet.

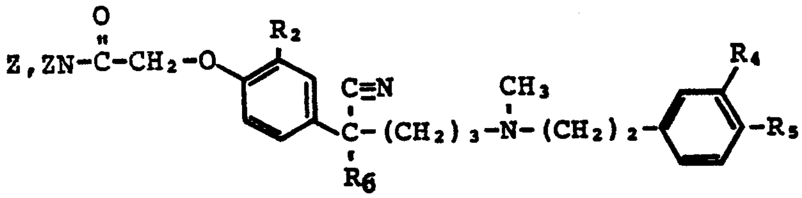

| $-NZ,Z$ | $R_2$ | $R_6$ | $R_4$ | $R_5$ |
|---|---|---|---|---|
| 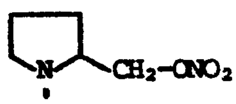 | $-OCH_3$ | $-CH(CH_3)_2$ | $-OCH_3$ | $-OCH_3$ |
| 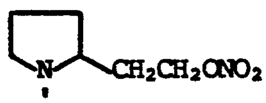 | $-OCH_3$ | $-CH(CH_3)_2$ | $-OCH_3$ | $-OCH_3$ |
| 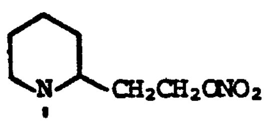 | $-OCH_3$ | $-CH(CH_3)_2$ | $-OCH_3$ | -OCH |
| 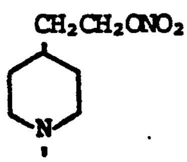 | $-OCH_3$ | $-CH(CH_3)_2$ | $-OCH_3$ | $-OCH_3$ |
| 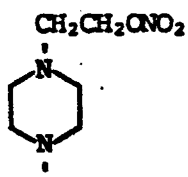 | $-OCH_3$ | $-CH(CH_3)_2$ | $-OCH_3$ | -OCH |
| 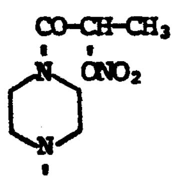 | $-OCH_3$ | $-CH(CH_3)_2$ | $-OCH_3$ | -OCH |
| 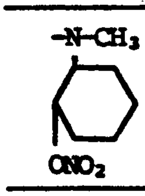 | $-OCH_3$ | $-CH(CH_3)_2$ | $-OCH_3$ | -OCH |

| -NZ,Z | $R_2$ | $R_6$ | $R_4$ | $R_5$ |
|---|---|---|---|---|
| 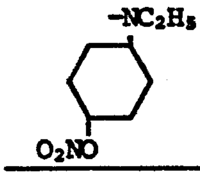 | $-OCH_3$ | $-CH(CH_3)_2$ | $-OCH_3$ | $-OCH_3$ |
| 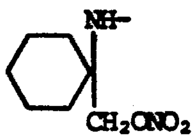 | $-OCH_3$ | $-CH(CH_3)_2$ | $-OCH_3$ | $-OCH_3$ |
| 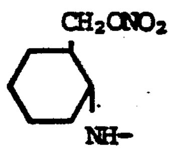 | $-OCH_3$ | $-CH(CH_3)_2$ | $-OCH_3$ | $-OCH_3$ |
| 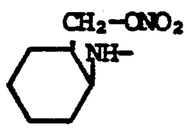 | $-OCH_3$ | $-CH(CH_3)_2$ | $-OCH_3$ | $-OCH_3$ |
| 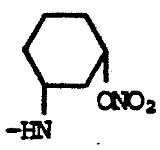 | $-OCH_3$ | $-CH(CH_3)_2$ | $-OCH_3$ | $-OCH_3$ |
| 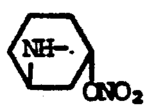 | $-OCH_3$ | $-CH(CH_3)_2$ | $-OCH_3$ | $-OCH_3$ |
| 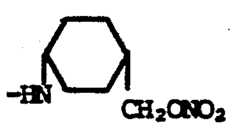 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 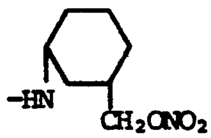 | $-OCH_3$ | $-CH(CH_3)_2$ | $-OCH_3$ | $-OCH_3$ |
| 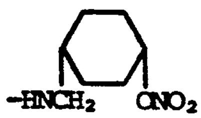 | $-OCH_3$ | $-CH(CH_3)_2$ | $-OCH_3$ | $-OCH_3$ |
| 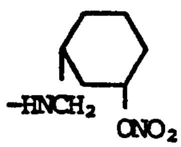 | $-OCH_3$ | $-CH(CH_3)_2$ | $-OCH_3$ | $-OCH_3$ |
| 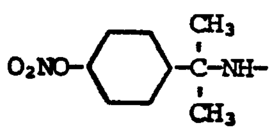 cis u. trans | $-OCH_3$ | $-CH(CH_3)_2$ | $-OCH_3$ | $-OCH_3$ |

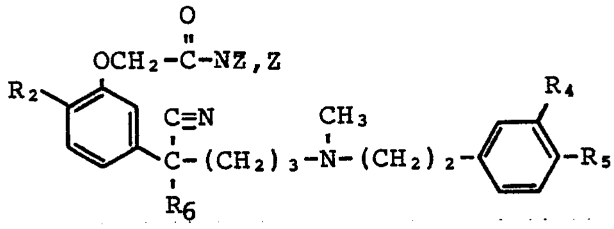

| -NZ,Z | $R_2$ | $R_6$ | $R_4$ | $R_5$ |
|---|---|---|---|---|
| 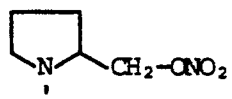 | $-OCH_3$ | $-CH(CH_3)_2$ | $-OCH_3$ | $-OCH_3$ |
| 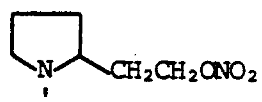 | $-OCH_3$ | $-CH(CH_3)_2$ | $-OCH_3$ | $-OCH_3$ |
| 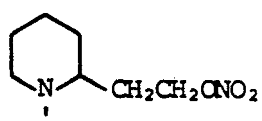 | $-OCH_3$ | $-CH(CH_3)_2$ | $-OCH_3$ | $-OCH_3$ |
| 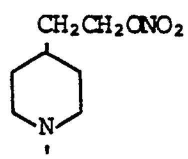 | $-OCH_3$ | $-CH(CH_3)_2$ | $-OCH_3$ | $-OCH_3$ |
| 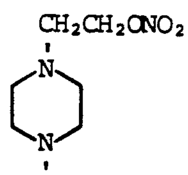 | $-OCH_3$ | $-CH(CH_3)_2$ | $-OCH_3$ | $-OCH_3$ |
| 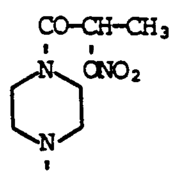 | $-OCH_3$ | $-CH(CH_3)_2$ | $-OCH_3$ | $-OCH_3$ |
| 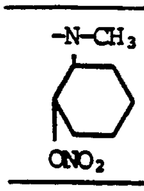 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 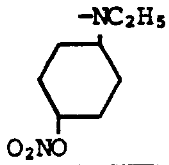 | $-OCH_3$ | $-CH(CH_3)_2$ | $-OCH_3$ | $-OCH_3$ |
| 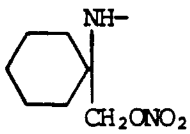 | $-OCH_3$ | $-CH(CH_3)_2$ | $-OCH_3$ | $-OCH_3$ |
| 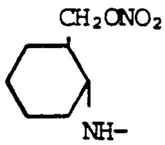 | $-OCH_3$ | $-CH(CH_3)_2$ | $-OCH_3$ | $-OCH_3$ |
| 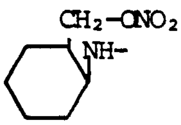 | $-OCH_3$ | $-CH(CH_3)_2$ | $-OCH_3$ | $-OCH_3$ |
| 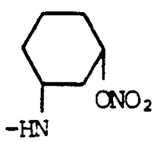 | $-OCH_3$ | $-CH(CH_3)_2$ | $-OCH_3$ | $-OCH_3$ |
| 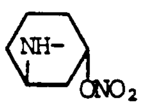 | $-OCH_3$ | $-CH(CH_3)_2$ | $-OCH_3$ | $-OCH_3$ |
| 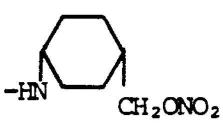 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 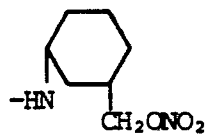 | $-OCH_3$ | $-CH(CH_3)_2$ | $-OCH_3$ | $-OCH_3$ |
| 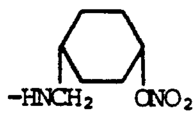 | $-OCH_3$ | $-CH(CH_3)_2$ | $-OCH_3$ | $-OCH_3$ |
| 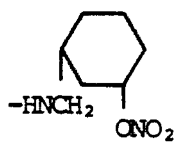 | $-OCH_3$ | $-CH(CH_3)_2$ | $-OCH_3$ | $-OCH_3$ |
| 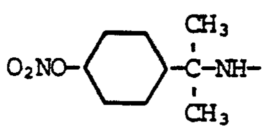 cis u. trans | $-OCH_3$ | $-CH(CH_3)_2$ | $-OCH_3$ | $-OCH_3$ |

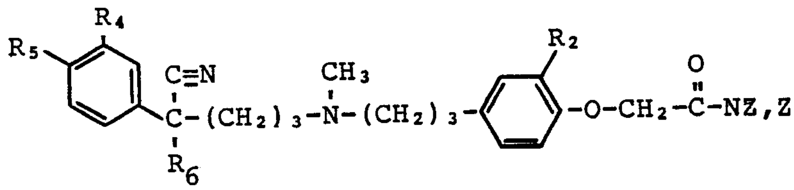

| -NZ,Z | $R_2$ | $R_6$ | $R_4$ | $R_5$ |
|---|---|---|---|---|
| 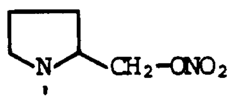 | $-OCH_3$ | $-CH(CH_3)_2$ | $-OCH_3$ | $-OCH_3$ |
| 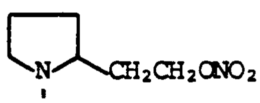 | $-OCH_3$ | $-CH(CH_3)_2$ | $-OCH_3$ | $-OCH_3$ |
| 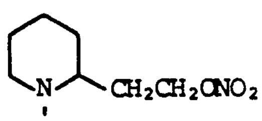 | $-OCH_3$ | $-CH(CH_3)_2$ | $-OCH_3$ | $-OCH_3$ |
| 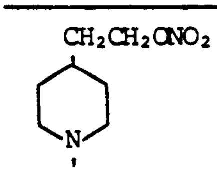 | $-OCH_3$ | $-CH(CH_3)_2$ | $-OCH_3$ | $-OCH_3$ |
| 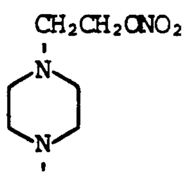 | $-OCH_3$ | $-CH(CH_3)_2$ | $-OCH_3$ | $-OCH_3$ |
| 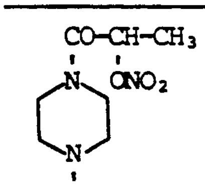 | $-OCH_3$ | $-CH(CH_3)_2$ | $-OCH_3$ | $-OCH_3$ |
| 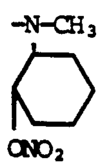 | $-OCH_3$ | $-CH(CH_3)_2$ | $-OCH_3$ | $-OCH_3$ |

| -NZ, Z | $R_2$ | $R_6$ | $R_4$ | $R_5$ |
|---|---|---|---|---|
| 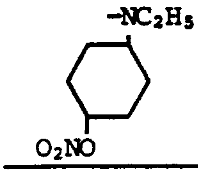 | $-OCH_3$ | $-CH(CH_3)_2$ | $-OCH_3$ | $-OCH_3$ |
| 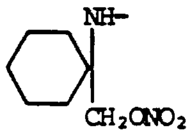 | $-OCH_3$ | $-CH(CH_3)_2$ | $-OCH_3$ | $-OCH_3$ |
| 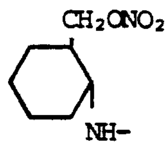 | $-OCH_3$ | $-CH(CH_3)_2$ | $-OCH_3$ | $-OCH_3$ |
| 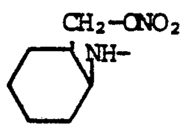 | $-OCH_3$ | $-CH(CH_3)_2$ | $-OCH_3$ | $-OCH_3$ |
| 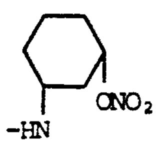 | $-OCH_3$ | $-CH(CH_3)_2$ | $-OCH_3$ | $-OCH_3$ |
| 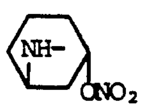 | $-OCH_3$ | $-CH(CH_3)_2$ | $-OCH_3$ | $-OCH_3$ |
| 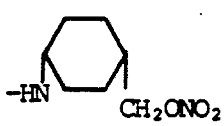 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 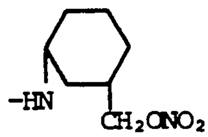 | $-OCH_3$ | $-CH(CH_3)_2$ | $-OCH_3$ | $-OCH_3$ |
| 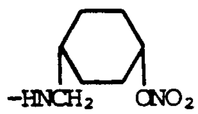 | $-OCH_3$ | $-CH(CH_3)_2$ | $-OCH_3$ | $-OCH_3$ |
| 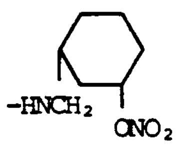 | $-OCH_3$ | $-CH(CH_3)_2$ | $-OCH_3$ | $-OCH_3$ |
| 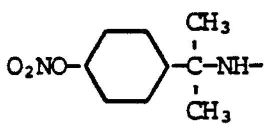 cis u. trans | $-OCH_3$ | $-CH(CH_3)_2$ | $-OCH_3$ | $-OCH_3$ |

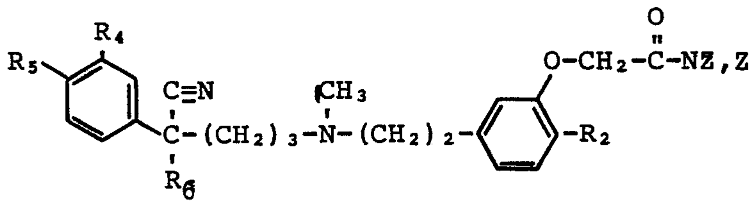

| -NZ,Z | $R_2$ | $R_6$ | $R_4$ | $R_5$ |
|---|---|---|---|---|
| 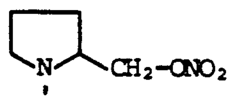 | $-OCH_3$ | $-CH(CH_3)_2$ | $-OCH_3$ | $-OCH_3$ |
| 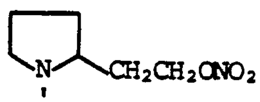 | $-OCH_3$ | $-CH(CH_3)_2$ | $-OCH_3$ | $-OCH_3$ |
| 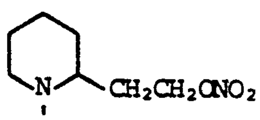 | $-OCH_3$ | $-CH(CH_3)_2$ | $-OCH_3$ | $-OCH_3$ |
| 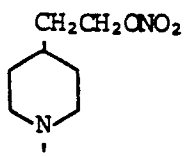 | $-OCH_3$ | $-CH(CH_3)_2$ | $-OCH_3$ | $-OCH_3$ |
| 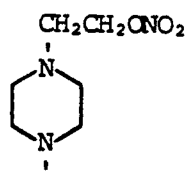 | $-OCH_3$ | $-CH(CH_3)_2$ | $-OCH_3$ | $-OCH_3$ |
| 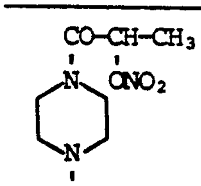 | $-OCH_3$ | $-CH(CH_3)_2$ | $-OCH_3$ | $-OCH_3$ |
| 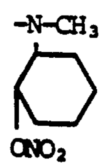 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 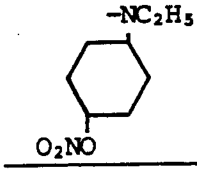 | $-OCH_3$ | $-CH(CH_3)_2$ | $-OCH_3$ | $-OCH_3$ |
| 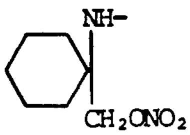 | $-OCH_3$ | $-CH(CH_3)_2$ | $-OCH_3$ | $-OCH_3$ |
| 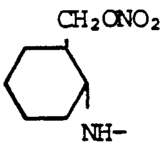 | $-OCH_3$ | $-CH(CH_3)_2$ | $-OCH_3$ | $-OCH_3$ |
| 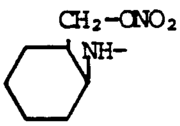 | $-OCH_3$ | $-CH(CH_3)_2$ | $-OCH_3$ | $-OCH_3$ |
| 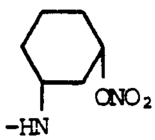 | $-OCH_3$ | $-CH(CH_3)_2$ | $-OCH_3$ | $-OCH_3$ |
| 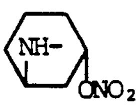 | $-OCH_3$ | $-CH(CH_3)_2$ | $-OCH_3$ | $-OCH_3$ |
| 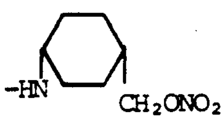 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 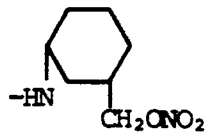 | $-OCH_3$ | $-CH(CH_3)_2$ | $-OCH_3$ | $-OCH_3$ |
| 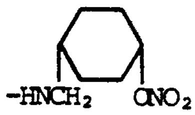 | $-OCH_3$ | $-CH(CH_3)_2$ | $-OCH_3$ | $-OCH_3$ |
| 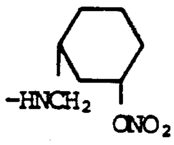 | $-OCH_3$ | $-CH(CH_3)_2$ | $-OCH_3$ | $-OCH_3$ |
| 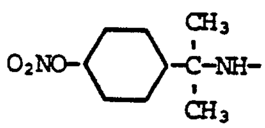<br>cis u. trans | $-OCH_3$ | $-CH(CH_3)_2$ | $-OCH_3$ | $-OCH_3$ |

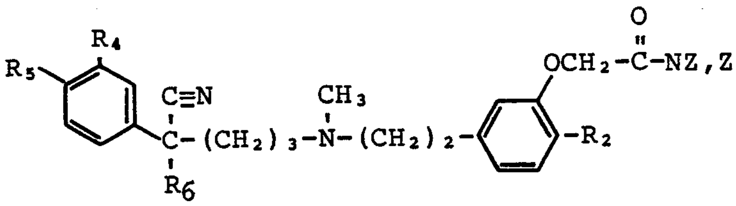

| -NZ,Z | $R_2$ | $R_6$ | $R_4$ | $R_5$ |
|---|---|---|---|---|
| 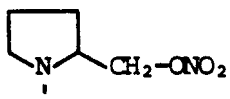 | $-OCH_3$ | $-CH(CH_3)_2$ | $-OCH_3$ | $-OCH_3$ |
| 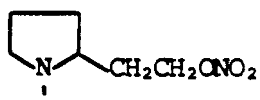 | $-OCH_3$ | $-CH(CH_3)_2$ | $-OCH_3$ | $-OCH_3$ |
| 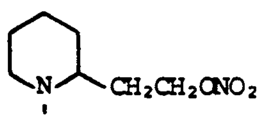 | $-OCH_3$ | $-CH(CH_3)_2$ | $-OCH_3$ | $-OCH_3$ |
| 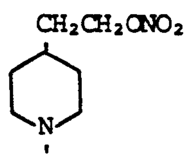 | $-OCH_3$ | $-CH(CH_3)_2$ | $-OCH_3$ | $-OCH_3$ |
| 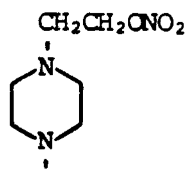 | $-OCH_3$ | $-CH(CH_3)_2$ | $-OCH_3$ | $-OCH_3$ |
| 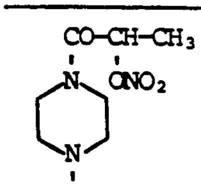 | $-OCH_3$ | $-CH(CH_3)_2$ | $-OCH_3$ | $-OCH_3$ |
| 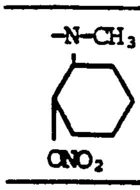 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 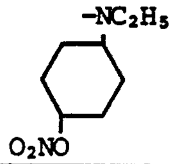 | $-OCH_3$ | $-CH(CH_3)_2$ | $-OCH_3$ | $-OCH_3$ |
| 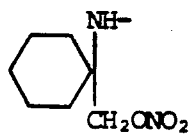 | $-OCH_3$ | $-CH(CH_3)_2$ | $-OCH_3$ | $-OCH_3$ |
| 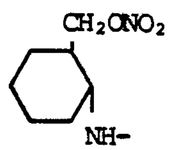 | $-OCH_3$ | $-CH(CH_3)_2$ | $-OCH_3$ | $-OCH_3$ |
| 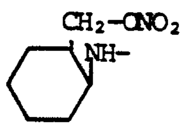 | $-OCH_3$ | $-CH(CH_3)_2$ | $-OCH_3$ | $-OCH_3$ |
| 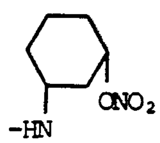 | $-OCH_3$ | $-CH(CH_3)_2$ | $-OCH_3$ | $-OCH_3$ |
| 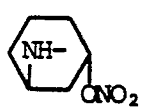 | $-OCH_3$ | $-CH(CH_3)_2$ | $-OCH_3$ | $-OCH_3$ |
| 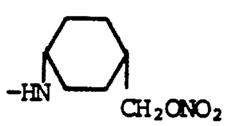 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 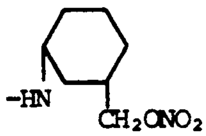 | $-OCH_3$ | $-CH(CH_3)_2$ | $-OCH_3$ | $-OCH_3$ |
| 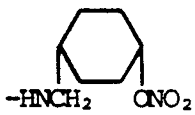 | $-OCH_3$ | $-CH(CH_3)_2$ | $-OCH_3$ | $-OCH_3$ |
| 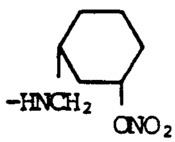 | $-OCH_3$ | $-CH(CH_3)_2$ | $-OCH_3$ | $-OCH_3$ |
| 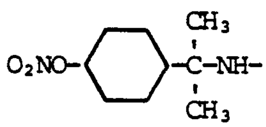 cis u. trans | $-OCH_3$ | $-CH(CH_3)_2$ | $-OCH_3$ | $-OCH_3$ |

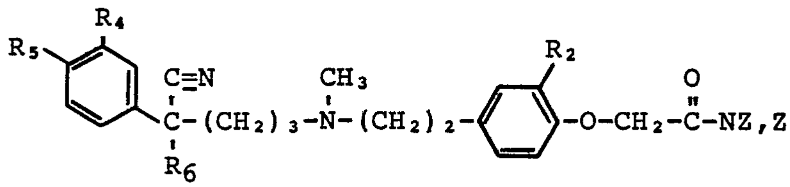

| -NZ,Z | $R_2$ | $R_6$ | $R_4$ | $R_5$ |
|---|---|---|---|---|
| 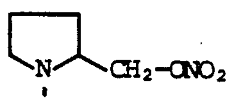 | $-OCH_3$ | $-CH(CH_3)_2$ | $-OCH_3$ | $-OCH_3$ |
| 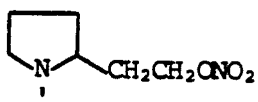 | $-OCH_3$ | $-CH(CH_3)_2$ | $-OCH_3$ | $-OCH_3$ |
| 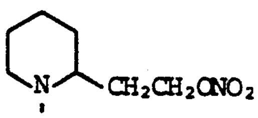 | $-OCH_3$ | $-CH(CH_3)_2$ | $-OCH_3$ | $-OCH_3$ |
| 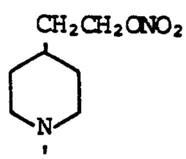 | $-OCH_3$ | $-CH(CH_3)_2$ | $-OCH_3$ | $-OCH_3$ |
| 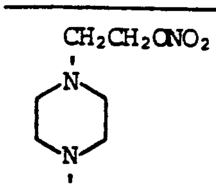 | $-OCH_3$ | $-CH(CH_3)_2$ | $-OCH_3$ | $-OCH_3$ |
| 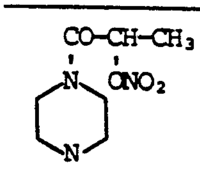 | $-OCH_3$ | $-CH(CH_3)_2$ | $-OCH_3$ | $-OCH_3$ |
| 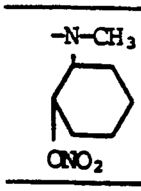 | $-OCH_3$ | $-CH(CH_3)_2$ | $-OCH_3$ | $-OCH_3$ |

| -NZ, Z | $R_2$ | $R_6$ | $R_4$ | $R_5$ |
|---|---|---|---|---|
| 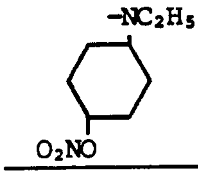 | $-OCH_3$ | $-CH(CH_3)_2$ | $-OCH_3$ | $-OCH_3$ |
| 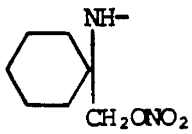 | $-OCH_3$ | $-CH(CH_3)_2$ | $-OCH_3$ | $-OCH_3$ |
| 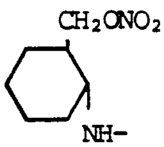 | $-OCH_3$ | $-CH(CH_3)_2$ | $-OCH_3$ | $-OCH_3$ |
| 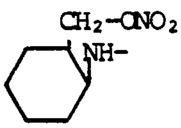 | $-OCH_3$ | $-CH(CH_3)_2$ | $-OCH_3$ | $-OCH_3$ |
| 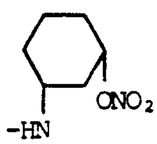 | $-OCH_3$ | $-CH(CH_3)_2$ | $-OCH_3$ | $-OCH_3$ |
| 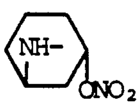 | $-OCH_3$ | $-CH(CH_3)_2$ | $-OCH_3$ | $-OCH_3$ |
| 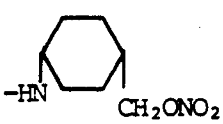 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 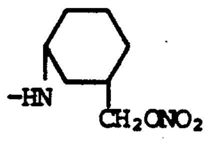 | $-OCH_3$ | $-CH(CH_3)_2$ | $-OCH_3$ | $-OCH_3$ |
| 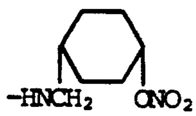 | $-OCH_3$ | $-CH(CH_3)_2$ | $-OCH_3$ | $-OCH_3$ |
| 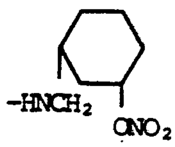 | $-OCH_3$ | $-CH(CH_3)_2$ | $-OCH_3$ | $-OCH_3$ |
| 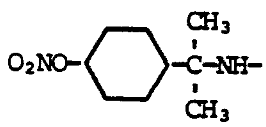 cis u. trans | $-OCH_3$ | $-CH(CH_3)_2$ | $-OCH_3$ | $-OCH_3$ |

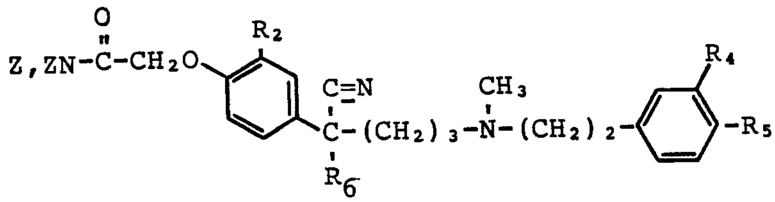

| $-NZ,Z$ | $R_2$ | $R_6$ | $R_4$ | $R_5$ |
|---|---|---|---|---|
| 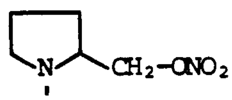 | $-OCH_3$ | $-CH(CH_3)_2$ | $-OCH_3$ | $-OCH_3$ |
| 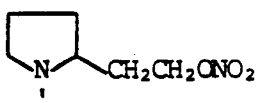 | $-OCH_3$ | $-CH(CH_3)_2$ | $-OCH_3$ | $-OCH_3$ |
| 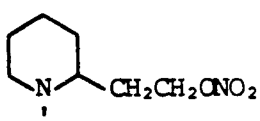 | $-OCH_3$ | $-CH(CH_3)_2$ | $-OCH_3$ | $-OCH_3$ |
| 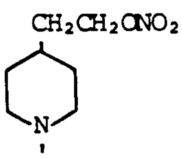 | $-OCH_3$ | $-CH(CH_3)_2$ | $-OCH_3$ | $-OCH_3$ |
| 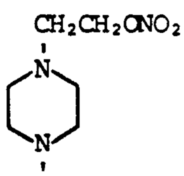 | $-OCH_3$ | $-CH(CH_3)_2$ | $-OCH_3$ | $-OCH_3$ |
| 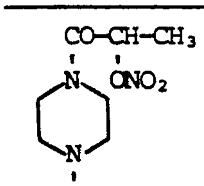 | $-OCH_3$ | $-CH(CH_3)_2$ | $-OCH_3$ | $-OCH_3$ |
| 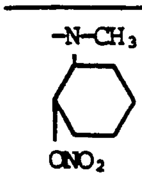 | $-OCH_3$ | $-CH(CH_3)_2$ | $-OCH_3$ | $-OCH_3$ |

| -NZ, Z | $R_2$ | $R_6$ | $R_4$ | $R_5$ |
|---|---|---|---|---|
| 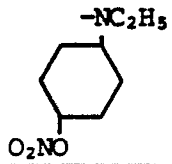 | $-OCH_3$ | $-CH(CH_3)_2$ | $-OCH_3$ | $-OCH_3$ |
| 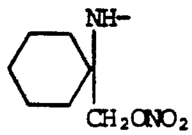 | $-OCH_3$ | $-CH(CH_3)_2$ | $-OCH_3$ | $-OCH_3$ |
| 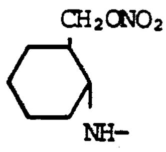 | $-OCH_3$ | $-CH(CH_3)_2$ | $-OCH_3$ | $-OCH_3$ |
| 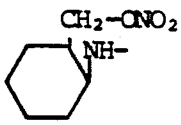 | $-OCH_3$ | $-CH(CH_3)_2$ | $-OCH_3$ | $-OCH_3$ |
| 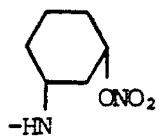 | $-OCH_3$ | $-CH(CH_3)_2$ | $-OCH_3$ | $-OCH_3$ |
| 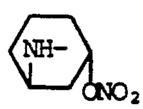 | $-OCH_3$ | $-CH(CH_3)_2$ | $-OCH_3$ | $-OCH_3$ |
| 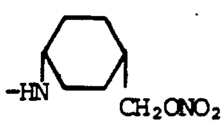 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 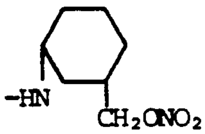 | $-OCH_3$ | $-CH(CH_3)_2$ | $-OCH_3$ | $-OCH_3$ |
| 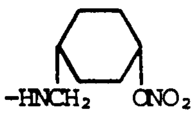 | $-OCH_3$ | $-CH(CH_3)_2$ | $-OCH_3$ | $-OCH_3$ |
| 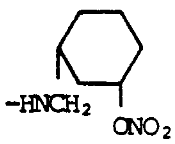 | $-OCH_3$ | $-CH(CH_3)_2$ | $-OCH_3$ | $-OCH_3$ |
| 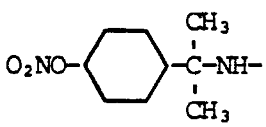 cis u. trans | $-OCH_3$ | $-CH(CH_3)_2$ | $-OCH_3$ | $-OCH_3$ |

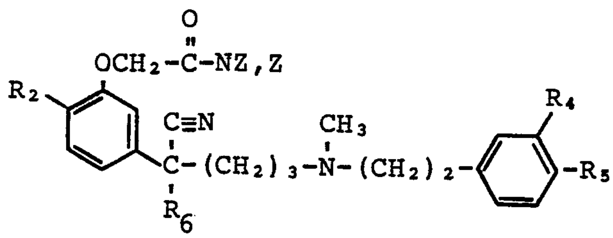

| -NZ,Z | $R_2$ | $R_6$ | $R_4$ | $R_5$ |
|---|---|---|---|---|
| 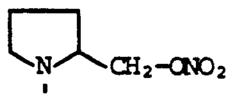 | $-OCH_3$ | $-CH(CH_3)_2$ | $-OCH_3$ | $-OCH_3$ |
| 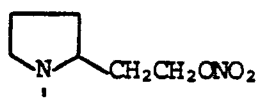 | $-OCH_3$ | $-CH(CH_3)_2$ | $-OCH_3$ | $-OCH_3$ |
| 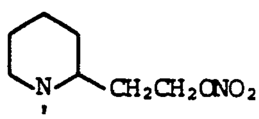 | $-OCH_3$ | $-CH(CH_3)_2$ | $-OCH_3$ | $-OCH_3$ |
| 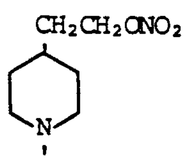 | $-OCH_3$ | $-CH(CH_3)_2$ | $-OCH_3$ | $-OCH_3$ |
| 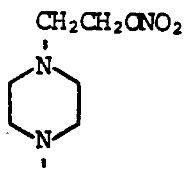 | $-OCH_3$ | $-CH(CH_3)_2$ | $-OCH_3$ | $-OCH_3$ |
| 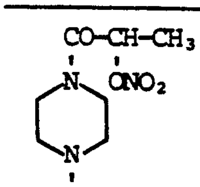 | $-OCH_3$ | $-CH(CH_3)_2$ | $-OCH_3$ | $-OCH_3$ |
| 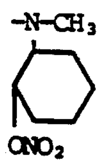 | $-OCH_3$ | $-CH(CH_3)_2$ | $-OCH_3$ | $-OCH_3$ |

0175249

| -NZ, Z | $R_2$ | $R_6$ | $R_4$ | $R_5$ |
|---|---|---|---|---|
| 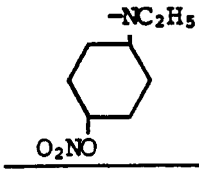 | $-OCH_3$ | $-CH(CH_3)_2$ | $-OCH_3$ | $-OCH_3$ |
| 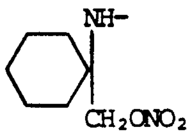 | $-OCH_3$ | $-CH(CH_3)_2$ | $-OCH_3$ | $-OCH_3$ |
| 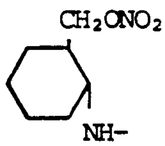 | $-OCH_3$ | $-CH(CH_3)_2$ | $-OCH_3$ | $-OCH_3$ |
| 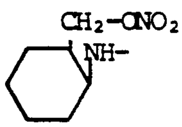 | $-OCH_3$ | $-CH(CH_3)_2$ | $-OCH_3$ | $-OCH_3$ |
| 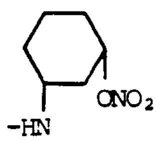 | $-OCH_3$ | $-CH(CH_3)_2$ | $-OCH_3$ | $-OCH_3$ |
| 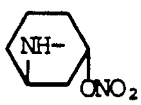 | $-OCH_3$ | $-CH(CH_3)_2$ | $-OCH_3$ | $-OCH_3$ |
| 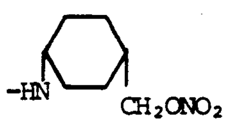 | $-OCH_3$ | $-CH(CH_3)_2$ | $-OCH_3$ | $-OCH_3$ |

| -NZ,Z | $R_2$ | $R_6$ | $R_4$ | $R_5$ |
|---|---|---|---|---|
| 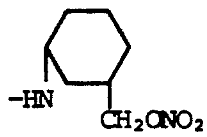 | $-OCH_3$ | $-CH(CH_3)_2$ | $-OCH_3$ | $-OCH_3$ |
| 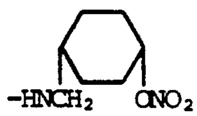 | $-OCH_3$ | $-CH(CH_3)_2$ | $-OCH_3$ | $-OCH_3$ |
| 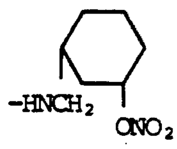 | $-OCH_3$ | $-CH(CH_3)_2$ | $-OCH_3$ | $-OCH_3$ |
| 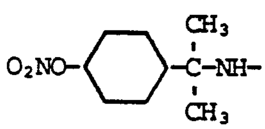 cis u. trans | $-OCH_3$ | $-CH(CH_3)_2$ | $-OCH_3$ | $-OCH_3$ |

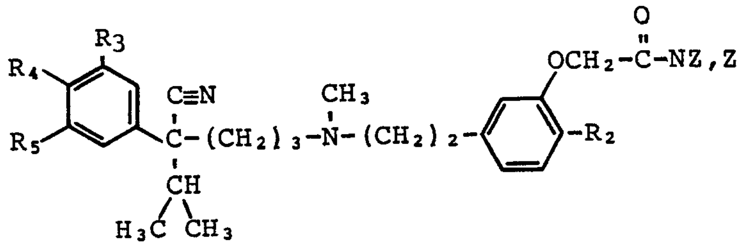

| -NZ,Z | $R_2$ | $R_3$ | $R_4$ | $R_5$ |
|---|---|---|---|---|
| 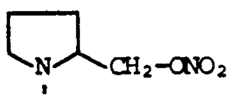 | $-OCH_3$ | $-CH(CH_3)_2$ | $-OCH_3$ | $-OCH_3$ |
| 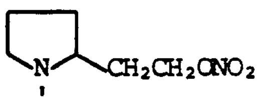 | $-OCH_3$ | $-CH(CH_3)_2$ | $-OCH_3$ | $-OCH_3$ |
| 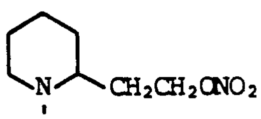 | $-OCH_3$ | $-CH(CH_3)_2$ | $-OCH_3$ | $-OCH_3$ |
| 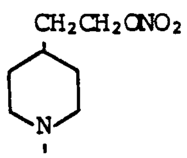 | $-OCH_3$ | $-CH(CH_3)_2$ | $-OCH_3$ | $-OCH_3$ |
| 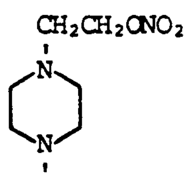 | $-OCH_3$ | $-CH(CH_3)_2$ | $-OCH_3$ | $-OCH_3$ |
| 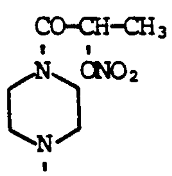 | $-OCH_3$ | $-CH(CH_3)_2$ | $-OCH_3$ | $-OCH_3$ |
| 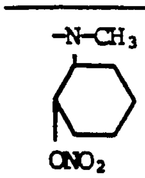 | $-OCH_3$ | $-CH(CH_3)_2$ | $-OCH_3$ | $-OCH_3$ |

| -NZ,Z | $R_2$ | $R_3$ | $R_4$ | $R_5$ |
|---|---|---|---|---|
| 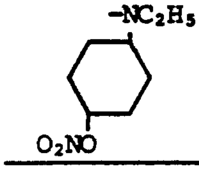 | $-OCH_3$ | $-CH(CH_3)_2$ | $-OCH_3$ | $-OCH_3$ |
| 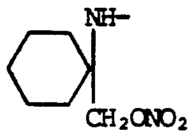 | $-OCH_3$ | $-CH(CH_3)_2$ | $-OCH_3$ | $-OCH_3$ |
| 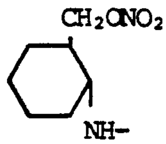 | $-OCH_3$ | $-CH(CH_3)_2$ | $-OCH_3$ | $-OCH_3$ |
| 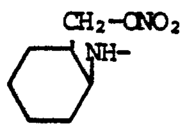 | $-OCH_3$ | $-CH(CH_3)_2$ | $-OCH_3$ | $-OCH_3$ |
| 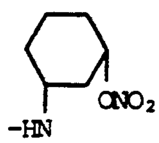 | $-OCH_3$ | $-CH(CH_3)_2$ | $-OCH_3$ | $-OCH_3$ |
| 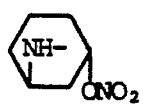 | $-OCH_3$ | $-CH(CH_3)_2$ | $-OCH_3$ | $-OCH_3$ |
| 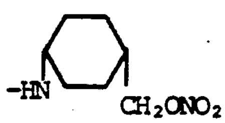 | $-OCH_3$ | $-CH(CH_3)_2$ | $-OCH_3$ | $-OCH_3$ |

| -NZ,Z | $R_2$ | $R_3$ | $R_4$ | $R_5$ |
|---|---|---|---|---|
| 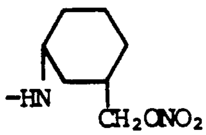 | $-OCH_3$ | $-CH(CH_3)_2$ | $-OCH_3$ | $-OCH_3$ |
| 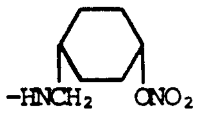 | $-OCH_3$ | $-CH(CH_3)_2$ | $-OCH_3$ | $-OCH_3$ |
| 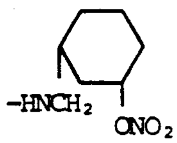 | $-OCH_3$ | $-CH(CH_3)_2$ | $-OCH_3$ | $-OCH_3$ |
| 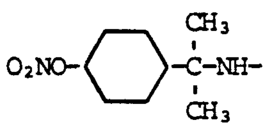 cis u. trans | $-OCH_3$ | $-CH(CH_3)_2$ | $-OCH_3$ | $-OCH_3$ |

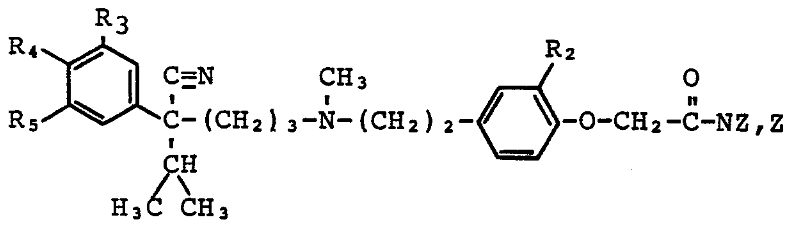

| -NZ,Z | $R_2$ | $R_3$ | $R_4$ | $R_5$ |
|---|---|---|---|---|
| 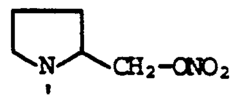 | $-OCH_3$ | $-CH(CH_3)_2$ | $-OCH_3$ | $-OCH_3$ |
| 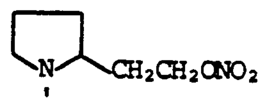 | $-OCH_3$ | $-CH(CH_3)_2$ | $-OCH_3$ | $-OCH_3$ |
| 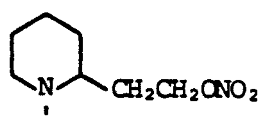 | $-OCH_3$ | $-CH(CH_3)_2$ | $-OCH_3$ | $-OCH_3$ |
| 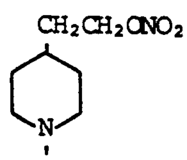 | $-OCH_3$ | $-CH(CH_3)_2$ | $-OCH_3$ | $-OCH_3$ |
| 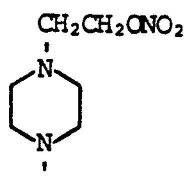 | $-OCH_3$ | $-CH(CH_3)_2$ | $-OCH_3$ | $-OCH_3$ |
| 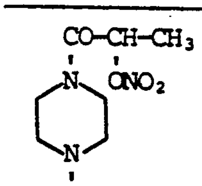 | $-OCH_3$ | $-CH(CH_3)_2$ | $-OCH_3$ | $-OCH_3$ |
| 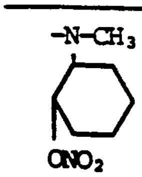 | $-OCH_3$ | $-CH(CH_3)_2$ | $-OCH_3$ | $-OCH_3$ |

| -NZ, Z | $R_2$ | $R_3$ | $R_4$ | $R_5$ |
|---|---|---|---|---|
| 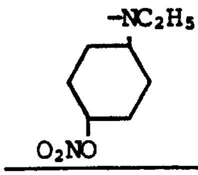 | $-OCH_3$ | $-CH(CH_3)_2$ | $-OCH_3$ | $-OCH_3$ |
| 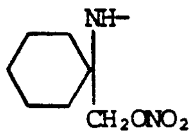 | $-OCH_3$ | $-CH(CH_3)_2$ | $-OCH_3$ | $-OCH_3$ |
| 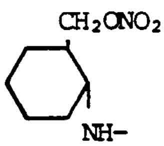 | $-OCH_3$ | $-CH(CH_3)_2$ | $-OCH_3$ | $-OCH_3$ |
| 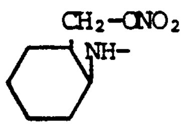 | $-OCH_3$ | $-CH(CH_3)_2$ | $-OCH_3$ | $-OCH_3$ |
| 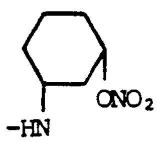 | $-OCH_3$ | $-CH(CH_3)_2$ | $-OCH_3$ | $-OCH_3$ |
| 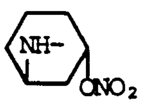 | $-OCH_3$ | $-CH(CH_3)_2$ | $-OCH_3$ | $-OCH_3$ |
| 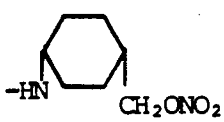 | $-OCH_3$ | $-CH(CH_3)_2$ | $-OCH_3$ | $-OCH_3$ |

| -NZ,Z | $R_2$ | $R_3$ | $R_4$ | $R_5$ |
|---|---|---|---|---|
| 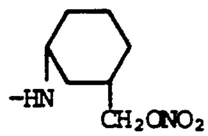 | $-OCH_3$ | $-CH(CH_3)_2$ | $-OCH_3$ | $-OCH_3$ |
| 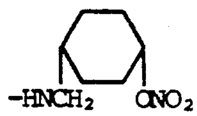 | $-OCH_3$ | $-CH(CH_3)_2$ | $-OCH_3$ | $-OCH_3$ |
| 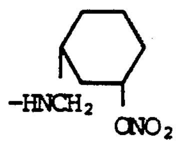 | $-OCH_3$ | $-CH(CH_3)_2$ | $-OCH_3$ | $-OCH_3$ |
| 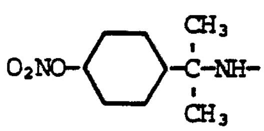 cis u. trans | $-OCH_3$ | $-CH(CH_3)_2$ | $-OCH_3$ | $-OCH_3$ |

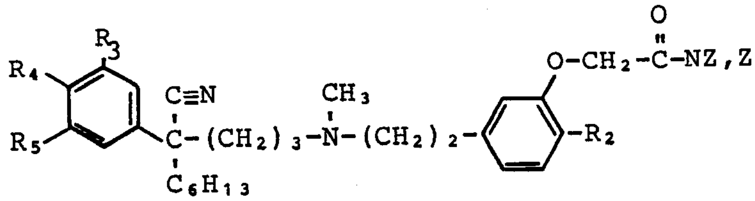

| $-NZ,Z$ | $R_2$ | $R_3$ | $R_4$ | $R_5$ |
|---|---|---|---|---|
| 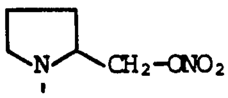 | $-OCH_3$ | $-CH(CH_3)_2$ | $-OCH_3$ | $-OCH_3$ |
| 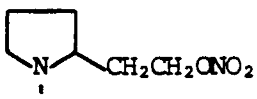 | $-OCH_3$ | $-CH(CH_3)_2$ | $-OCH_3$ | $-OCH_3$ |
| 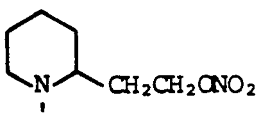 | $-OCH_3$ | $-CH(CH_3)_2$ | $-OCH_3$ | $-OCH_3$ |
| 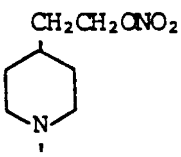 | $-OCH_3$ | $-CH(CH_3)_2$ | $-OCH_3$ | $-OCH_3$ |
| 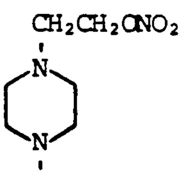 | $-OCH_3$ | $-CH(CH_3)_2$ | $-OCH_3$ | $-OCH_3$ |
| 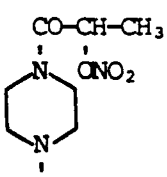 | $-OCH_3$ | $-CH(CH_3)_2$ | $-OCH_3$ | $-OCH_3$ |
| 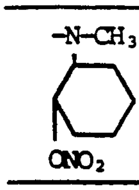 | $-OCH_3$ | $-CH(CH_3)_2$ | $-OCH_3$ | $-OCH_3$ |

| -NZ, Z | $R_2$ | $R_3$ | $R_4$ | $R_5$ |
|---|---|---|---|---|
| 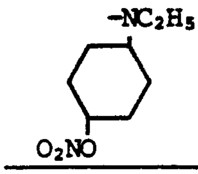 | $-OCH_3$ | $-CH(CH_3)_2$ | $-OCH_3$ | $-OCH_3$ |
| 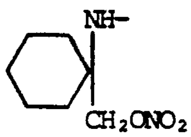 | $-OCH_3$ | $-CH(CH_3)_2$ | $-OCH_3$ | $-OCH_3$ |
| 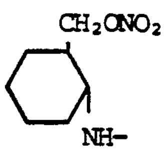 | $-OCH_3$ | $-CH(CH_3)_2$ | $-OCH_3$ | $-OCH_3$ |
| 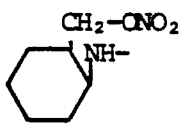 | $-OCH_3$ | $-CH(CH_3)_2$ | $-OCH_3$ | $-OCH_3$ |
| 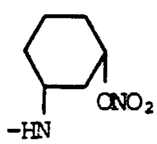 | $-OCH_3$ | $-CH(CH_3)_2$ | $-OCH_3$ | $-OCH_3$ |
| 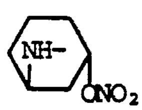 | $-OCH_3$ | $-CH(CH_3)_2$ | $-OCH_3$ | $-OCH_3$ |
| 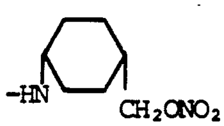 | $-OCH_3$ | $-CH(CH_3)_2$ | $-OCH_3$ | $-OCH_3$ |

| -NZ,Z | $R_2$ | $R_3$ | $R_4$ | $R_5$ |
| --- | --- | --- | --- | --- |
| 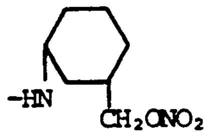 | $-OCH_3$ | $-CH(CH_3)_2$ | $-OCH_3$ | $-OCH_3$ |
| 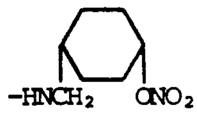 | $-OCH_3$ | $-CH(CH_3)_2$ | $-OCH_3$ | $-OCH_3$ |
| 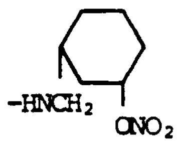 | $-OCH_3$ | $-CH(CH_3)_2$ | $-OCH_3$ | $-OCH_3$ |
| 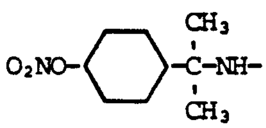 cis u. trans | $-OCH_3$ | $-CH(CH_3)_2$ | $-OCH_3$ | $-OCH_3$ |

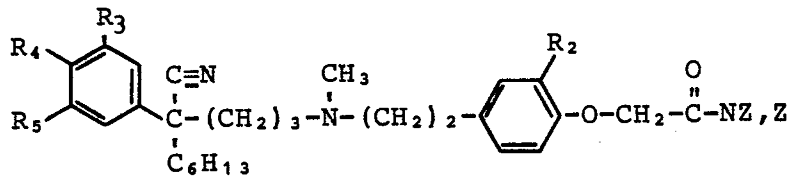

| -NZ,Z | $R_2$ | $R_3$ | $R_4$ | $R_5$ |
|---|---|---|---|---|
| 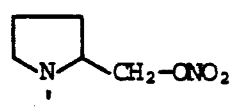 | $-OCH_3$ | $-CH(CH_3)_2$ | $-OCH_3$ | $-OCH_3$ |
| 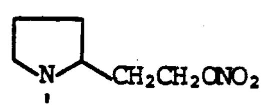 | $-OCH_3$ | $-CH(CH_3)_2$ | $-OCH_3$ | $-OCH_3$ |
| 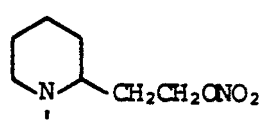 | $-OCH_3$ | $-CH(CH_3)_2$ | $-OCH_3$ | $-OCH_3$ |
| 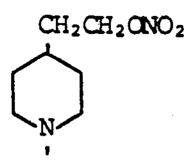 | $-OCH_3$ | $-CH(CH_3)_2$ | $-OCH_3$ | $-OCH_3$ |
| 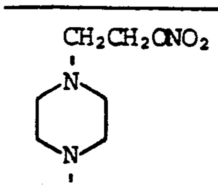 | $-OCH_3$ | $-CH(CH_3)_2$ | $-OCH_3$ | $-OCH_3$ |
| 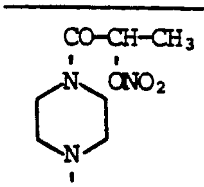 | $-OCH_3$ | $-CH(CH_3)_2$ | $-OCH_3$ | $-OCH_3$ |
| 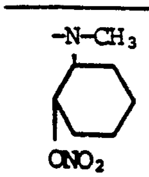 | $-OCH_3$ | $-CH(CH_3)_2$ | $-OCH_3$ | $-OCH_3$ |

| -NZ, Z | $R_2$ | $R_3$ | $R_4$ | $R_5$ |
|---|---|---|---|---|
| 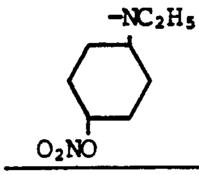 | $-OCH_3$ | $-CH(CH_3)_2$ | $-OCH_3$ | $-OCH_3$ |
| 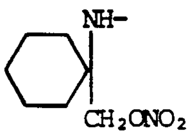 | $-OCH_3$ | $-CH(CH_3)_2$ | $-OCH_3$ | $-OCH_3$ |
| 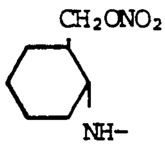 | $-OCH_3$ | $-CH(CH_3)_2$ | $-OCH_3$ | $-OCH_3$ |
| 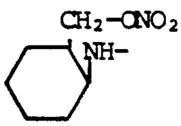 | $-OCH_3$ | $-CH(CH_3)_2$ | $-OCH_3$ | $-OCH_3$ |
| 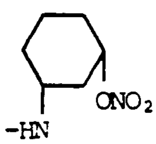 | $-OCH_3$ | $-CH(CH_3)_2$ | $-OCH_3$ | $-OCH_3$ |
| 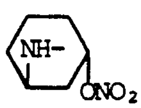 | $-OCH_3$ | $-CH(CH_3)_2$ | $-OCH_3$ | $-OCH_3$ |
| 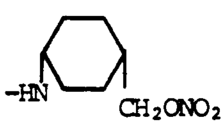 | $-OCH_3$ | $-CH(CH_3)_2$ | $-OCH_3$ | $-OCH_3$ |

| -NZ,Z | $R_2$ | $R_3$ | $R_4$ | $R_5$ |
|---|---|---|---|---|
| 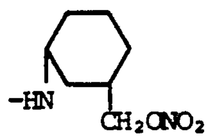 | $-OCH_3$ | $-CH(CH_3)_2$ | $-OCH_3$ | $-OCH_3$ |
| 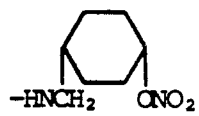 | $-OCH_3$ | $-CH(CH_3)_2$ | $-OCH_3$ | $-OCH_3$ |
| 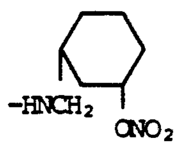 | $-OCH_3$ | $-CH(CH_3)_2$ | $-OCH_3$ | $-OCH_3$ |
| 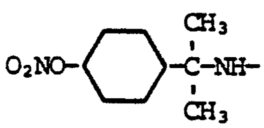 cis u. trans | $-OCH_3$ | $-CH(CH_3)_2$ | $-OCH_3$ | $-OCH_3$ |

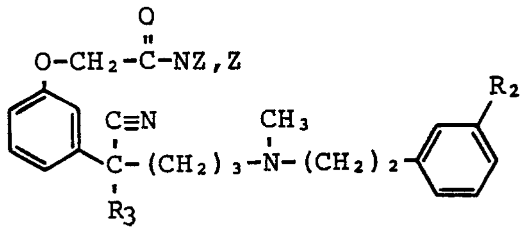

| -NZ,Z | $R_2$ | $R_3$ | $R_4$ | $R_5$ |
|---|---|---|---|---|
| 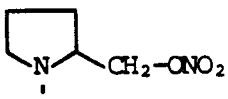 | $-OCH_3$ | $-CH(CH_3)_2$ | $-OCH_3$ | $-OCH_3$ |
| 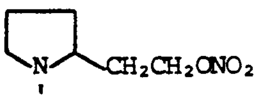 | $-OCH_3$ | $-CH(CH_3)_2$ | $-OCH_3$ | $-OCH_3$ |
| 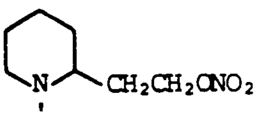 | $-OCH_3$ | $-CH(CH_3)_2$ | $-OCH_3$ | $-OCH_3$ |
| 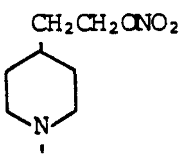 | $-OCH_3$ | $-CH(CH_3)_2$ | $-OCH_3$ | $-OCH_3$ |
| 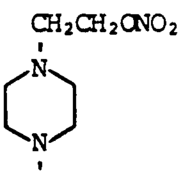 | $-OCH_3$ | $-CH(CH_3)_2$ | $-OCH_3$ | $-OCH_3$ |
| 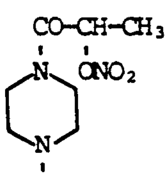 | $-OCH_3$ | $-CH(CH_3)_2$ | $-OCH_3$ | $-OCH_3$ |
| 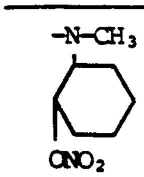 | $-OCH_3$ | $-CH(CH_3)_2$ | $-OCH_3$ | $-OCH_3$ |

| -NZ, Z | $R_2$ | $R_3$ | $R_4$ | $R_5$ |
|---|---|---|---|---|
| 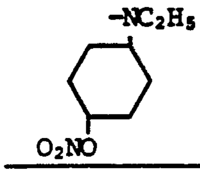 | $-OCH_3$ | $-CH(CH_3)_2$ | $-OCH_3$ | $-OCH_3$ |
| 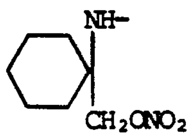 | $-OCH_3$ | $-CH(CH_3)_2$ | $-OCH_3$ | $-OCH_3$ |
| 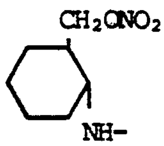 | $-OCH_3$ | $-CH(CH_3)_2$ | $-OCH_3$ | $-OCH_3$ |
| 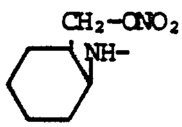 | $-OCH_3$ | $-CH(CH_3)_2$ | $-OCH_3$ | $-OCH_3$ |
| 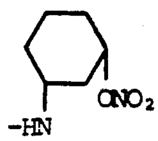 | $-OCH_3$ | $-CH(CH_3)_2$ | $-OCH_3$ | $-OCH_3$ |
| 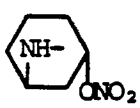 | $-OCH_3$ | $-CH(CH_3)_2$ | $-OCH_3$ | $-OCH_3$ |
| 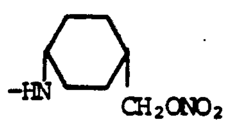 | $-OCH_3$ | $-CH(CH_3)_2$ | $-OCH_3$ | $-OCH_3$ |

| -NZ,Z | $R_2$ | $R_3$ | $R_4$ | $R_5$ |
|---|---|---|---|---|
| 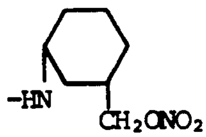 | $-OCH_3$ | $-CH(CH_3)_2$ | $-OCH_3$ | $-OCH_3$ |
| 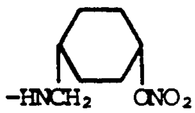 | $-OCH_3$ | $-CH(CH_3)_2$ | $-OCH_3$ | $-OCH_3$ |
| 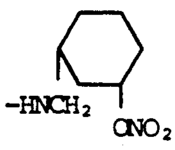 | $-OCH_3$ | $-CH(CH_3)_2$ | $-OCH_3$ | $-OCH_3$ |
| 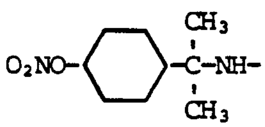 cis u. trans | $-OCH_3$ | $-CH(CH_3)_2$ | $-OCH_3$ | $-OCH_3$ |

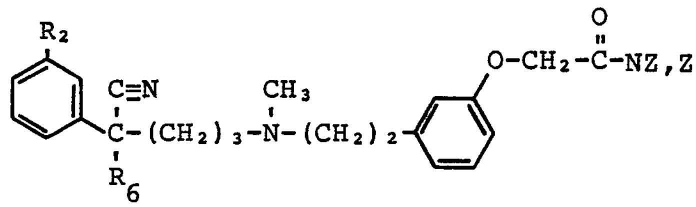

| -NZ,Z | $R_2$ | $R_6$ | $R_4$ | $R_5$ |
|---|---|---|---|---|
| 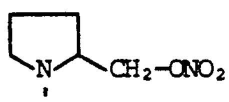 | $-OCH_3$ | $-CH(CH_3)_2$ | $-OCH_3$ | $-OCH_3$ |
| 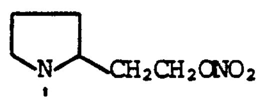 | $-OCH_3$ | $-CH(CH_3)_2$ | $-OCH_3$ | $-OCH_3$ |
| 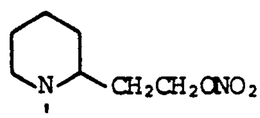 | $-OCH_3$ | $-CH(CH_3)_2$ | $-OCH_3$ | $-OCH_3$ |
| 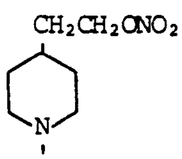 | $-OCH_3$ | $-CH(CH_3)_2$ | $-OCH_3$ | $-OCH_3$ |
| 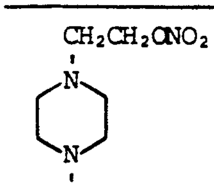 | $-OCH_3$ | $-CH(CH_3)_2$ | $-OCH_3$ | $-OCH_3$ |
| 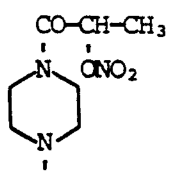 | $-OCH_3$ | $-CH(CH_3)_2$ | $-OCH_3$ | $-OCH_3$ |
| 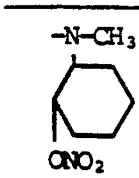 | $-OCH_3$ | $-CH(CH_3)_2$ | $-OCH_3$ | $-OCH_3$ |

| -NZ, Z | $R_2$ | $R_6$ | $R_4$ | $R_5$ |
|---|---|---|---|---|
| 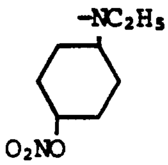 | $-OCH_3$ | $-CH(CH_3)_2$ | $-OCH_3$ | $-OCH_3$ |
| 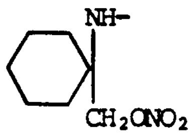 | $-OCH_3$ | $-CH(CH_3)_2$ | $-OCH_3$ | $-OCH_3$ |
| 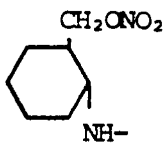 | $-OCH_3$ | $-CH(CH_3)_2$ | $-OCH_3$ | $-OCH_3$ |
| 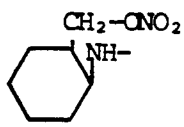 | $-OCH_3$ | $-CH(CH_3)_2$ | $-OCH_3$ | $-OCH_3$ |
| 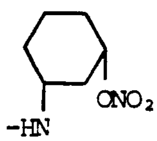 | $-OCH_3$ | $-CH(CH_3)_2$ | $-OCH_3$ | $-OCH_3$ |
| 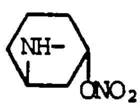 | $-OCH_3$ | $-CH(CH_3)_2$ | $-OCH_3$ | $-OCH_3$ |
| 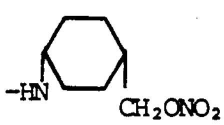 | $-OCH_3$ | $-CH(CH_3)_2$ | $-OCH_3$ | $-OCH_3$ |

| -NZ, Z | $R_2$ | $R_6$ | $R_4$ | $R_5$ |
|---|---|---|---|---|
| 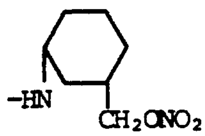 | $-OCH_3$ | $-CH(CH_3)_2$ | $-OCH_3$ | $-OCH_3$ |
| 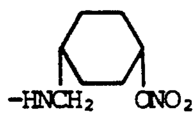 | $-OCH_3$ | $-CH(CH_3)_2$ | $-OCH_3$ | $-OCH_3$ |
| 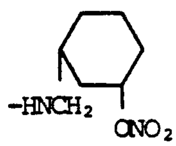 | $-OCH_3$ | $-CH(CH_3)_2$ | $-OCH_3$ | $-OCH_3$ |
| 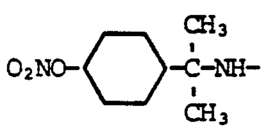 cis u. trans | $-OCH_3$ | $-CH(CH_3)_2$ | $-OCH_3$ | $-OCH_3$ |

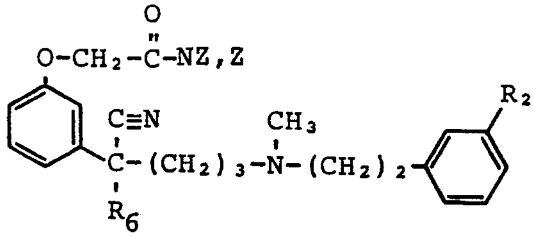

| -NZ,Z | $R_2$ | $R_6$ |
|---|---|---|
| 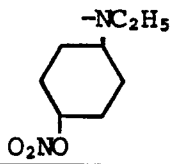 | $-OCH_3$ | $-CH(CH_3)_2$ |
| 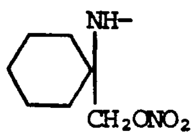 | $-OCH_3$ | $-CH(CH_3)_2$ |
| 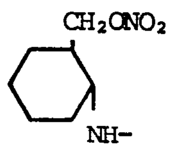 | $-OCH_3$ | $-CH(CH_3)_2$ |
| 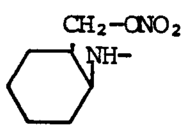 | $-OCH_3$ | $-CH(CH_3)_2$ |
| 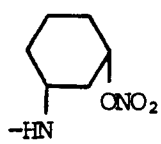 | $-OCH_3$ | $-CH(CH_3)_2$ |
| 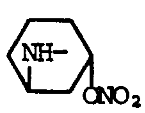 | $-OCH_3$ | $-CH(CH_3)_2$ |
| 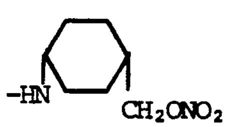 | $-OCH_3$ | $-CH(CH_3)_2$ |

| -NZ,Z | $R_2$ | $R_6$ |
|---|---|---|
| 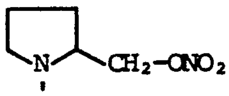 | $-OCH_3$ | $-CH(CH_3)_2$ |
| 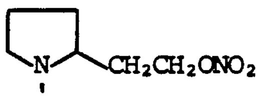 | $-OCH_3$ | $-CH(CH_3)_2$ |
| 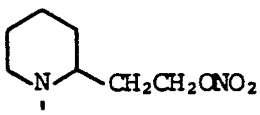 | $-OCH_3$ | $-CH(CH_3)_2$ |
| 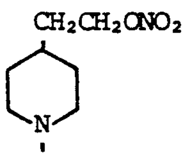 | $-OCH_3$ | $-CH(CH_3)_2$ |
| 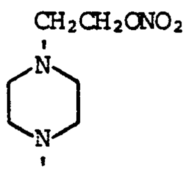 | $-OCH_3$ | $-CH(CH_3)_2$ |
| 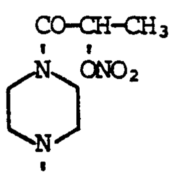 | $-OCH_3$ | $-CH(CH_3)_2$ |
| 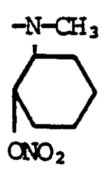 | $-OCH_3$ | $-CH(CH_3)_2$ |

| -NZ,Z | $R_2$ | $R_6$ |
| --- | --- | --- |
| 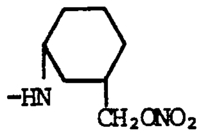 | $-OCH_3$ | $-CH(CH_3)_2$ |
| 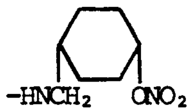 | $-OCH_3$ | $-CH(CH_3)_2$ |
| 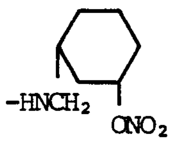 | $-OCH_3$ | $-CH(CH_3)_2$ |
| 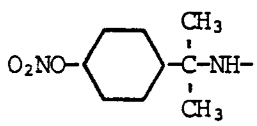<br>cis u. trans | $-OCH_3$ | $-CH(CH_3)_2$ |

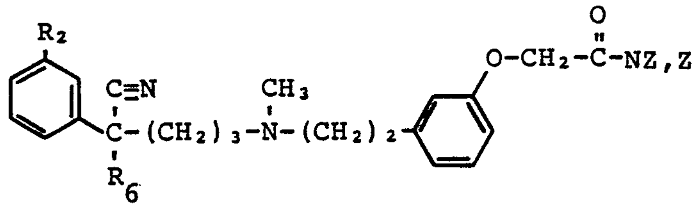

| -NZ,Z | $R_2$ | $R_6$ |
| --- | --- | --- |
| 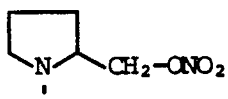 | $-OCH_3$ | $-CH(CH_3)_2$ |
| 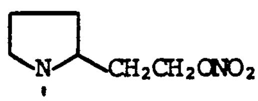 | $-OCH_3$ | $-CH(CH_3)_2$ |
| 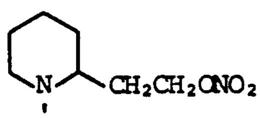 | $-OCH_3$ | $-CH(CH_3)_2$ |
| 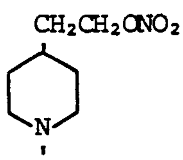 | $-OCH_3$ | $-CH(CH_3)_2$ |
| 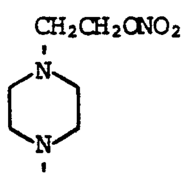 | $-OCH_3$ | $-CH(CH_3)_2$ |
| 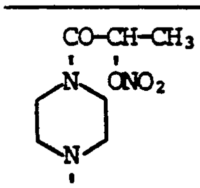 | $-OCH_3$ | $-CH(CH_3)_2$ |
| 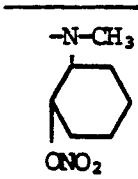 | $-OCH_3$ | $-CH(CH_3)_2$ |

| -NZ, Z | $R_2$ | $R_6$ |
|---|---|---|
| 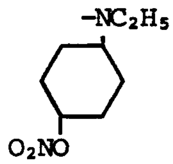 | $-OCH_3$ | $-CH(CH_3)_2$ |
| 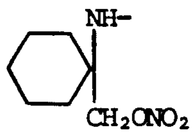 | $-OCH_3$ | $-CH(CH_3)_2$ |
| 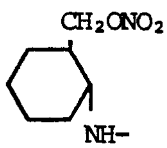 | $-OCH_3$ | $-CH(CH_3)_2$ |
| 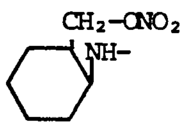 | $-OCH_3$ | $-CH(CH_3)_2$ |
| 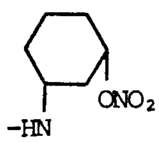 | $-OCH_3$ | $-CH(CH_3)_2$ |
| 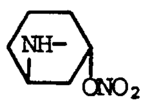 | $-OCH_3$ | $-CH(CH_3)_2$ |
| 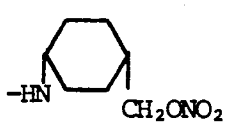 | $-OCH_3$ | $-CH(CH_3)_2$ |

| -NZ,Z | $R_2$ | $R_6$ |
|---|---|---|
| 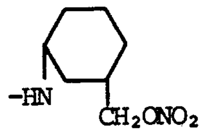 | $-OCH_3$ | $-CH(CH_3)_2$ |
| 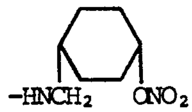 | $-OCH_3$ | $-CH(CH_3)_2$ |
| 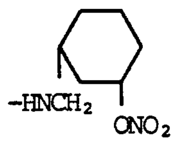 | $-OCH_3$ | $-CH(CH_3)_2$ |
| 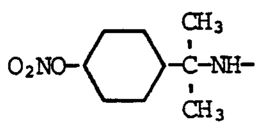 cis u. trans | $-OCH_3$ | $-CH(CH_3)_2$ |

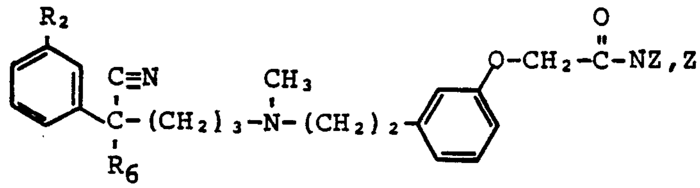

| -NZ,Z | $R_2$ | $R_6$ |
|---|---|---|
| 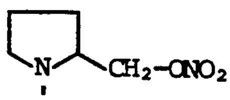 | $-OCH_3$ | $-C_6H_{13}$ |
| 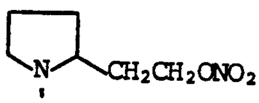 | $-OCH_3$ | " |
| 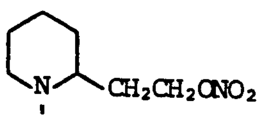 | $-OCH_3$ | " |
| 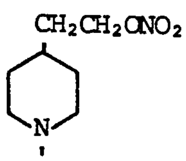 | $-OCH_3$ | " |
| 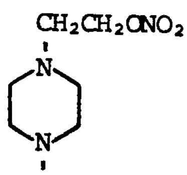 | $-OCH_3$ | " |
| 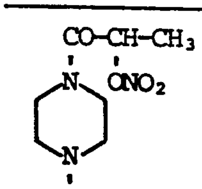 | $-OCH_3$ | " |
| 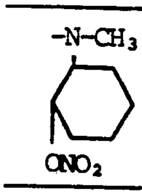 | $-OCH_3$ | " |

| -NZ,Z | $R_2$ | $R_6$ |
|---|---|---|
| 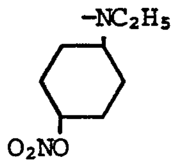 | $-OCH_3$ | $-C_6H_{13}$ |
| 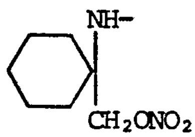 | $-OCH_3$ | " |
| 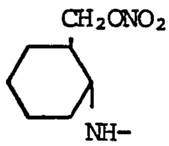 | $-OCH_3$ | " |
| 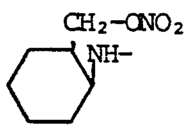 | $-OCH_3$ | " |
| 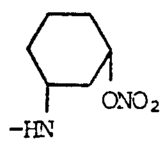 | $-OCH_3$ | " |
| 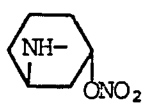 | $-OCH_3$ | " |
| 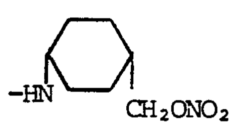 | $-OCH_3$ | " |

| -NZ,Z | $R_2$ | $R_6$ |
|---|---|---|
| 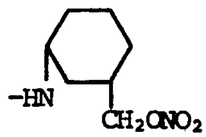 | $-OCH_3$ | $-C_6H_{13}$ |
| 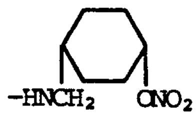 | $-OCH_3$ | " |
| 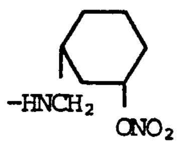 | $-OCH_3$ | " |
| 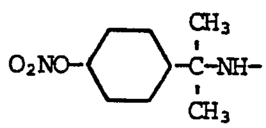 cis u. trans | $-OCH_3$ | " |

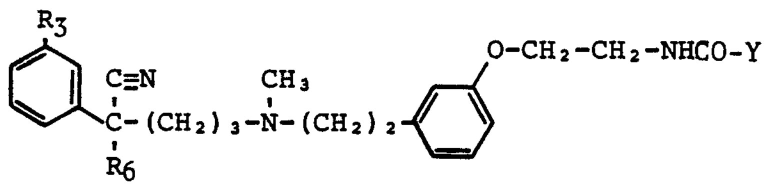

| Y | $R_3$ | $R_6$ |
|---|---|---|
| $-CH_2-CH(CH_3)-ONO_2$ | $-OCH_3$ | $-C_6H_{13}$ |
| $-CH(CH_3)-(CH_2)_2-ONO_2$ | $-OCH_3$ | " |
| $-(CH_2)_4-ONO_2$ | $-OCH_3$ | " |
| $-(CH_2)_3-CH(CH_3)-ONO_2$ | $-OCH_3$ | " |
| $-CH_2-O-CH_2-CH_2-ONO_2$ | $-OCH_3$ | " |
| $-CH_2-S-CH_2-CH_2-ONO_2$ | $-OCH_3$ | " |
| $-CH(CH_3)-S-CH_2-CH_2-ONO_2$ | $-OCH_3$ | " |
| 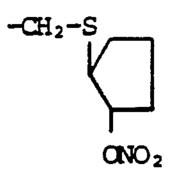 | $-OCH_3$ | " |
| 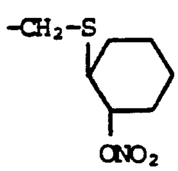 | $-OCH_3$ | " |

| Y | $R_3$ | $R_6$ |
|---|---|---|
| 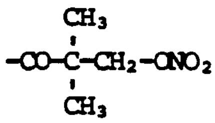 | $-OCH_3$ | $-C_6H_{13}$ |
| 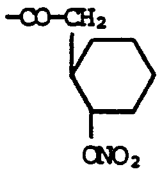 | $-OCH_3$ | " |
| 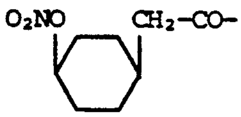 | $-OCH_3$ | " |
| 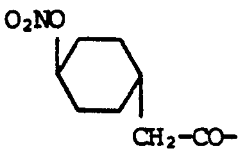 | $-OCH_3$ | " |
| 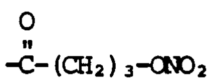 | $-OCH_3$ | " |

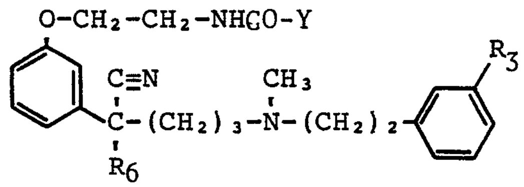

| Y | $R_3$ | $R_6$ |
|---|---|---|
| $-CH_2-CH(CH_3)-ONO_2$ | $-OCH_3$ | $-C_6H_{13}$ |
| $-CH(CH_3)-(CH_2)_2-ONO_2$ | $-OCH_3$ | " |
| $-(CH_2)_4-ONO_2$ | $-OCH_3$ | " |
| $-(CH_2)_3-CH(CH_3)-ONO_2$ | $-OCH_3$ | " |
| $-CH_2-O-CH_2-CH_2-ONO_2$ | $-OCH_3$ | " |
| $-CH_2-S-CH_2-CH_2-ONO_2$ | $-OCH_3$ | " |
| $-CH(CH_3)-S-CH_2-CH_2-ONO_2$ | $-OCH_3$ | " |
| 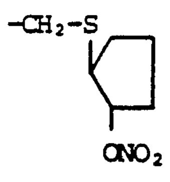 | $-OCH_3$ | " |
| 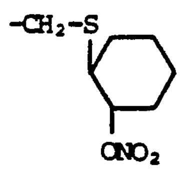 | $-OCH_3$ | " |

| Y | $R_5$ | $R_6$ |
|---|---|---|
| 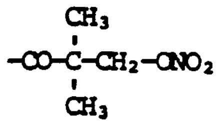 | $-OCH_3$ | $-C_6H_{13}$ |
| 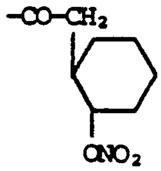 | $-OCH_3$ | " |
| 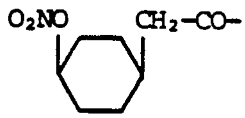 | $-OCH_3$ | " |
| 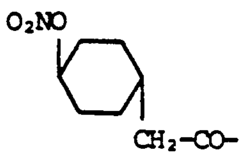 | $-OCH_3$ | " |
| 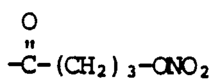 | $-OCH_3$ | " |

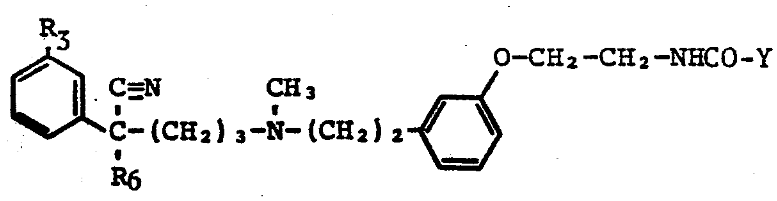

| Y | $R_3$ | $R_6$ |
| --- | --- | --- |
| $-CH_2-CH(CH_3)-ONO_2$ | $-OCH_3$ | $-C_6H_{13}$ |
| $-CH(CH_3)-(CH_2)_2-ONO_2$ | $-OCH_3$ | " |
| $-(CH_2)_4-ONO_2$ | $-OCH_3$ | " |
| $-(CH_2)_3-CH(CH_3)-ONO_2$ | $-OCH_3$ | " |
| $-CH_2-O-CH_2-CH_2-ONO_2$ | $-OCH_3$ | " |
| $-CH_2-S-CH_2-CH_2-ONO_2$ | $-OCH_3$ | " |
| $-CH(CH_3)-S-CH_2-CH_2-ONO_2$ | $-OCH_3$ | " |
| 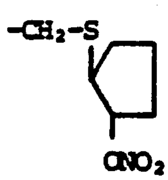 | $-OCH_3$ | " |
| 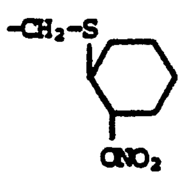 | $-OCH_3$ | " |

| Y | $R_5$ | $R_6$ |
|---|---|---|
| 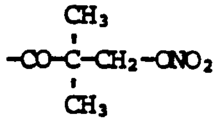 | $-OCH_3$ | $-C_6H_{13}$ |
| 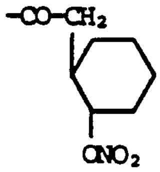 | $-OCH_3$ | " |
| 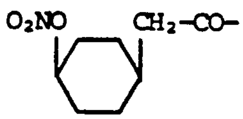 | $-OCH_3$ | " |
| 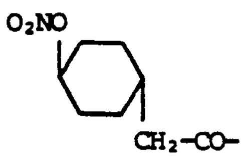 | $-OCH_3$ | " |
| 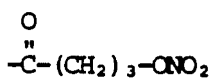 | $-OCH_3$ | " |

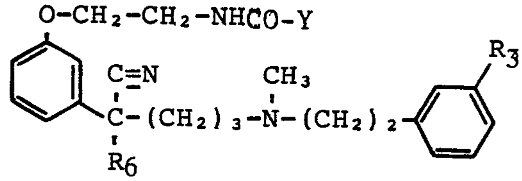

| Y | $R_3$ | $R_6$ |
|---|---|---|
| $-CH_2-CH(CH_3)-ONO_2$ | $-OCH_3$ | $-C_6H_{13}$ |
| $-CH(CH_3)-(CH_2)_2-ONO_2$ | $-OCH_3$ | " |
| $-(CH_2)_4-ONO_2$ | $-OCH_3$ | " |
| $-(CH_2)_3-CH(CH_3)-ONO_2$ | $-OCH_3$ | " |
| $-CH_2-O-CH_2-CH_2-ONO_2$ | $-OCH_3$ | " |
| $-CH_2-S-CH_2-CH_2-ONO_2$ | $-OCH_3$ | " |
| $-CH(CH_3)-S-CH_2-CH_2-ONO_2$ | $-OCH_3$ | " |
| 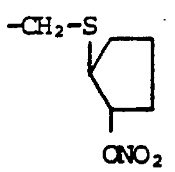 | $-OCH_3$ | " |
| 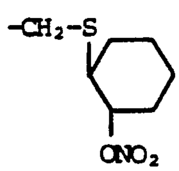 | $-OCH_3$ | " |

| Y | $R_5$ | $R_6$ |
|---|---|---|
| 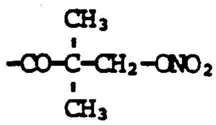 | $-OCH_3$ | $-C_6H_{13}$ |
| 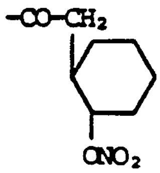 | $-OCH_3$ | " |
| 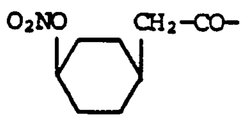 | $-OCH_3$ | " |
| 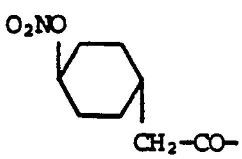 | $-OCH_3$ | " |
| 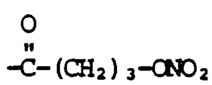 | $-OCH_3$ | " |

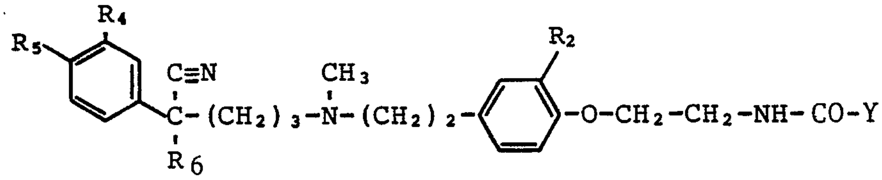

| Y | $R_2$ | $R_6$ | $R_4$ | $R_5$ |
|---|---|---|---|---|
| $-CH_2-\overset{CH_3}{CH}-ONO_2$ | $-OCH_3$ | $-CH(CH_3)_2$ | $-OCH_3$ | $-OCH_3$ |
| $-\overset{CH_3}{CH}-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-\overset{CH_3}{CH}-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-\overset{CH_3}{CH}-S-CH_2-CH_2-ONO_2$ | $-OCH_3$ | " | $-OCH_3$ | $-OCH_3$ |
| 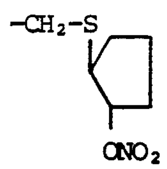 | $-OCH_3$ | " | $-OCH_3$ | $-OCH_3$ |
| 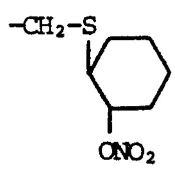 | $-OCH_3$ | " | $-OCH_3$ | $-OCH_3$ |

| Y | $R_2$ | $R_6$ | $R_4$ | $R_5$ |
|---|---|---|---|---|
| 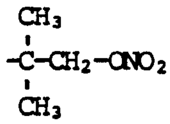 | $-OCH_3$ | $-CH(CH_3)_2$ | $-OCH_3$ | $-OCH_3$ |
| 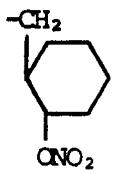 | $-OCH_3$ | " | $-OCH_3$ | $-OCH_3$ |
| 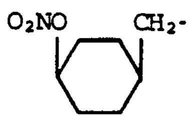 | $-OCH_3$ | " | $-OCH_3$ | $-OCH_3$ |
| 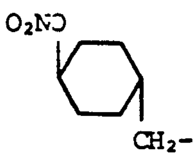 | $-OCH_3$ | " | $-OCH_3$ | $-OCH_3$ |
| $-(CH_2)_3-ONO_2$ | $-OCH_3$ | " | $-OCH_3$ | $-OCH_3$ |

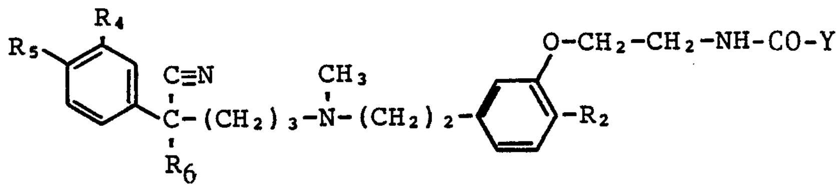

| Y | $R_2$ | $R_6$ | $R_4$ | $R_5$ |
|---|---|---|---|---|
| $CH_3$<br>$-CH_2-CH-ONO_2$ | $-OCH_3$ | $-CH(CH_3)_2$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 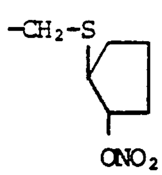 | $-OCH_3$ | " | $-OCH_3$ | $-OCH_3$ |
| 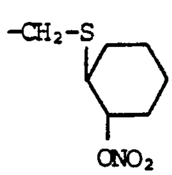 | $-OCH_3$ | " | $-OCH_3$ | $-OCH_3$ |

| Y | $R_2$ | $R_6$ | $R_4$ | $R_5$ |
|---|---|---|---|---|
| 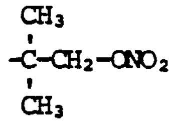 | $-OCH_3$ | $-CH(CH_3)_2$ | $-OCH_3$ | $-OCH_3$ |
| 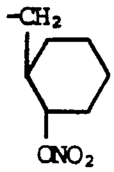 | $-OCH_3$ | " | $-OCH_3$ | $-OCH_3$ |
| 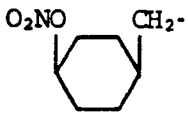 | $-OCH_3$ | " | $-OCH_3$ | $-OCH_3$ |
| 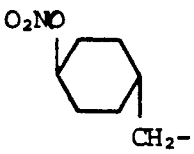 | $-OCH_3$ | " | $-OCH_3$ | $-OCH_3$ |
| $-(CH_2)_3-ONO_2$ | $-OCH_3$ | " | $-OCH_3$ | $-OCH_3$ |

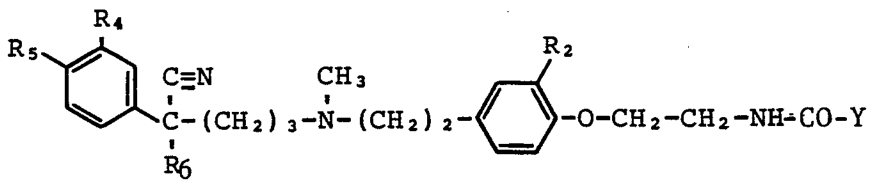

| Y | $R_2$ | $R_6$ | $R_4$ | $R_5$ |
|---|---|---|---|---|
| $CH_3$<br>$-CH_2-CH-ONO_2$ | $-OCH_3$ | $-CH(CH_3)_2$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 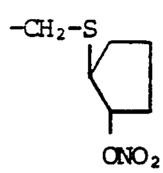 | $-OCH_3$ | " | $-OCH_3$ | $-OCH_3$ |
| 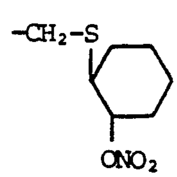 | $-OCH_3$ | " | $-OCH_3$ | $-OCH_3$ |

| Y | $R_2$ | $R_6$ | $R_4$ | $R_5$ |
|---|---|---|---|---|
| 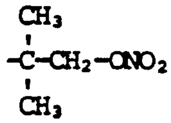 | $-OCH_3$ | $-CH(CH_3)_2$ | $-OCH_3$ | $-OCH_3$ |
| 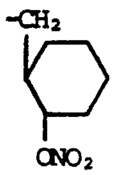 | $-OCH_3$ | " | $-OCH_3$ | $-OCH_3$ |
| 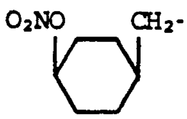 | $-OCH_3$ | " | $-OCH_3$ | $-OCH_3$ |
| 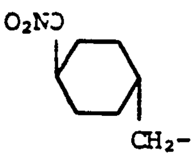 | $-OCH_3$ | " | $-OCH_3$ | $-OCH_3$ |
| $-(CH_2)_3-ONO_2$ | $-OCH_3$ | " | $-OCH_3$ | $-OCH_3$ |

0175249

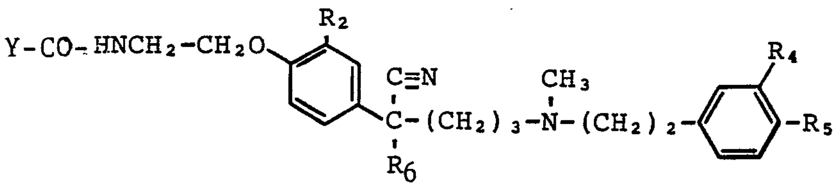

| Y | $R_2$ | $R_6$ | $R_4$ | $R_5$ |
|---|---|---|---|---|
| $CH_3$<br>$-CH_2-CH-ONO_2$ | $-OCH_3$ | $-CH(CH_3)_2$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 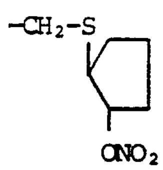 | $-OCH_3$ | " | $-OCH_3$ | $-OCH_3$ |
| 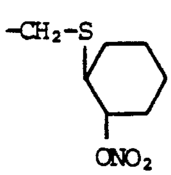 | $-OCH_3$ | " | $-OCH_3$ | $-OCH_3$ |

| Y | $R_2$ | $R_6$ | $R_4$ | $R_5$ |
|---|---|---|---|---|
| 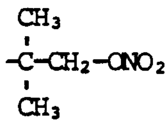 | $-OCH_3$ | $-CH(CH_3)_2$ | $-OCH_3$ | $-OCH_3$ |
| 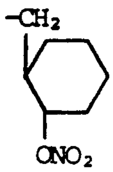 | $-OCH_3$ | " | $-OCH_3$ | $-OCH_3$ |
| 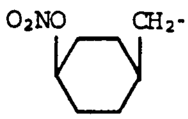 | $-OCH_3$ | " | $-OCH_3$ | $-OCH_3$ |
| 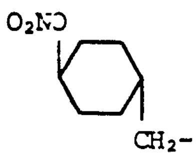 | $-OCH_3$ | " | $-OCH_3$ | $-OCH_3$ |
| $-(CH_2)_3-ONO_2$ | $-OCH_3$ | " | $-OCH_3$ | $-OCH_3$ |

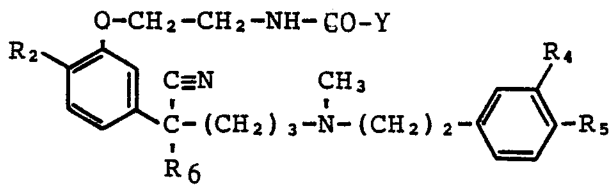

| Y | $R_2$ | $R_6$ | $R_4$ | $R_5$ |
|---|---|---|---|---|
| $-CH_2-\overset{CH_3}{CH}-ONO_2$ | $-OCH_3$ | $-CH(CH_3)_2$ | $-OCH_3$ | $-OCH_3$ |
| $-\overset{CH_3}{CH}-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-\overset{CH_3}{CH}-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-\overset{CH_3}{CH}-S-CH_2-CH_2-ONO_2$ | $-OCH_3$ | " | $-OCH_3$ | $-OCH_3$ |
| 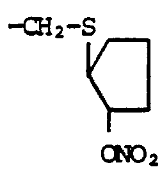 | $-OCH_3$ | " | $-OCH_3$ | $-OCH_3$ |
| 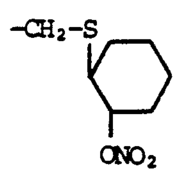 | $-OCH_3$ | " | $-OCH_3$ | $-OCH_3$ |

| Y | $R_2$ | $R_6$ | $R_4$ | $R_5$ |
|---|---|---|---|---|
| 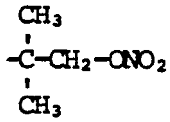 | $-OCH_3$ | $-CH(CH_3)_2$ | $-OCH_3$ | $-OCH_3$ |
| 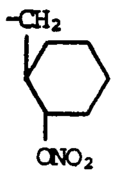 | $-OCH_3$ | " | $-OCH_3$ | $-OCH_3$ |
| 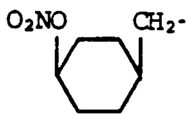 | $-OCH_3$ | " | $-OCH_3$ | $-OCH_3$ |
| 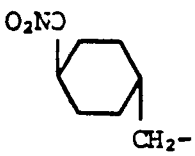 | $-OCH_3$ | " | $-OCH_3$ | $-OCH_3$ |
| $-(CH_2)_3-ONO_2$ | $-OCH_3$ | " | $-OCH_3$ | $-OCH_3$ |

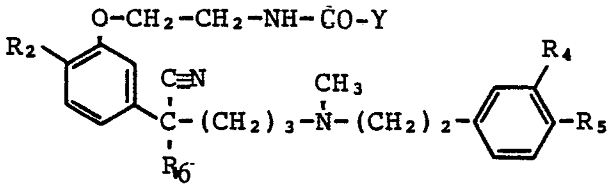

| Y | $R_2$ | $R_6$ | $R_4$ | $R_5$ |
|---|---|---|---|---|
| $-CH_2-CH(CH_3)-ONO_2$ | $-OCH_3$ | $-CH(CH_3)_2$ | $-OCH_3$ | $-OCH_3$ |
| $-CH(CH_3)-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-CH(CH_3)-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-CH(CH_3)-S-CH_2-CH_2-ONO_2$ | $-OCH_3$ | " | $-OCH_3$ | $-OCH_3$ |
| 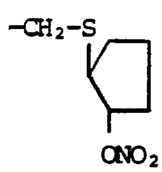 | $-OCH_3$ | " | $-OCH_3$ | $-OCH_3$ |
| 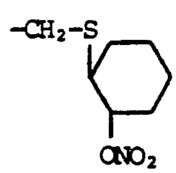 | $-OCH_3$ | " | $-OCH_3$ | $-OCH_3$ |

| Y | $R_2$ | $R_6$ | $R_4$ | $R_5$ |
|---|---|---|---|---|
| 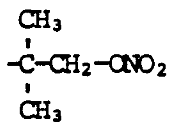 | $-OCH_3$ | $-CH(CH_3)_2$ | $-OCH_3$ | $-OCH_3$ |
| 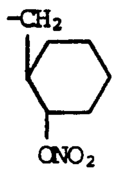 | $-OCH_3$ | " | $-OCH_3$ | $-OCH_3$ |
| 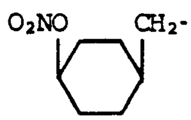 | $-OCH_3$ | " | $-OCH_3$ | $-OCH_3$ |
| 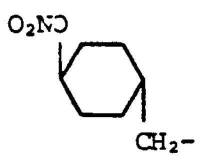 | $-OCH_3$ | " | $-OCH_3$ | $-OCH_3$ |
| $-(CH_2)_3-ONO_2$ | $-OCH_3$ | " | $-OCH_3$ | $-OCH_3$ |

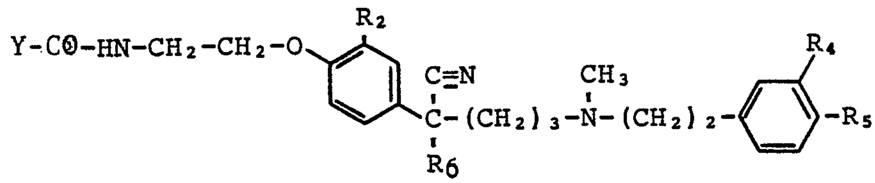

| Y | $R_2$ | $R_6$ | $R_4$ | $R_5$ |
|---|---|---|---|---|
| $-CH_2-\overset{CH_3}{CH}-ONO_2$ | $-OCH_3$ | $-CH(CH_3)_2$ | $-OCH_3$ | $-OCH_3$ |
| $-\overset{CH_3}{CH}-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-\overset{CH_3}{CH}-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-\overset{CH_3}{CH}-S-CH_2-CH_2-ONO_2$ | $-OCH_3$ | " | $-OCH_3$ | $-OCH_3$ |
| 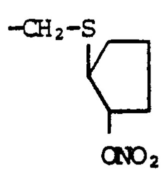 | $-OCH_3$ | " | $-OCH_3$ | $-OCH_3$ |
| 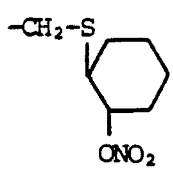 | $-OCH_3$ | " | $-OCH_3$ | $-OCH_3$ |

| Y | $R_2$ | $R_6$ | $R_4$ | $R_5$ |
|---|---|---|---|---|
| 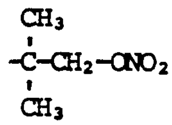 | $-OCH_3$ | $-CH(CH_3)_2$ | $-OCH_3$ | $-OCH_3$ |
| 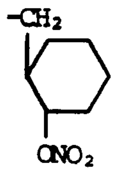 | $-OCH_3$ | " | $-OCH_3$ | $-OCH_3$ |
| 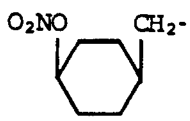 | $-OCH_3$ | " | $-OCH_3$ | $-OCH_3$ |
| 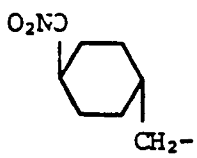 | $-OCH_3$ | " | $-OCH_3$ | $-OCH_3$ |
| $-(CH_2)_3-ONO_2$ | $-OCH_3$ | " | $-OCH_3$ | $-OCH_3$ |

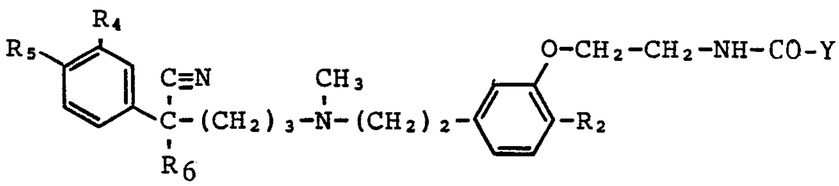

| Y | $R_2$ | $R_6$ | $R_4$ | $R_5$ |
|---|---|---|---|---|
| $-CH_2-CH(CH_3)-ONO_2$ | $-OCH_3$ | $-CH(CH_3)_2$ | $-OCH_3$ | $-OCH_3$ |
| $-CH(CH_3)-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-CH(CH_3)-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-CH(CH_3)-S-CH_2-CH_2-ONO_2$ | $-OCH_3$ | " | $-OCH_3$ | $-OCH_3$ |
| 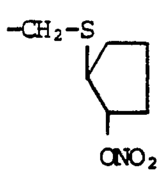 | $-OCH_3$ | " | $-OCH_3$ | $-OCH_3$ |
| 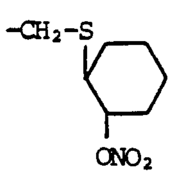 | $-OCH_3$ | " | $-OCH_3$ | $-OCH_3$ |

| Y | $R_2$ | $R_6$ | $R_4$ | $R_5$ |
|---|---|---|---|---|
| 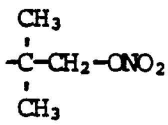 | $-OCH_3$ | $-CH(CH_3)_2$ | $-OCH_3$ | $-OCH_3$ |
| 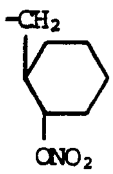 | $-OCH_3$ | " | $-OCH_3$ | $-OCH_3$ |
| 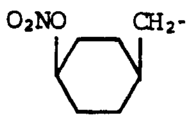 | $-OCH_3$ | " | $-OCH_3$ | $-OCH_3$ |
| 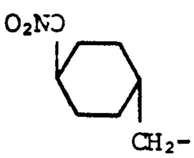 | $-OCH_3$ | " | $-OCH_3$ | $-OCH_3$ |
| $-(CH_2)_3-ONO_2$ | $-OCH_3$ | " | $-OCH_3$ | $-OCH_3$ |

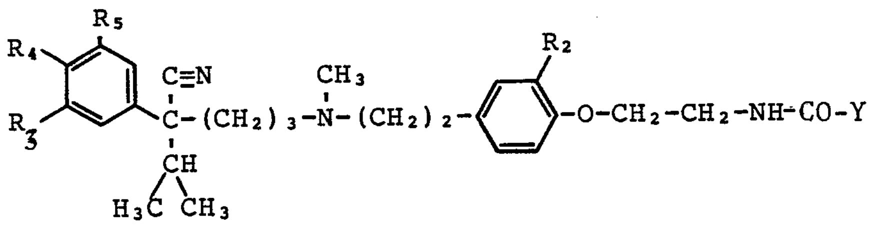

| Y | $R_2$ | $R_3$ | $R_4$ | $R_5$ |
|---|---|---|---|---|
| $CH_3$<br>$-CH_2-CH-ONO_2$ | $-OCH_3$ | $-OCH_3$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 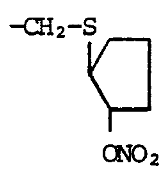 | $-OCH_3$ | " | $-OCH_3$ | $-OCH_3$ |
| 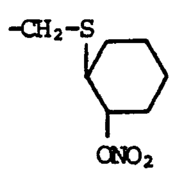 | $-OCH_3$ | " | $-OCH_3$ | $-OCH_3$ |

| Y | $R_2$ | $R_3$ | $R_4$ | $R_5$ |
|---|---|---|---|---|
| 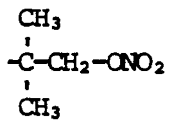 | $-OCH_3$ | $-OCH_3$ | $-OCH_3$ | $-OCH_3$ |
| 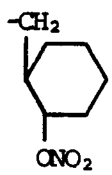 | $-OCH_3$ | " | $-OCH_3$ | $-OCH_3$ |
| 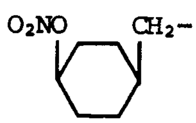 | $-OCH_3$ | " | $-OCH_3$ | $-OCH_3$ |
| 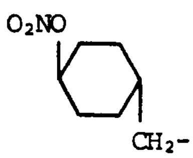 | $-OCH_3$ | " | $-OCH_3$ | $-OCH_3$ |
| $-(CH_2)_3-ONO_2$ | $-OCH_3$ | " | $-OCH_3$ | $-OCH_3$ |

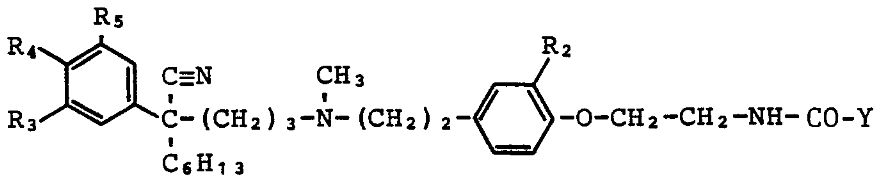

| Y | $R_2$ | $R_3$ | $R_4$ | $R_5$ |
|---|---|---|---|---|
| $CH_3$<br>$-CH_2-CH-ONO_2$ | $-OCH_3$ | $-OCH_3$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 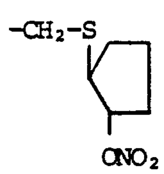 | $-OCH_3$ | " | $-OCH_3$ | $-OCH_3$ |
| 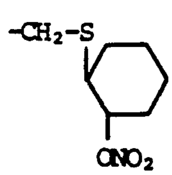 | $-OCH_3$ | " | $-OCH_3$ | $-OCH_3$ |

| Y | $R_2$ | $R_3$ | $R_4$ | $R_5$ |
|---|---|---|---|---|
| 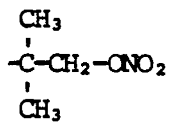 | $-OCH_3$ | $-OCH_3$ | $-OCH_3$ | $-OCH_3$ |
| 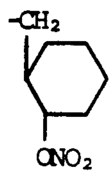 | $-OCH_3$ | " | $-OCH_3$ | $-OCH_3$ |
| 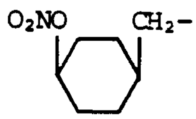 | $-OCH_3$ | " | $-OCH_3$ | $-OCH_3$ |
| 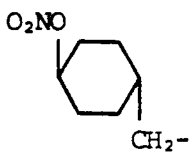 | $-OCH_3$ | " | $-OCH_3$ | $-OCH_3$ |
| $-(CH_2)_3-ONO_2$ | $-OCH_3$ | " | $-OCH_3$ | $-OCH_3$ |

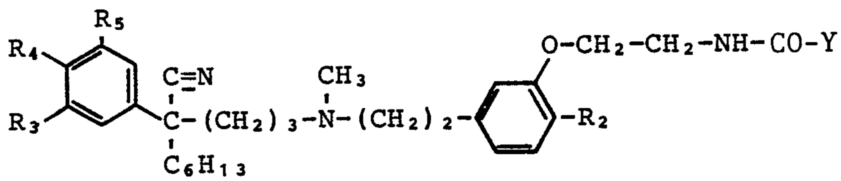

| Y | $R_2$ | $R_3$ | $R_4$ | $R_5$ |
|---|---|---|---|---|
| $CH_3$<br>$-CH_2-CH-ONO_2$ | $-OCH_3$ | $-OCH_3$ | $-OCH_3$ | $-OCH_3$ |
| $CH_3$<br>$-CH-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $CH_3$<br>$-(CH_2)_3-CH-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $CH_3$<br>$-CH-S-CH_2-CH_2-ONO_2$ | $-OCH_3$ | " | $-OCH_3$ | $-OCH_3$ |
| 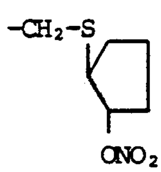 | $-OCH_3$ | " | $-OCH_3$ | $-OCH_3$ |
| 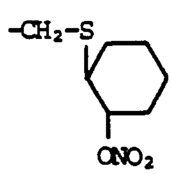 | $-OCH_3$ | " | $-OCH_3$ | $-OCH_3$ |

| Y | $R_2$ | $R_3$ | $R_4$ | $R_5$ |
|---|---|---|---|---|
| 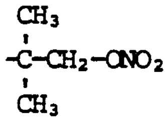 | $-OCH_3$ | $-OCH_3$ | $-OCH_3$ | $-OCH_3$ |
| 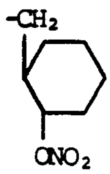 | $-OCH_3$ | " | $-OCH_3$ | $-OCH_3$ |
| 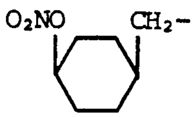 | $-OCH_3$ | " | $-OCH_3$ | $-OCH_3$ |
| 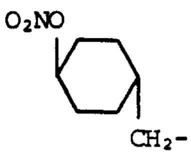 | $-OCH_3$ | " | $-OCH_3$ | $-OCH_3$ |
| $-(CH_2)_3-ONO_2$ | $-OCH_3$ | " | $-OCH_3$ | $-OCH_3$ |

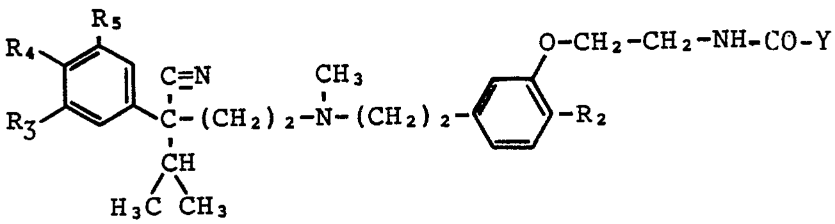

| Y | $R_2$ | $R_3$ | $R_4$ | $R_5$ |
|---|---|---|---|---|
| $-CH_2-CH(CH_3)-ONO_2$ | $-OCH_3$ | $-OCH_3$ | $-OCH_3$ | $-OCH_3$ |
| $-CH(CH_3)-(CH_2)_2-ONO_2$ | $-OCH_3$ | " | $-OCH_3$ | $-OCH_3$ |
| $-(CH_2)_4-ONO_2$ | $-OCH_3$ | " | $-OCH_3$ | $-OCH_3$ |
| $-(CH_2)_3-CH(CH_3)-ONO_2$ | $-OCH_3$ | " | $-OCH_3$ | $-OCH_3$ |
| $-CH_2-O-CH_2-CH_2-ONO_2$ | $-OCH_3$ | " | $-OCH_3$ | $-OCH_3$ |
| $-CH_2-S-CH_2-CH_2-ONO_2$ | $-OCH_3$ | " | $-OCH_3$ | $-OCH_3$ |
| $-CH(CH_3)-S-CH_2-CH_2-ONO_2$ | $-OCH_3$ | " | $-OCH_3$ | $-OCH_3$ |
| 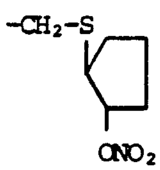 | $-OCH_3$ | " | $-OCH_3$ | $-OCH_3$ |
| 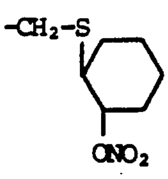 | $-OCH_3$ | " | $-OCH_3$ | $-OCH_3$ |

| Y | $R_2$ | $R_3$ | $R_4$ | $R_5$ |
|---|---|---|---|---|
| 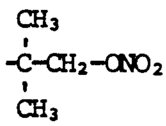 | $-OCH_3$ | $-OCH_3$ | $-OCH_3$ | $-OCH_3$ |
| 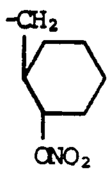 | $-OCH_3$ | " | $-OCH_3$ | $-OCH_3$ |
| 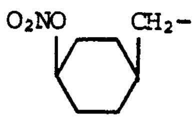 | $-OCH_3$ | " | $-OCH_3$ | $-OCH_3$ |
| 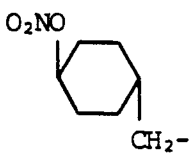 | $-OCH_3$ | " | $-OCH_3$ | $-OCH_3$ |
| $-(CH_2)_3-ONO_2$ | $-OCH_3$ | " | $-OCH_3$ | $-OCH_3$ |

## Beschreibung der Versuche

### Beispiel 1:

**2-Methoxy-4-<1-cyano-1-(methylethyl)-4-[N-methyl-N-[2-(3,4-dimethoxy-phenyl)-ethyl]-amino-butyl]>-phenoxy-N-(nitro-oxy-ethyl)-acetamid-oxalat-Dihydrat**

3 g 2-Methoxy-4-<1-cyano-1-(methylethyl)-4-[N-methyl-N-[2-(3,4-dimethoxy-phenyl)-ethyl]-amino-butyl]>-phenoxyessigsaeure werden in einer Mischung aus 60 ml Methylenchlorid und 1.27 ml Triethylamin geloest. Hierzu tropft man unter Ruehren bei -15°C eine Loesung von 1 ml Chlorameisensaeureethylester in 30 ml Methylenchlorid. Nach beendeter Zugabe ruehrt man noch 45 min bei -15°C weiter und gibt dann 1.6 g Nitro-oxyethyl-ammonium-nitrat zu. Anschließend tropft man unter Ruehren bei -15°C eine Loesung von 2.75 ml Triethylamin in 25 ml Methylenchlorid zu. Man ruehrt noch 30 min bei -15°C weiter, verduennt die Reaktionsloesung mit 50 ml Methylenchlorid und waescht mehrmals mit Wasser. Die Methylenchloridphase wird ueber Natriumsulfat getrocknet und eingedampft.

Der Rueckstand wird zur weiteren Reinigung an einer Kieselgelsaeule chromatographiert (220 g Kieselgel-60; Laufmittel: Methylenchlorid/4 % Methanol). Die erhaltenen Saeulenfraktionen werden eingedampft, der Rueckstand in Methanol geloest und die Loesung mit einer methanolischer Loesung von 0.8 g Oxalsaeure-Dihydrat versetzt. Die Loesung wird eingedampft und der Rueckstand mit Diisopropylether behandelt. Man erhaelt 3.75 g (= 87 % d.Th.) Kristalle vom Schmp: 124-125°C (Z).

**Analog wurden dargestellt:**

**Beispiel 2:**

**2-Methoxy-4-<1-cyano-1-(methylethyl)-4-[N-methyl-N-[2-(3,4-dimethoxy-phenyl)-ethyl]-amino-butyl]>-phenoxy-N-(2-nitro-oxy-propyl)-acetamid-Oxalat Schmp: 115-116°C (Z)**

**Beispiel 3:**

2-Methoxy-4-<1-cyano-1-(methylethyl)-4-[N-methyl-N-[2-(3,4-dimethoxy-phenyl)-ethyl]-amino-butyl]>-phenoxy-N-(3-nitro-oxy-butyl-2)-acetamid-Oxalat Schmp:136-137°C (Z)

**Beispiel 4:**

2-Methoxy-4-<1-cyano-1-(methylethyl)-4-[N-methyl-N-[2-(3,4-dimethoxy-phenyl)-ethyl]-amino-butyl]>-phenoxy-N-(2-methyl-3-nitro-oxy-propyl-2)-acetamid

**Beispiel 5:**

2-Methoxy-4-<1-cyano-1-(methylethyl)-4-[N-methyl-N-[2-(3,4-dimethoxy-phenyl)-ethyl]-amino-butyl]>-phenoxy-N-(1,3-bis-nitro-oxy-cyclohexyl-2)-acetamid

**Beispiel 6:**

**2-Methoxy-4-<1-cyano-1-(methylethyl)-4-[N-methyl-N-[2-(3,4-dimethoxy-phenyl)-ethyl]-amino-butyl]>-phenoxy-N,N-bis-(nitro-oxy-ethyl)-acetamid-oxalat-Dihydrat Schmp: 105-107°C (Z)**

**Beispiel 7:**

2-Methoxy-4-<1-cyano-1-(methylethyl)-4-[N-methyl-N-[2-(3,4-dimethoxy-phenyl)-ethyl]-amino-butyl]>-phenoxy-N,N-bis-(2-nitro-oxy-propyl)-acetamid-oxalat-Hydrat Schmp: 118-120°C (Z)

Die als Ausgangsprodukt benoetigte 2-Methoxy-4-<1-cyano-1-(methylethyl)-4-[N-methyl-N-[2-(3,4-dimethoxy-phenyl)-ethyl]-amino-butyl]>-phenoxyessigsaeure wurde wie folgt hergestellt.

1. Stufe: 3-Methoxy-4-benzyloxy-α-isopropyl-benzylcyanid

150 g 3-Methoxy-4-benzyloxy-benzylcyanid werden zusammen mit 470 ml 50proz. Natronlauge, 76 ml 2-Brompropan und 6 g Benzyltributylammonium-bromid 5 Stdn. bei 55°C geruehrt. Dann gießt man die Reaktionsmischung auf 4 Liter Wasser und extrahiert mehrmals mit Methylenchlorid. Die vereinigten Methylenchloridphasen werden ueber Natriumsulfat getrocknet, eingedampft und der Rueckstand im Hochvakuum destilliert.

Man erhaelt 155 g (= 90 % d.Th.) 3-Methoxy-4-benzyloxy-α-isopropyl-benzylcyanid.
Kp.: 184-186°C/$_{6.10^{-2} mm}$ ; Schmp: 68-70°C.

2. Stufe: α-Isopropyl-α-[(N-methyl-N-homoveratryl)-amino-γ-propyl]-3-methoxy-4-benzyloxy-phenylacetonitril

36.6 g 3-Methoxy-4-benzyloxy-α-isopropyl-benzylcyanid werden in 150 ml absol. Dimethylformamid geloest. Hierzu gibt man 30.3 g (N-Methyl-N-homoveratryl)-amino-γ-chlorpropan und erwaermt die Mischung auf 50°C. Hierzu gibt man im Verlauf einer Stunde unter Ruehren 5.7 g 55proz. Natriumhdrid. Nach beendeter Zugabe ruehrt man noch zwei Stunden bei 50°C weiter und gießt dann das Reaktionsgemisch auf 4 Liter Wasser. Man extrahiert mehrmals mit Methylenchlorid, trocknet die vereinigten Methylenchloridphasen ueber Natriumsulfat und dampft zur Trockne ein. Der Rueckstand wird zur Reinigung an einer Kieselgelsaeule chromatographiert (1.2 kg Kieselgel; Elutionsmittel: Methylenchlorid/2 % Methanol).

Nach dem Eindampfen der Saeulenfraktionen erhaelt man 45 g (= 70 % d.Th.) α-Isopropyl-α-[(N-methyl-N-homoveratryl)-amino-γ-propyl]-3-methoxy-4-benzyloxy-phenylacetonitril als oeliges Produkt.

3. Stufe: α-Isopropyl-α-[(N-methyl-N-homoveratryl)-amino-γ-propyl]-3-methoxy-4-hydroxy-phenylacetonitril

45 g α-Isopropyl-α-[(N-methyl-N-homoveratryl)-amino-γ-propyl]-3-methoxy-4-benzyloxy-phenylacetonitril werden in 400 ml Methanol geloest und nach Zugabe von 4 g Pd/C (10proz.)-Katalysator hydriert. Nach Aufnahme der berechneten Menge an Wasserstoff wird vom Katalysator abfiltriert und eingedampft. Man erhaelt 36 g (= 97 % d.Th.) α-Isopropyl-α-[(N-methyl-N-homoveratryl)-amino-γ-propyl]-3-methoxy-4-hydroxy-phenylacetonitril als oeliges Produkt.

4. Stufe: 2-Methoxy-4-<1-cyano-1-(methylethyl)-4-[N-methyl-N-[2-(3,4-dimethoxy-phenyl)-ethyl]-amino-butyl]>-phenoxy-essigsaeureethylester

34.5 g α-Isopropyl-α-[(N-methyl-N-homoveratryl)-amino-γ-propyl]-3-methoxy-4-hydroxy-phenylacetonitril werden in 200 ml Dimethylformamid geloest und unter Ruehren bei Raumtemperatur mit 3.5 g Natriumhydrid (55proz.) versetzt. Man ruehrt noch 15 min weiter und tropft dann eine Loesung von 9.8 ml Bromessigsaeureethylester in 50 ml Dimethylformamid zu. Die Temperatur soll hierbei 30°C nicht uebersteigen. Nach beendeter Zugabe ruehrt man noch 1 Stunde weiter, gießt das Reaktionsgemisch auf 4 Liter Wasser und extrahiert mehrmals mit Methylenchlorid. Die vereinigten Methylenchloridphasen werden ueber Natriumsulfat getrocknet und dann eingedampft. Der Rueckstand wird zur Reinigung an einer Kieselgelsaeule chromatographiert. (1.2 kg Kieselgel; Elutionsmittel: Methylenchlorid/3 % Methanol).

Nach dem Eindampfen der Saeulenfraktionen erhaelt man 33.3 g (= 81 % d.Th.) 2-Methoxy-4-<1-cyano-1-(methylethyl)-4-[N-methyl-N-[2-(3,4-dimethoxy-phenyl)-ethyl]-amino-butyl]>-phenoxyessigsaeureethvlester als oeliges Produkt.

5. Stufe: 2-Methoxy-4-<1-cyano-1-(methylethyl)-4-[N-methyl-N-[2-(3,4-dimethoxy-phenyl)-ethyl]-amino-butyl]>-phenoxyessigsaeure

2-Methoxy-4-<1-cyano-1-(methylethyl)-4-[N-methyl-N-[2-(3,4-dimethoxy-phenyl)-ethyl]-amino-butyl]>-phenoxyessigsäureethylester werden in 150 ml Ethanol gelöst. Hierzu tropft man unter Ruehren bei Raumtemperatur 125 ml 1 N . Natronlauge. Nach beendeter Zugabe ruehrt man noch 1 Stunde bei Raumtemperatur weiter und dampft dann den Alkohol ab. Dann gibt man 125 ml 1 N Salzsaeure zu und extrahiert mehrmals mit Methylenchlorid.

Die vereinigten Methylenchloridphasen werden ueber Natriumsulfat getrocknet und eingedampft. Man erhaelt 29.5 g (= 94 % d.Th.) 2-Methoxy-4-<1-cyano-1-(methylethyl-4-[N-methyl-N-[2-(3,4-dimethoxy-phenyl)-ethyl]-amino-butyl]>-phenoxyessigsaeure als oeliges Produkt.

Beispiel 8:

2-Methoxy-5-<1-cyano-1-(methylethyl)-4-[N-methyl-N-[2-(3,4-dimethoxy-phenyl)-ethyl]-amino-butyl]>-phenoxy-N-(nitro-oxy-ethyl)-acetamid-oxalat-Hydrat

4.5 g 2-Methoxy-5-<1-cyano-1-(methylethyl)-4-[N-methyl-N-[2-(3,4-dimethoxy-phenyl)-ethyl]-amino-butyl]>-phenoxyessigsaeure werden in einer Mischung aus 80 ml Methylenchlorid und 1.9 ml Triethylamin geloest. Hierzu tropft man unter Ruehren bei -15°C eine Loesung von 1.35 ml Chlorameisensaeureethylester in 30 ml Methylenchlorid. Nach beendeter Zugabe ruehrt man noch 45 min bei -15°C weiter und gibt dann 2.3 g Nitro-oxyethyl-ammoniumnitrat zu. Anschließend tropft man unter Ruehren bei -15°C eine Loesung von 3.8 ml Triethylamin in 25 ml Methylenchlorid zu. Man ruehrt noch 30 min bei -15°C weiter, verduennt die Reaktionsloesung mit 50 ml Methylenchlorid und waescht mehrmals mit Wasser. Die Methylenchloridphase wird ueber Natriumsulfat getrocknet und eingedampft. Der Rueckstand wird zur Reinigung an einer Kieselgelsaeule chromatographiert. (250 g Kieselgel; Elutionsmittel: Methylenchlorid/3 % Methanol).

Die erhaltenen Saeulenfraktionen werden eingedampft, der Rueckstand in Methanol geloest und die Loesung mit einer methanolischen Loesung von 1.25 g Oxalsaeure-Dihydrat versetzt. Die Loesung wird eingedampft und der Rueckstand mit Diisopropylether behandelt. Man erhaelt 4.9 g (= 78 % d.Th.) Kristalle vom Schmp. 127-129°C (Z).

**Analog wurden dargestellt:**

Beispiel 9:

2-Methoxy-5-<1-cyano-1-(methylethyl-4-[N-methyl-N-[2-(3,4-dimethoxy-phenyl)-ethyl]-amino-butyl]>-phenoxy-N-(2-nitro-oxy-propyl)-acetamid-oxalat-Hydrat. Schmp. 132-134°C (Z).

Beispiel 10:

2-Methoxy-5-<1-cyano-1-(methylethyl)-4-[N-methyl-N-[2-(3,4-dimethoxy-phenyl)-ethyl]-amino-butyl]>-phenoxy-N-(3-nitro-oxy-butyl-2)-acetamid-oxalat-Dihydrat. Schmp. 123-125°C (Z).

Beispiel 11:

2-Methoxy-5-<1-cyano-1-(methylethyl)-4-[N-methyl-N-[2-(3,4-dimethoxy-phenyl)-ethyl]-amino-butyl]>-phenoxy-N-(2-methyl-3-nitro-oxy-propyl-2)-acetamid-oxalat-Hydrat. Schmp. 125-127°C (Z).

Beispiel 12:

2-Methoxy-5-<1-cyano-1-(methylethyl)-4-[N-methyl-N-[2-(3,4-dimethoxy-phenyl)-ethyl]-amino-butyl]>-phenoxy-N-(1,3-bis-nitro-oxy-cyclohexyl-2)-acetamid-oxalat-Hydrat. Schmp. 140-142°C (Z).

**Beispiel 13:**

2-Methoxy-5-<1-cyano-1-(methylethyl)-4-[N-methyl-N-[2-(3,4-dimethoxy-phenyl)-ethyl]-amino-butyl]>-phenoxy-N,N-bis-(nitro-oxy-ethyl)-acetamid-oxalat-Hydrat. Schmp. 105-107°C (Z).

**Beispiel 14:**

2-Methoxy-5-<1-cyano-1-(methylethyl)-4-[N-methyl-N-[2-(3,4-dimethoxy-phenyl)-ethyl]-amino-butyl]>-phenoxy-N,N-bis-(2-nitro-oxy-propyl)-acetamid-oxalat-Hydrat. Schmp. 118-120°C (Z).

Die als Ausgangsmaterial benoetigte 2-Methoxy-5-<1-cyano-1-(methylethyl)-4-[N-methyl-N-[2-(3,4-dimethoxy-phenyl)-ethyl]-amino-butyl]>-phenoxyessigsaeure wurde analog der 2-Methoxy-4-<1-cyano-1-(methylethyl)-4-[N-methyl-N-[2-(3,4-dimethoxy-phenyl)-ethyl]-amino-butyl]>-phenoxyessigsaeure dargestellt, nur daß anstelle von 3-Methoxy-4-benzyloxy-benzylcyanid das 3-Benzyloxy-4-methoxy-benzylcyanid als Ausgangsmaterial benutzt wurde und mit 2-Nitro-oxy-propyl-ammoniumnitrat umgesetzt wird.

Beispiel 15

2-Methoxy-4-<2-[N-methyl-N-[4-cyano-4-(methylethyl)-4-(3,4-dimethoxy-phenyl)-butyl]-amino-ethyl]>-phenoxy-N-(nitro-oxy-ethyl)-acetamid-oxalat-Hydrat

3 g 2-Methoxy-4-<2-[N-methyl-N-[4-cyano-4-(methylethyl)-4-(3,4-dimethoxy-phenyl)-butyl]-amino-ethyl]>-phenoxyessigsaeure werden in einer Mischung aus 60 ml Methylenchlorid und 1.3 ml Triethylamin geloest. Hierzu tropft man unter Ruehren bei -15°C eine

0175249

Loesung von 1 ml Chlorameisensaeureethylester in 30 ml Methylenchlorid. Nach beendeter Zugabe ruehrt man noch 45 min bei -15°C weiter, gibt 1.6 g Nitro-oxy-ethyl-ammonium-nitrat zu und tropft dann eine Loesung von 2.9 ml Triethylamin in 30 ml Methylenchlorid zu. Man ruehrt noch 30 min bei -15°C weiter, verduennt die Reaktionsloesung mit 50 ml Methylenchlorid und waescht mehrmals mit Wasser. Die Methylenchloridphase wird ueber Natriumsulfat getrocknet und eingedampft. Der Rueckstand wird zu weiteren Reinigung an einer Kieselgelsaeule chromatographiert. (220 g Kieselgel; Elutionsmittel: Methanol/4 % Methylenchlorid).

Die erhaltenen Saeulenfraktionen werden eingedampft, der Rueckstand in Methanol geloest und die Loesung mit einer methanolischen Loesung von 850 mg Oxalsaeure-Dihydrat versetzt. Die Loesung wird eingedampft und der Rueckstand mit Diisopropylether behandelt. Man erhaelt 3.7 g (= 85 % d.Th.) Kristalle vom Schmp. 132°C (Z).

Analog wurden dargestellt:

Beispiel 16:

2-Methoxy-4-<2-[N-methyl-N-[4-cyano-4-(methylethyl)-4-(3,4-dimethoxy-phenyl)-butyl]-amino-ethyl]>-phenoxy-N-(2-nitro-oxy-propyl)-acetamid-oxalat-Hydrat. Schmp. 135-136°C (Z).

Beispiel 17:

2-Methoxy-4-<2-[N-methyl-N-[4-cyano-4-(methylethyl)-4-(3,4-dimethoxy-phenyl)-butyl]-amino-ethyl]>-phenoxy-N-(3-nitro-oxy-butyl-2)-acetamid-oxalat-Hydrat. Schmp. 132-134°C (Z).

**Beispiel 18:**

2-Methoxy-4-<2-[N-methyl-N-[4-cyano-4-(methylethyl)-4-(3,4-dimethoxy-phenyl)-butyl]-amino-ethyl]>-phenoxy-N-(2-methyl-3-nitro-oxy-propyl-2)-acetamid-oxalat-Hydrat. Schmp. 122-124°C (Z).

**Beispiel 19:**

2-Methoxy-4-<2-[N-methyl-N-[4-cyano-4-(methylethyl)-4-(3,4-dimethoxy-phenyl)-butyl]-amino-ethyl]>-phenoxy-N-(1,3-bis-nitro-oxy-cyclohexyl-2)-acetamid-Oxalat. Schmp. 90-92°C (Z).

**Beispiel 20:**

2-Methoxy-4-<2-[N-methyl-N-[4-cyano-4-(methylethyl)-4-(3,4-dimethoxy-phenyl)-butyl]-amino-ethyl]>-phenoxy-N,N-bis-(nitro-oxy-ethyl)-acetamid-oxalat-Hydrat. Schmp. 110-112°C (Z).

Beispiel 21:

2-Methoxy-4-<2-[N-methyl-N-[4-cyano-4-(methylethyl)-4-(3,4-dimethoxy-phenyl)-butyl]-amino-ethyl]>-phenoxy-N,N-bis-(2-nitro-oxy-propyl)-acetamid-oxalat-Di-hydrat. Schmp. 110-112°C (Z).

Beispiel 22:

2-Methoxy-4-<2-[N-methyl-N-[4-cyano-4-(methylethyl)-4-(3,4,5-trimethoxy-phenyl)-butyl]-amino-ethyl]>-phenoxy-N-(nitro-oxy-ethyl)acetamid-oxalat-Dihydrat. Schmp. 128-130°C (Z).

Beispiel 23:

2-Methoxy-4-<2-[N-methyl-N-(4-cyano-4-(methylethyl)-4-(3,4,5-trimethoxy-phenyl)-butyl]-amino-ethyl]>-phenoxy-N-(2-nitro-oxy-propyl)-acetamid-oxalat-Dihydrat. Schmp. 135-136°C (Z).

Beispiel 24:

2-Methoxy-4-<2-[N-methyl-N-(4-cyano-4-(methylethyl)-4-(3,4,5-trimethoxy-phenyl)-butyl]-amino-ethyl]>-phenoxy-N-(3-nitro-oxy-butyl-2)-acetamid-oxalat-Dihydrat. Schmp. 123-124°C (Z).

Beispiel 25:

2-Methoxy-4-<2-[N-methyl-N-(4-cyano-4-(methylethyl)-4-(3,4,5-trimethoxy-phenyl)-butyl]-amino-ethyl]>-phenoxy-N-(1,3-bis-nitro-oxy-cyclohexyl-2)-acetamid-Oxalat. Schmp. 147-149°C (Z).

**Die als Ausgangsmaterial benoetigte 2-Methoxy-4-<2-[N-methyl-N-[4-cyano-4-(methylethyl)-4-(3,4-dimethoxy-phenyl)-butyl]-amino-ethyl]>-phenoxyessigsaeure wurde wie folgt dargestellt.**

**1. Stufe: α-Isopropyl-α-<[N-methyl-N-[2-(3-methoxy-4-benzyloxy)-ethyl]]-amino-γ-propyl>-3,4-dimethoxy-phenylacetonitril**

48.5 g α-Isopropyl-3,4-dimethoxy-phenylacetonitril werden in 200 ml Dimethylformamid geloest. Hierzu gibt man 40.8 g N-Methyl-N-[2-(3-Methoxy-4-benzyloxy)-ethyl]-N-(γ-chlor-propyl)-amin und erwaermt die Loesung auf 50°C. Hierzu fuegt man im Verlauf einer Stunde 8.15 g Natriumhydrid (55 proz.). Nach beendeter Zugabe ruehrt man noch 3 Stunden bei 50°C weiter, gießt das Reaktionsgemisch auf 4 Liter Wasser und extrahiert mehrmals mit Methylenchlorid. Die vereinigten Methylenchloridphasen werden mit Wasser gewaschen, ueber Natriumsulfat getrocknet und eingedampft. Der Rueckstand wird zur Reinigung an einer Kieselgelsaeule chromatographiert. (1.8 kg Kieselgel; Elutionsmittel: Methylenchlorid/ 3 % Methanol).

Nach Eindampfen der Saeulenfraktionen erhaelt man 52.8 g (= 86 % d.Th.) α-Isopropyl-α-<[N-methyl-N-[2-(3-methoxy-4-benzyloxy)-ethyl]]-amino-γ-propyl>-3,4-dimethoxy-phenylacetonitril als oeliges Produkt.

**2. Stufe: α-Isopropyl-α-<[N-methyl-N-[2-(3-methoxy-4-hydroxy-phenyl)-ethyl]]-amino-γ-propyl>-3,4-dimethoxy-phenyl-acetonitril**

47.9 g α-Isoproyy-α-<[N-methyl-N-[2-(3-methoxy-4-benzyloxy)-ethyl]]-amino-γ-propyl>-3,4-dimethoxy-phenyl-acetonitril werden in 400 ml Methanol geloest und nach Zugabe von 2 g Pd/C (10proz.) Katalysator hydriert. Nach Aufnahme der berechneten Menge an Wasserstoff wird vom Katalysator abfiltriert und eingedampft. Man erhaelt 38 g (= 95 % d.Th.) α-Isopropyl-α-<[N-methyl-N-[2-(3-methoxy-4-hydroxy)-ethyl]]-amino-γ-propyl>-3,4-dimethoxy-phenyl-acetonitril als oeliges Produkt.

3. Stufe: 2-Methoxy-4-<2-[N-methyl-N-[4-cyano-4-(methylethyl)-4-(3,4-dimethoxy-phenyl)-butyl]-amino-ethyl]>-phenoxyessigsaeureethylester

37.7 g α-Isopropyl-α-<[N-methyl-N-[2-(3-methoxy-4-hydroxy)-ethyl]]-amino-γ-propyl>-3,4-dimethoxy-phenylacetonitril werden in 200 ml absol. Dimethylformamid geloest und unter Ruehren mit 3.7 g Natriumhydrid (55proz.) versetzt. Man ruehrt noch 15 min weiter und tropft dann eine Loesung von 10.7 g Bromessigsaeureethylester in 100 ml Dimethylformamid zu. Die Temperatur soll hierbei 30°C nicht uebersteigen. Nach beendeter Zugabe ruehrt man noch 1 Stunde weiter, gießt das Reaktionsgemisch auf 4 Liter Wasser und extrahiert mehrmals mit Methylenchlorid. Die vereinigten Methylenchloridphasen werden mit Wasser gewaschen, ueber Natriumsulfat getrocknet und eingedampft. Der Rueckstand wird zur Reinigung an einer Kieselgelsaeule chromatographiert. (1.2 kg Kieselgel; Elutionsmittel: Methylenchlorid/3 % Methanol).

**Nach dem Eindampfen der Saeulenfraktionen erhaelt man 36.6 g (= 81 % d.Th.) 2-Methoxy-4-<2-[N-methyl-N-[4-cyano-4-(methyl-ethyl)-4-(3,4-dimethoxy-phenyl)-butyl]-amino-ethyl]>-phenoxy-essigsaeureethylester als oeliges Produkt.**

4. Stufe: <u>2-Methoxy-4-<2-[N-methyl-N-[4-cyano-4-(methylethyl)-4-(3,4-dimethoxy-phenyl)-butyl]-amino-ethyl]>-phenoxyessigsaeure</u>

36.5 g 2-Methoxy-4-<2-[N-methyl-N-[4-cyano-4-(methylethyl)-4-(3,4-dimethoxy-phenyl)-butyl]-amino-ethyl]>-phenoxyessigsaeureethylester werden in 150 ml Ethanol geloest. Hierzu tropft man bei Raumtemperatur unter Ruehren 140 ml 1 N Natronlauge. Man ruehrt noch 1 Stunde weiter, gibt dann 140 ml 1 N Salzsaeure zu und dampft den Alkohol ab. Die waeßrige Phase wird mit Methylchlorid extrahiert. Die Methylenchloridphase wird ueber Natriumsulfat getrocknet und eingedampft. Man erhaelt 33.7 g (= 97 % d.Th.) 2-Methoxy-4-<2-[N-methyl-N-[4-cyano-4-(methyl-ethyl)-4-(3,4-dimethoxy-phenyl)-butyl]-amino-ethyl]>-phenoxyessigsaeure als oeliges Produkt.

Das in Stufe 1 eingesetzte N-Methyl-N-[2-(3-methoxy-4-benzyl-oxy)-ethyl]-N-(γ-chlor-propyl)-amin wurde wie folgt dargestellt.

148 g 3-Methoxy-4-benzyloxy-benzylcyanid werden in 2.5 Liter Methanol geloest und nach Zugabe von 350 ml fluessigem Ammoniak und 16 g Raney-Nickel bei 100°C und 80 atm. hydriert. Die Hydrierung ist nach 4 Stunden beendet. Man filtriert vom Katalysator ab, dampft ein und reinigt den Rueckstand durch Chromatographie an einer Kieselgelsaeule. (1.6 kg Kieselgel; Elutionsmittel: Methylenchlorid/5 % Isopropylamin).

Nach dem Eindampfen der Saeulenfraktionen erhaelt man 126 g (= 84 % d.Th.) 3-Methoxy-4-benzyloxy-ß-phenyl-ethyl-amin. Dieses wird in 500 ml Toluol geloest und nach Zugabe von 48.5 ml Benzaldehyd am Wasserabscheider erhitzt. Nach beendeter Wasserabscheidung laeßt man auf Raumtemperatur abkuehlen und gibt 46 ml Dimethylsulfat zu. Anschließend wird 30 min am Rueckfluß erhitzt. Man kuehlt auf Raumtemperatur ab. Hierbei bilden sich zwei Schichten. Die obere Toluolschicht wird abgetrennt und verworfen. Die untere Schicht wird mit 500 ml 80proz. Ethanol versetzt und anschließend 30 min am Rueckfluß erhitzt. Dann wird vom Alkohol abdestilliert und der Rueckstand in Methylenchlorid aufgenommen. Die Methylenchloridphase wird mit konz. Salzsaeure extrahiert und dann verworfen.

Die waeßrige Phase wird unter Kuehlung mit konz. Natronlauge alkalisch gestellt, wobei sich ein Oel abscheidet. Dieses wird in Methylenchlorid aufgenommen, die Methylenchloridphase ueber Natriumsulfat getrocknet und eingedampft. Man erhaelt 106 g (= 80 % d.Th.) 3-Methoxy-4-benzyloxy-ß-phenyl-N-methyl-ethylamin als oeliges Produkt.

Dieses wird in 500 ml Dimethylformamid geloest und nach Zugabe von 132 g Pottasche und 52 ml 1-Brom-3-chlor-propan 5 Stunden bei Raumtemperatur geruehrt. Dann wird das Reaktionsgemisch mit Methylenchlorid verduennt und der unloesliche Rueckstand abgesaugt. Das Filtrat wird mit Wasser verduennt und mehrmals mit Methylenchlorid extrahiert. Die Methylenchloridphasen werden vereinigt, ueber Natriumsulfat getrocknet und eingedampft. Der Rueckstand wird zur Reinigung an einer Kieselgelsaeule chromatographiert. (1.2 kg Kieselgel; Elutionsmittel: Methylenchlorid/3 % Methanol). Nach dem Eindampfen der Saeulenfraktionen erhaelt man 48.5 g (= 36 % d.Th.) N-Methyl-N-[2-(3-methoxy-4-benzyloxy)-ethyl]-N-(γ-chlor-propyl)-amim als oeliges Produkt.

**Beispiel 26:**

**2-Methoxy-5-<2-[N-methyl-N-[4-cyano-4-(methylethyl)-4-(3,4-dimethoxy-phenyl)-butyl]-amino-ethyl]>-phenoxy-N-(nitro-oxy-ethyl)-acetamid-Oxalat**

4.5 g 2-Methoxy-5-<2-[N-methyl-N-[4-cyano-4-(methylethyl)-4-(3,4-dimethoxy-phenyl)-butyl]-amino-ethyl]>-phenoxyessigsaeure werden in einer Mischung aus 80 ml Methylenchlorid und 1.9 ml Triethylamin geloest. Hierzu tropft man unter Ruehren bei -15°C eine Loesung von 1.4 ml Chlorameisensaeureethylester in 30 ml Methylenchlorid. Nach beendeter Zugabe ruehrt man noch 45 min bei -15°C weiter, gibt 2.3 g Nitro-oxy-ethyl-ammonium-nitrat zu und tropft dann eine Loesung von 3.8 ml Triethylamin in 30 ml absol. Methylenchlorid zu. Man ruehrt noch 30 min bei -15°C weiter, verduennt die Reaktionsloesung mit 50 ml Methylenchlorid und waescht mehrmals mit Wasser. Die Methylenchloridphase wird ueber Natriumsulfat getrocknet und eingedampft. Der Rueckstand wird zur weiteren Reinigung an einer Kieselgelsaeule chromatographiert. (220 g Kieselgel; Elutionsmittel: Methylenchlorid/3 % Methanol). Die Saeulenfraktionen werden eingedampft, der Rueckstand in Methanol geloest und die Loesung mit einer methanolischen Loesung von 1.2 g Oxalsaeure-Dihydrat versetzt. Die Loesung wird eingedampft und mit Diisopropylether behandelt. Man erhaelt 4.5 g (= 74 % d.Th.) Kristalle vom Schmp. 128-130°C (Z).

Analog wurden hergestellt:

Beispiel 27:

2-Methoxy-5-<2-[N-methyl-N-[4-cyano-4-(methylethyl)-4-(3,4-dimethoxy-phenyl)-butyl]-amino-ethyl]>-phenoxy-N-(2-nitro-oxy-propyl)-acetamid-oxalat-Hydrat. Schmp. 133-135°C (Z).

Beispiel 28:

2-Methoxy-5-<2-[N-methyl-N-[4-cyano-4-(methylethyl)-4-(3,4-dimethoxy-phenyl)-butyl]-amino-ethyl]>-phenoxy-N-(3-nitro-oxy-butyl-2)-acetamid-oxalat-Di-Hydrat. Schmp. 123-125°C.

Beispiel 29:

2-Methoxy-5-<2-[N-methyl-N-[4-cyano-4-(methylethyl)-4-(3,4-dimethoxy-phenyl)-butyl]-amino-ethyl]>-phenoxy-N-(2-methyl-3-nitro-oxy-propyl-2)-acetamid-oxalat-Hydrat.
Schmp. 128-130°C (Z).

Beispiel 30:

2-Methoxy-5-<2-[N-methyl-N-[4-cyano-4-(methylethyl)-4-(3,4-dimethoxy-phenyl)-butyl]-amino-ethyl]>-phenoxy-N-(1,3-bis-nitro-oxy-cyclohexyl-2)-acetamid-oxalat-Hydrat.
Schmp. 143-145°C (Z).

**Beispiel 31:**

2-Methoxy-5-<2-[N-methyl-N-[4-cyano-4-(methylethyl)-4-(3,4-dimethoxy-phenyl)-butyl]-amino-ethyl]>-phenoxy-N,N-bis-(nitro-oxy-ethyl)-acetamid-oxalat-Hydrat. Schmp. 110-112°C (Z).

**Beispiel 32:**

2-Methoxy-5-<2-[N-methyl-N-[4-cyano-4-(methylethyl)-4-(3,4-dimethoxy-phenyl)-butyl]-amino-ethyl]>-phenoxy-N,N-bis-(2-nitro-oxy-propyl)-acetamid-oxalat-Hydrat. Schmp. 118-120°C (Z).

Die als Ausgangsmaterial benoetigte 2-Methoxy-5-<2-[N-methyl-N-[4-cyano-4-(methylethyl)-4-(3,4-dimethoxy-phenyl)-butyl]-amino-ethyl]>-phenoxyessigsaeure wurde analog der 2-Methoxy-4-<2-[N-methyl-N-[4-cyano-4-(methylethyl)-4-(3,4-dimethoxy-phenyl)-butyl]-amino-ethyl]>-phenoxyessigsaeure dargestellt.

Analog wurden dargestellt:

**Beispiel 33:**

3-<2-[N-Methyl-N-[4-cyano-4-dodecyl-4-(3,4-dimethoxy-phenyl)-butyl]-amino-ethyl]>-phenoxy-N-(nitro-oxy-ethyl)-acetamid-Oxalat. Schmp. 134-136°C (Z).

**Beispiel 34:**

3-<2-[N-methyl-N-[4-cyano-4-dodecyl-4-(3,4-dimethoxy-phenyl)-butyl]-amino-ethyl]>-phenoxy-N-(2-nitro-oxy-propyl)-acetamid-oxalat-Hydrat. Schmp. 140-142°C (Z).

**Beispiel 35:**

3-<2-[N-methyl-N-[4-cyano-4-dodecyl-4-(3,4,5-trimethoxy-phenyl)-butyl]-amino-ethyl]>-phenoxy-N-(2-nitro-oxy-ethyl)-acetamid-oxalat-Hydrat. Schmp. 127-129°C (Z).

**Beispiel 36:**

3-<1-cyano-1-hexyl-4-[N-methyl-N-[2-(3-methoxy-phenyl)-ethyl)-amino-butyl]>-phenoxy-N-(2-nitro-oxy-propyl)-acetamid-Oxalat. Schmp. 134-136°C.

Beispiel 37:

3-<1-cyano-1-hexyl-4-[N-methyl-N-[2-(3-methoxy-phenyl)-ethyl]-amino-butyl]>-phenoxy-N-(nitro-oxy-ethyl)-acetamid-Oxalat. Schmp. ölig.

Beispiel 38:

2-Methoxy-5-<1-cyano-1-(methylethyl)-4-[N-methyl-N-[2-(3,4-dimethoxy-phenyl)-ethyl]-amino-butyl]>-phenoxy-N-(2,2-dimethyl-3-nitro-oxy-propyl-1)-acetamid-Oxalat-di-Hydrat.
Schmp. 140 C°(Z).

 0175249

Beispiel 39:

**2-Methoxy-5-<2-(N-methyl-N-[4-cyano-4-(methylethyl)-4-(3,4-dimethoxy-phenyl)-butyl]-amino-ethyl]>-phenoxy-N-(2,2-dimethyl-3-nitro-oxy-propyl-1)-acetamid**-Oxalat-di-Hydrat.

Schmp. 140°C (Z).

Beispiel 40:

**2-Methoxy-4-<1-cyano-1-(methylethyl)-4-[N-methyl-N-[2-(3,4-dimethoxy-phenyl)-ethyl]-amino-butyl]>-phenoxy-N-(2,2-dimethyl-3-nitro-oxy-propyl-1)-acetamid**-Oxalat-di-Hydrat.

Schmp. 140°C (Z).

Beispiel 41:

**2-Methoxy-5-<2-[N-methyl-N-[4-cyano-4-(methylethyl)-4-(3,4-dimethoxy-phenyl)-butyl]-amino-ethyl]>-phenoxy-N-(3-nitro-oxy-propyl-1)-acetamid**-Oxalat-di-Hydrat.

Schmp. 132-135 C°(Z).

Beispiel 42:

**2-Methoxy-5-<2-[N-methyl-N-[4-cyano-4-(methylethyl)-4-(3,4-dimethoxy-phenyl)-butyl]-amino-ethyl]>-phenoxyessigsaeure-4-(α-nitro-oxy-ethyl)-piperidylamid**-Citrat-Hydrat.

Schmp. 142-145°C (Z).

Beispiel 43:

**2-Methoxy-5-<2-[N-methyl-N-[4-cyano-4-(methylethyl)-4-(3,4-dimethoxy-phenyl)-butyl]-amino-ethyl]>-phenoxyessigsaeure-4-(nitro-oxy-methyl)-piperidylamid**-Citrat-Hydrat.

Schmp. 146-147°C (Z).

Beispiel 44:

2-Methoxy-4-<2-[N-methyl-N-[4-cyano-4-(methylethyl)-4-(3,4-dimethoxy-phenyl)-butyl]-amino-ethyl]>-phenoxyessigsaeure-4-(nitro-oxy-methyl)-piperidylamid-Citrat-Hydrat.

Schmp. 144-146°C (Z).

Beispiel 45:

2-Methoxy-4-<2-[N-methyl-N-[4-cyano-4-(methylethyl)-4-(3,4-dimethoxy-phenyl)-butyl]-amino-ethyl]>-phenoxyessigsaeure-4-(α-nitro-oxy-ethyl)-piperidylamid-Citrat-Hydrat.

Schmp. 143-144°C (Z).

Beispiel 46:

2-Methoxy-5-<1-cyano-1-(methylethyl)-4-[N-methyl-N-[2-(3,4-dimethoxy-phenyl)-ethyl]-amino-butyl]>-phenoxyessigsaeure-4-(nitro-oxy-methyl)-piperidylamid-Citrat-Hydrat.

Schmp. 142-145°C(Z).

Beispiel 47:

2-Methoxy-5-<1-cyano-1-(methylethyl)-4-[N-methyl-N-[2-(3,4-dimethoxy-phenyl)-ethyl]-amino-butyl]>-phenoxyessigsaeure-4-(α-nitro-oxy-ethyl)-piperidylamid-Citrat-Hydrat.

Schmp. 144-146°C (Z).

Beispiel 48:

2-Methoxy-5-<2-[N-methyl-N-[4-cyano-4-(methylethyl)-4-(3,4,5-trimethoxy-phenyl)-butyl]-amino-ethyl]>-phenoxy-N-(3-nitro-oxy-propyl-1)-acetamid-Citrat-Hydrat.

Schmp. 141-142°C (Z).

Beispiel 49:

**2-Methoxy-5-<2-[N-methyl-N-[4-cyano-4-(methylethyl)-4-(3,4,5-trimethoxy-phenyl)-butyl]-amino-ethyl]>-phenoxyessigsaeure-4-(nitro-oxy-methyl)-piperidylamid-**Citrat-Hydrat.

Schmp. 139-142°C (Z).

Beispiel 50:

**2-Methoxy-4-<2-[N-methyl-N-[4-cyano-4-(methylethyl)-4-(3,4,5-trimethoxy-phenyl)-butyl]-amino-ethyl]>-phenoxyessigsaeure-4-(α-nitro-oxy-ethyl)-piperidylamid-**Citrat-Hydrat.

Schmp. 143-144°C (Z).

Beispiel 51:

**2-Methoxy-4-<2-[N-methyl-N-[4-cyano-4-(methylethyl)-4-(3,4,5-trimethoxy-phenyl)-butyl]-amino-ethyl]>-phenoxyessigsaeure-4-(nitro-oxy-methyl)-piperidylamid-**Citrat-Hydrat.

Schmp. 142-144°C (Z).

Beispiel 52:

**2-Methoxy-4-<1-cyano-1-(methylethyl)-4-[N-methyl-N-[2-(3,4-dimethoxy-phenyl)-ethyl]-amino-butyl]>- phenoxyessigsaeure-4-(nitro-oxy-methyl)-piperidylamid-**Citrat-Hydrat.

Schmp. 145-147°C (Z).

Beispiel 53:

**2-Methoxy-4-<1-cyano-1-(methylethyl)-4-[N-methyl-N-[2-(3,4-dimethoxy-phenyl)-ethyl]-amino-butyl]>-phenoxyessigsaeure-4(α-nitro-oxy-ethyl)-piperidylamid-**Citrat-Hydrat.

Schmp. 143-145°C (Z).

Beispiel 54:

3-<2-[N-methyl-N-(4-cyano-4-hexyl-4-(3-methoxy-phenyl)-butyl)-amino-ethyl]>-phenoxy-N-(nitro-oxy-ethyl)-acetamid-oxalat-Hydrat Fp: 134-135°C Z

Beispiel 55:

3-<2-[N-methyl-N-(4-cyano-4-hexyl-4-(3-methoxy-phenyl)-butyl)-amino-ethyl]>-phenoxy-N-(2-nitro-oxy-propyl)-acetamid-oxalat-Hydrat Fp: 136-137°C Z

Beispiel 56:

2-Methoxy-4-<2-[N-methyl-N-[4-cyano-4-(methylethyl)-4-(3,4-dimethoxy-phenyl)-butyl]-amino-ethyl]>-phenoxy-N-(S(+)-2-nitro-oxy-propyl)-acetamid-oxalat-Hydrat,
Fp. 134-135°C Z

Beispiel 57:

2-Methoxy-4-<2-[N-methyl-N-[4-cyano-4-(methylethyl)-4-(3,4-dimethoxy-phenyl)-butyl]-amino-ethyl]>-phenoxy-N-(R(-)-2-nitro-oxy-propyl)-acetamid-oxalat-Hydrat.
Fp. 126-128°C Z

Beispiel 58:

2-Methoxy-4-<2-[N-methyl-N-[4-cyano-4-(methylethyl)-4-(3,4-dimethoxy-phenyl)-butyl]-amino-ethyl]>-phenoxy-N-[methyl-(2-nitro-oxy-cyclohexyl)]-citrat-Hydrat,
Fp. 144-146°C Z

Beispiel 59:

2-Methoxy-5-<1-cyano-1-(methylethyl)-4-[N-methyl-N-[2-(3,4-dimethoxy-phenyl)-ethyl]-amino-butyl]>-phenoxy-N-[methyl-(2-nitro-oxy-cyclohexyl)]-acetamid-citrat-Hydrat. Fp. 142-143°C Z.

Beispiel 60:

2-Methoxy-5-<2-[N-methyl-N-[4-cyano-4-(methylethyl)-4-(3,4-dimethoxy-phenyl)-butyl]-amino-ethyl]>phenoxy-N-[methyl-(2-nitro-oxy-cyclohexyl)]-acetamid-citrat-Hydrat. Fp. 80°C Z.

Die als Ausgangsmaterial benötigten Phenoxy-essigsäuren werden analog den beschriebenen Beispielen dargestellt.

## Patentansprüche

1) Phenyl-acetonitril-Derivate der allgemeinen Formel I,

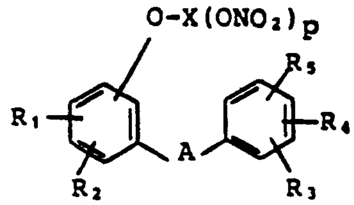

I

worin

$R_1$, $R_2$, $R_3$, $R_4$, $R_5$ gleich oder verschieden sind und Wasserstoffatome, Halogenatome, Alkylgruppen, Alkoxygruppen, Nitrogruppen, Aminogruppen und Acylaminogruppen bedeuten, wobei auch jeweils zwei Reste in Nachbarstellung zusammen einen Methylendioxy- oder Ethylendioxy-Ring bilden können,

A folgende Bedeutungen besitzt,

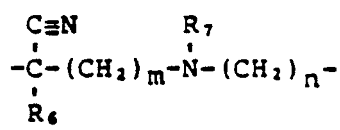

oder

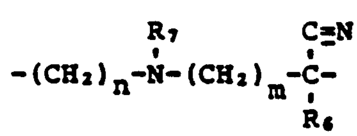

worin

$R_6$ einen geradkettigen, cyclischen oder verzweigten, gesättigten oder ungesättigten Alkylrest mit 2-12 Kohlenstoffatomen und

$R_7$ ein Wasserstoffatom oder einen geradkettigen oder verzweigten, gesättigten Alkylrest mit 1-6 Kohlenstoffatomen darstellen,

m und

n gleich oder verschieden sind und die Zahlen 2 u. 3 bedeuten und

p die Zahlen 1 und 2 bedeutet,

X einen geradkettigen, cyclischen oder verzweigten Alkylrest mit 2-10 Kohlenstoffatomen, der gegebenenfalls noch durch eine Aminogruppe substituiert sein kann oder eine Gruppierung der allgemeinen Formeln II oder II'

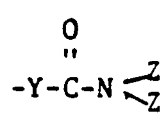

II

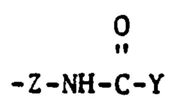

II'

worin Y und Z gleich oder verschieden sind und einen geradkettigen oder verzweigten Alkylrest mit 1-8 Kohlenstoffatomen oder einen Cycloalkylrest, Alkylcycloalkyl oder Cycloalkylalkyl darstellen, wobei diese Reste ggf. noch durch ein Sauerstoff- oder Schwefelatom unterbrochen sein können, und eine der Gruppen Z in Formel II auch Wasserstoff sein kann oder die beiden Gruppen Z zu einem Ring mit 4-6 C-Atomen geschlossen sind, der ggf. noch durch ein weiteres Stickstoffatom unterbrochen ist, welches durch Alkyl oder Alkanoyl substituiert sein kann, wobei die $O-NO_2$-Gruppen sowohl Substituenten von Y als auch Z sein können,
sowie deren Salze mit physiologisch verträglichen Säuren.

0175249

2) Verfahren zur Herstellung von Verbindungen der allgemeinen Formel I,

in der $R_1$, $R_2$, $R_3$, $R_4$, $R_5$, A, p und X die oben genannten Bedeutungen besitzen

dadurch gekennzeichnet, daß man

a) eine Verbindung der allgemeinen Formel III,

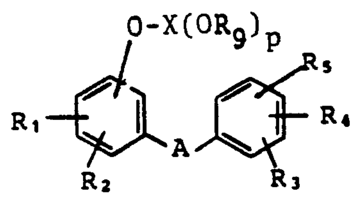

III

in der $R_1$, $R_2$, $R_3$, $R_4$, $R_5$, A, X und p die oben angegebenen Bedeutungen besitzen

und $R_9$ Wasserstoff oder eine leicht abspaltbare Gruppe darstellt

einer Nitratester-Bildungsreaktion unterwirft, oder

b) eine Verbindung der allgemeinen Formel IV,

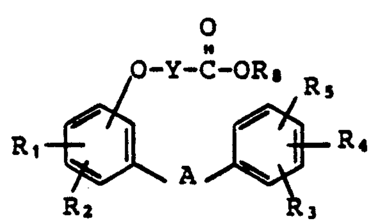

IV

in der $R_1$, $R_2$, $R_3$, $R_4$, A und Y die oben angegebenen Bedeutungen besitzen und

$R_8$ Wasserstoff oder eine niedere Alkylgruppe bedeutet,

einer Amidbildungsreaktion mit einer Verbindung der allgemeinen Formel V,

$$H_2N\text{-}Z\text{-}(ONO_2)_p \qquad V$$

wobei Z und p die angegebenen Bedeutungen besitzen,

unterwirft, oder

c) eine Verbindung der allgemeinen Formel VI

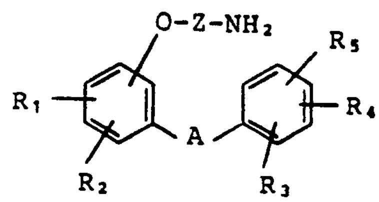

VI

in der $R_1$, $R_2$, $R_3$, $R_4$, $R_5$, A und Z die oben angegebenen Bedeutungen besitzen,

einer Amidbildungsreaktion mit einer Verbindung der allgemeinen Formel VII,

$$R_8O\text{-}\overset{O}{\overset{\|}{C}}\text{-}Y\text{-}(ONO_2)_p \qquad VII$$

wobei $R_8$, Y und p die angegebenen Bedeutungen besitzen

unterwirft, und

die erhaltenen Verbindungen ggf. mit Säuren oder Basen in die entsprechenden Salze überführt oder daraus freisetzt.

3) Arzneimittel gekennzeichnet durch einen Gehalt an Verbindungen der allgemeinen Formel I und üblichen Träger- und Hilfsstoffen.

4) Verwendung von Arzneimitteln gemäß Anspruch 3 zur Behandlung von Erkrankungen des Herz- und Kreislaufsystems.